(19) Europäisches Patentamt
European Patent Office
Office européen des brevets

(11) **EP 2 356 507 B1**

(12) **EUROPÄISCHE PATENTSCHRIFT**

(45) Veröffentlichungstag und Bekanntmachung des Hinweises auf die Patenterteilung:
**20.11.2013 Patentblatt 2013/47**

(21) Anmeldenummer: **09796941.4**

(22) Anmeldetag: **12.11.2009**

(51) Int Cl.:
***G02C 7/02*** *(2006.01)*

(86) Internationale Anmeldenummer:
**PCT/EP2009/008069**

(87) Internationale Veröffentlichungsnummer:
**WO 2010/054817 (20.05.2010 Gazette 2010/20)**

(54) **OPTIMIERUNG UND HERSTELLUNG EINES BRILLENGLASES ZUR KORREKTION EINER ASTIGMATISCHEN REFRAKTION**

OPTIMIZATION AND PRODUCTION OF AN EYEGLASS LENS FOR CORRECTING AN ASTIGMATIC REFRACTION

OPTIMISATION ET FABRICATION D'UN VERRE DE LUNETTES POUR LA CORRECTION D'UNE RÉFRACTION ASTIGMATIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **13.11.2008 DE 102008057205**
**13.11.2008 DE 102008057206**

(43) Veröffentlichungstag der Anmeldung:
**17.08.2011 Patentblatt 2011/33**

(73) Patentinhaber: **Rodenstock GmbH**
**80687 München (DE)**

(72) Erfinder:
• **BECKEN, Wolfgang**
**81541 München (DE)**
• **WELK, Andrea**
**81547 München (DE)**
• **SEIDEMANN, Anne, Dr.**
**81371 München (DE)**
• **ESSER, Gregor**
**81735 München (DE)**
• **ALTHEIMER, Helmut**
**87650 Baisweil-Lauchdorf (DE)**
• **UTTENWEILER, Dietmar**
**82057 Icking (DE)**

(74) Vertreter: **Müller-Boré & Partner**
**Patentanwälte**
**Grafinger Straße 2**
**81671 München (DE)**

(56) Entgegenhaltungen:
**WO-A1-2008/089998    US-A1- 2003 107 702**

**Beschreibung**

Die Erfindung betrifft ein Verfahren zur Optimierung und Herstellung zumindest eines Brillenglases, insbesondere eines Brillenglaspaares, zur Korrektion zumindest einer astigmatischen Refraktion eines ersten Auges eines Brillenträgers, insbesondere zur Korrektion einer ersten astigmatischen Refraktion des ersten Auges mittels eines ersten Brillenglases des Brillenglaspaares und einer zweiten astigmatischen Refraktion eines zweiten Auges des Brillenträgers mittels eines zweiten Brillenglases des Brillenglaspaares. Ferner bezieht sich die Erfindung auf zumindest ein Brillenglas, insbesondere ein Brillenglaspaar, zur Verwendung in einer bestimmten Gebrauchssituation zur Korrektion zumindest einer astigmatischen Refraktion eines ersten Auges eines Brillenträgers, insbesondere zur Korrektion einer ersten astigmatischen Refraktion des ersten Auges und einer zweiten astigmatischen Refraktion eines zweiten Auges des Brillenträgers, ein Computerprogrammerzeugnis, ein Speichermedium und eine Vorrichtung zur Herstellung zumindest eines Brillenglases, insbesondere eines Brillenglaspaares, zur Korrektion zumindest einer astigmatischen Refraktion eines ersten Auges eines Brillenträgers, insbesondere zur Korrektion einer ersten astigmatischen Refraktion des ersten Auges und einer zweiten astigmatischen Refraktion eines zweiten Auges eines Brillenträgers.

Für die Herstellung bzw. Optimierung von Brillengläsern, insbesondere von individuellen Brillengläsern wird jedes Brillenglas so gefertigt, dass für jede gewünschte Blickrichtung oder jeden gewünschten Objektpunkt eine möglichst gute Korrektur eines Refraktionsfehlers des jeweiligen Auges des Brillenträgers erreicht wird. Im Allgemeinen gilt ein Brillenglas für eine gegebene Blickrichtung dann als vollkorrigierend, wenn die Werte Sphäre, Zylinder und Achse der Wellenfront beim Passieren der Scheitelpunktkugel mit den Werten für Sphäre, Zylinder und Achse übereinstimmen. Eine vollständige Korrektur für alle Blickrichtungen gleichzeitig ist aber im Normalfall nicht möglich. Daher werden die Brillengläser derart gefertigt, dass sie vor allem in den hauptsächlichen Nutzungsbereichen, insbesondere in zentralen Durchblickbereichen eine gute Korrektur von Fehlsichtigkeiten des Auges und nur geringe Abbildungsfehler bewirken, während in peripheren Bereichen größere Abbildungsfehler zugelassen werden. Diese Abbildungsfehler hängen von Art und Umfang der erforderlichen Korrekturen sowie von der Position auf dem Brillenglas, also dem jeweiligen Durchblickpunkt ab.

Insbesondere für eine Korrektion einer astigmatischen Refraktionen eines Auges ist neben der Kenntnis des Betrags der astigmatischen Refraktion, d.h. des Wertes des Zylinders auch dessen Achslage entscheidend. Um eine astigmatische Refraktion des Auges korrigieren zu können, werden daher diese Werte für das zu korrigierende Auge vermessen, während sich das Auge in einer Messstellung, bzw. Referenzblickrichtung, insbesondere der Nullblickrichtung befindet. Dabei wird vorzugsweise ein Koordinatensystem festgelegt, und die Achslage der astigmatischen Refraktion in Bezug auf dieses Koordinatensystem bestimmt. Der Betrag des Astigmatismus kann als Differenz der Hauptbrechwerte angegeben werden. Als Koordinatensystem lässt sich dabei beispielsweise ein kartesisches Koordinatensystem mit den Achsen $e_x$, $e_y$ und $e_z$ angeben, dessen Koordinatenursprung insbesondere im Augendrehpunkt des zu korrigierenden Auges liegt. Dabei zeigt die Achse $e_z$ vorzugsweise parallel zur Referenzblickrichtung, insbesondere zur Nullblickrichtung und ist in Richtung des Hauptstrahls orientiert. Vorzugsweise ist die Achse $e_z$ eine horizontale Achse, die in Bezug auf das Auge in der Nullblickrichtung nach hinten, also in Richtung des Lichtstrahls zeigt. Die Achse $e_x$ liegt beispielsweise horizontal und senkrecht zur Achse $e_z$, insbesondere senkrecht zur Referenzblickrichtung bzw. Nullblickrichtung. Die Achse $e_y$ liegt schließlich senkrecht zu den beiden anderen Achsen und ist insbesondere vertikal nach oben orientiert. Damit bilden die drei Achsen $e_x$, $e_y$ und $e_z$ beispielsweise ein Basiskoordinatensystem, in dem sich auch die Achslage eines zu korrigierenden Astigmatismus beschreiben lässt.

Beim Blicken durch ein Brillenglas führt das Augenpaar ständig Blickbewegungen aus, wodurch sich die Durchblickpunkte innerhalb des Brillenglases verändern. Damit ergeben sich bei Blickbewegungen ständig Veränderungen der Abbildungseigenschaften, insbesondere der Abbildungsfehler für das Brillenglas. Außerdem führt jedes Auge bei Blickbewegungen auch eine Torsion um die momentane Achse der Blickrichtung aus, welche insbesondere von der Blickrichtung selbst abhängt. Bei einer astigmatischen Refraktion des Auges führt dies insbesondere im Nahbereich oft zu einer unbefriedigenden Korrektion des Astigmatismus. Um diesem Problem zu begegnen, wird beispielsweise in der Druckschrift WO 2008/089998 A1 vorgeschlagen, bei der Optimierung eines Brillenglases für ein Auge eine leichte Rollbewegung des Auges bei peripheren Blickauslenkungen gemäß der später noch genauer beschriebenen Listing'schen Regel "L1" zu berücksichtigen. Ein ähnlicher Vorschlag zur Berücksichtigung einer Rollbewegung eines Auges bei dessen Blickauslenkung gemäß der Listing'schen Regel "L1" ist auch bereits in der Druckschrift US 2003/0107702 A1 offenbart.

Aufgabe der vorliegenden Erfindung ist es, ein Verfahren und ein Computerprogrammprodukt zur Optimierung und Herstellung zumindest eines ersten Brillenglases, insbesondere eines Brillenglaspaares, insbesondere zur Korrektion einer astigmatischen Refraktion mit verbesserten optischen Eigenschaften insbesondere für die Nutzung des Brillenglases bzw. Brillenglaspaares in der Nähe bereitzustellen.

Diese Aufgabe wird durch die Erfindung gelöst, wie in den unabhängigen Ansprüchen angegeben. Bevorzugte Ausführungsformen sind Gegenstand der abhängigen Ansprüche.

Somit stellt die Erfindung insbesondere ein Verfahren zur Optimierung und Herstellung zumindest eines ersten Brillenglases für eine bestimmte Gebrauchssituation zur Korrektion zumindest einer ersten astigmatischen Refraktion (bzw. einer ersten vektoriellen astigmatischen Refraktion) eines ersten Auges eines Brillenträgers, welche in einer Referenzblickrichtung $-\mathbf{e}_z^{(1)}$ des ersten Auges einen ersten Zylinderwert und eine erste Achslage bzw. Zylinderreferenzachse $\boldsymbol{\alpha}_0^{(1)}$, d.h. eine Zylinderachse der Augenrefraktion des ersten Auges bei Stellung des ersten Auges in der Referenzblickrichtung des ersten Auges, aufweist, bereit, umfassend zumindest einen primären Berechnungs- bzw. Optimierungsschritt des ersten Brillenglases, das heißt zumindest einer Fläche oder eines Flächenbereichs des ersten Brillenglases (daher auch als erster primärer Berechnungs- bzw. Optimierungsschritt bezeichnet), welcher umfasst:

-- Ermitteln einer insbesondere von der Referenzblickrichtung $-\mathbf{e}_z^{(1)}$ des ersten Auges abweichenden primären Blickrichtung $-\mathbf{e}_\zeta^{(1,p)}$ des ersten Auges (daher auch als erste primäre Blickrichtung $-\mathbf{e}_\zeta^{(1,p)}$ bezeichnet) für zumindest eine primäre Bewertungsstelle $i_b^{(1,p)}$ des ersten Brillenglases (daher auch als erste primäre Bewertungsstelle $i_b^{(1,p)}$ bezeichnet); und

-- Ermitteln einer in der bestimmten Gebrauchssituation zur primären Blickrichtung $-\mathbf{e}_\zeta^{(1,p)}$ des ersten Auges korrespondierenden primären Blickrichtung $-\mathbf{e}_{\zeta,k}^{(2,p)}$ eines zweiten Auges (daher auch als korrespondierende zweite primäre Blickrichtung $-\mathbf{e}_{\zeta,k}^{(2,p)}$ bezeichnet) des Brillenträgers insbesondere in Abhängigkeit von Startvorgaben für ein zweites Brillenglas, welches insbesondere zur Benutzung mit dem ersten Brillenglas in einem Brillenlaspaar vorgesehen ist. Es wird also vorzugsweise ein zweites, insbesondere noch nicht gemäß der vorliegenden Erfindung optimiertes Brillenglas als Startvorgabe verwendet, um auf Basis dieses vorgegebenen zweiten Brillenlases für die bestimmte Gebrauchssituation die zur ermittelten primären Blickrichtung des ersten Auges korrespondierende primäre Blickrichtung des zweiten Auges zu ermitteln. Vorzugsweise geschieht dies unter Verwendung eines Ray-Tracing-Verfahrens. Damit wird insbesondere die lokale, also blickrichtungsabhängige prismatische Wirkung des ersten und/oder zweiten Brillenglases in der bestimmten Gebrauchssituation für die Berechnung des ersten Brillenglases berücksichtigt.

Der erste primäre Berechnungs- bzw. Optimierungsschritt umfasst außerdem ein Minimieren einer primären Zielfunktion für zumindest eine Fläche des ersten Brillenglases (auch als erste primäre Zielfunktion bezeichnet), wobei in der ersten primären Zielfunktion für die zumindest eine primäre Bewertungsstelle $i_b^{(1,p)}$ des ersten Brillenglases eine Korrektion einer ersten primären transformierten astigmatischen Refraktion bzw. ersten primären transformierten vektoriellen astigmatischen Refraktion (wobei unter einer astigmatischen Refraktion bzw. einer vektoriellen astigmatischen Refraktion insbesondere das Paar aus einem Zylinderwert und einem Einheitsvektor als Achse verstanden wird, der in der Ebene senkrecht zur Blickrichtung liegt und in Richtung der Achslage des Astigmatismus zeigt) durch das erste Brillenglas in der bestimmten Gebrauchssituation derart berücksichtigt bzw. ausgewertet wird, dass die erste primäre transformierte astigmatische Refraktion von der ermittelten korrespondierenden primären Blickrichtung $-\mathbf{e}_{\zeta,k}^{(2,p)}$ des zweiten Auges abhängt, d.h. für zumindest zwei verschiedene korrespondierende primäre Blickrichtungen $-\mathbf{e}_{\zeta,k}^{(2,p)}$ des zweiten Auges, wie sie sich beispielsweise für verschiedene Startvorgabe des zweiten Brillenglases ergeben könnten, verschiedene Werte aufweist.

Vorzugsweise wird ein ortsfestes bzw. objektfestes Basiskoordinatensystem für das jeweilige Auge, wie bereits oben beispielhaft beschrieben, festgelegt. Im jeweiligen Basiskoordinatensystem wird vorzugsweise die Zylinderachse einer astigmatischen Refraktion des jeweiligen Auges, also des ersten und/oder zweiten Auges, von einem Optiker oder Augenarzt für einen Patienten oder Brillenträger in der Referenzblickrichtung individuell ermittelt und als Zylinderreferenzachse für das Verfahren zur Optimierung und Herstellung zumindest des ersten Brillenglases, insbesondere des Brillenglaspaares, zur Korrektion der jeweiligen astigmatischen Refraktionen bereitgestellt. In einer bevorzugten Ausführungsform ist die Referenzblickrichtung des ersten und/oder zweiten Auges die jeweilige Nullblickrichtung und verläuft horizontal gerade aus in die Ferne bzw. ins Unendliche. Sie ist damit parallel zur dritten Basiskoordinatenachse $\mathbf{e}_z^{(1)}$ bzw. $\mathbf{e}_z^{(2)}$ des ersten bzw. zweiten Auges, wobei sie in einer bevorzugten Konvention entgegengesetzt orientiert ist, was durch das Minuszeichen zum Ausdruck gebracht werden soll. Auch für die erste und zweite primäre Blickrichtung soll diese Konvention gelten, weshalb diese Blickrichtungen parallel zum jeweiligen augenseitigen Hauptstrahl liegen und entgegengesetzt dazu orientiert sind. Die jeweilige Zylinderreferenzachse lässt sich beispielsweise über die anderen beiden Koordinatenachsen ausdrücken.

Es wird also ausgehend von der in der Referenzblickrichtung zu korrigierenden astigmatischen Refraktion des ersten Auges, welche beispielsweise von einem Augenoptiker in bekannter Weise gemessen bzw. ermittelt werden kann, eine Transformation durchgeführt, die von der in der bestimmten Gebrauchssituation korrespondierenden Blickrichtung des zweiten Auges insbesondere unter Berücksichtigung des zweiten Brillenglases in dessen Position vor dem zweiten Auge des Brillenträgers abhängt, um zu der transformierten astigmatischen Refraktion zu kommen. Die transformierte astigmatische Refraktion wird dabei vorzugsweise wie die astigmatische Refraktion in der Referenzblickrichtung in Form eines Zylinderwerts (insbesondere als skalare Größe mit der Einheit dpt) und einer Achslage (z.B. in Form eines transformierten Winkels relativ zu einer Torsionsreferenzachse der ersten Blickrichtung) angeben. Diese transformierte astigmatische Refraktion fließt dann als die zu korrigierende Refraktion des Auges in der zur ersten Bewertungsstelle gehörenden Blickrichtung in die Zielfunktion, insbesondere die erste primäre Zielfunktion, für die Berechnung bzw. Optimierung des ersten Brillenglases ein.

Vorzugsweise betrifft die Erfindung ein Verfahren zur Optimierung und Herstellung des ersten Brillenglases für ein Paar von Brillengläsern zur Benutzung zusammen mit einem zweiten Brillenglas des Paares von Brillengläsern in einer Brille für die bestimmte Gebrauchssituation, wobei das Ermitteln der korrespondierenden primären Blickrichtung $-\mathbf{e}_{\zeta,k}^{(2,p)}$ des zweiten Auges ein Ermitteln einer in der bestimmten Gebrauchssituation zur primären Bewertungsstelle $i_b^{(1,p)}$ des ersten Brillenglases korrespondierenden primären Bewertungsstelle $i_b^{(2,p)}$ des zweiten Brillenglases unter Berücksichtigung einer prismatischen Wirkung des zu optimierenden ersten Brillenglases und/oder des zweiten Brillenglases in der primären Bewertungsstelle des ersten bzw. zweiten Brillenglases in der bestimmten Gebrauchssituation umfasst.

Dabei umfasst das Verfahren vorzugsweise ein Erfassen einer zweiten Zylinderreferenzachse $\mathbf{\alpha}_0^{(2)}$ einer zweiten astigmatischen Refraktion des zweiten Auges in einer Referenzblickrichtung $-\mathbf{e}_z^{(2)}$ des zweiten Auges, wobei die erste primäre Zielfunktion für die zumindest eine Fläche des ersten Brillenglases von einer Korrektion einer zweiten primären transformierten astigmatischen Refraktion durch das zweite Brillenglas in der bestimmten Gebrauchssituation abhängt, wobei die zweite primäre transformierte astigmatische Refraktion eine zweite primäre Zylinderkorrektionsachse $\mathbf{\alpha}_K^{(2,p)}$ aufweist, welche mit einer sowohl zur Referenzblickrichtung $-\mathbf{e}_z^{(2)}$ des zweiten Auges als auch zur korrespondierenden primären Blickrichtung $-\mathbf{e}_{\zeta,k}^{(2,p)}$ des zweiten Auges senkrechten zweiten primären Torsionsreferenzachse $\mathbf{e}_L^{(2,p)}$ einen zweiten primären Korrektionstorsionswinkel $\psi_K^{(2,p)}$ einschließt, der von einem zweiten primären Referenztorsionswinkel $\psi_0^{(2,p)}$ zwischen der zweiten Zylinderreferenzachse $\mathbf{\alpha}_0^{(2)}$ und der zweiten primären Torsionsreferenzachse $\mathbf{e}_L^{(2,p)}$ um einen zweiten primären Torsionskorrekturwinkel $\psi_\Delta^{(2,p)}\!\left(\mathbf{e}_\zeta^{(1,p)}\right)$ abweicht, welcher zumindest von der ermittelten primären Blickrichtung $-\mathbf{e}_\zeta^{(1,p)}$ des ersten Auges abhängt.

Vorzugsweise hängt der zweite primäre Torsionskorrekturwinkel sowohl von der primären Blickrichtung des ersten Auges als auch von der primären Blickrichtung des zweiten Auges ab. Besonders bevorzugt gilt: $\psi_\Delta^{(2)} = -\psi_\Delta^{(1)}$, insbesondere $\psi_\Delta^{(2,p)} = -\psi_\Delta^{(1,p)}$, besonders bevorzugt wird dabei ein Mittelwert von Torsionswinkeln insbesondere in Helmholtz-Koordinaten oder anderen für die Darstellung der Torsion geeigneten Koordinaten gebildet.

In einer bevorzugten Ausführungsform ist das zweite Brillenglas vor der Optimierung des herzustellenden ersten Glases bekannt und wird bei der Optimierung festgehalten. Bei der Auswertung der Zielfunktion zu jedem Punkt bzw. zu jeder Bewertungsstelle des zu optimierenden Glases wird der korrespondierende Durchblickspunkt des festgehaltenen Glases berechnet, damit daraus die Helmholtz-Winkel bestimmt werden können. Für die Berechnung von Soll-Ist-Abweichungen des Astigmatismus in der Zielfunktion wird dabei die Torsionsstellung des Auges vorzugsweise nach Gl. (8) oder Gl. (9) bestimmt.

Vorzugsweise betrifft die Erfindung ein Verfahren zur Optimierung und Herstellung eines Brillenglaspaares für eine bestimmte Gebrauchssituation zur Korrektion einer ersten astigmatischen Refraktion eines ersten Auges eines Brillenträgers, welche in einer Referenzblickrichtung $-\mathbf{e}_z^{(1)}$ des ersten Auges einen ersten Zylinderwert und eine erste Achslage bzw. Zylinderreferenzachse $\alpha_0^{(1)}$, d.h. eine Zylinderachse der Augenrefraktion des ersten Auges bei Stellung des ersten Auges in der Referenzblickrichtung des ersten Auges, aufweist, mittels eines ersten Brillenglases des Brillenglaspaares und einer zweiten astigmatischen Refraktion eines zweiten Auges des Brillenträgers, welche in einer Referenzblickrichtung $-\mathbf{e}_z^{(2)}$ des zweiten Auges einen zweiten Zylinderwert und eine zweite Achslage bzw. Zylinderreferenzachse $\alpha_0^{(2)}$, d.h. eine Zylinderachse der Augenrefraktion des zweiten Auges bei Stellung des zweiten Auges in der Referenzblickrichtung des zweiten Auges, aufweist, mittels eines zweiten Brillenglases des Brillenglaspaares bereit, umfassend einen primären Berechnungs- bzw. Optimierungsschritt des ersten Brillenglases, das heißt zumindest einer Fläche oder eines Flächenbereichs des ersten Brillenglases (daher auch als erster primärer Berechnungs- bzw. Optimierungsschritt bezeichnet), welcher umfasst:

-- Ermitteln einer insbesondere von der Referenzblickrichtung $-\mathbf{e}_z^{(1)}$ des ersten Auges abweichenden primären Blickrichtung $-\mathbf{e}_\zeta^{(1,p)}$ des ersten Auges (daher auch als erste primäre Blickrichtung $-\mathbf{e}_\zeta^{(1,p)}$ bezeichnet) für zumindest eine primäre Bewertungsstelle $i_b^{(1,p)}$ des ersten Brillenglases (daher auch als erste primäre Bewertungsstelle $i_b^{(1,p)}$ bezeichnet); und

-- Ermitteln einer in der bestimmten Gebrauchssituation zur primären Blickrichtung $-\mathbf{e}_\zeta^{(1,p)}$ des ersten Auges korrespondierenden primären Blickrichtung $-\mathbf{e}_{\zeta,k}^{(2,p)}$ des zweiten Auges (daher auch als korrespondierende zweite primäre Blickrichtung $-\mathbf{e}_{\zeta,k}^{(2,p)}$ bezeichnet) des Brillenträgers in Abhängigkeit von Startvorgaben für das zweite Brillenglas.

Vorzugsweise umfasst das Verfahren außerdem einen sekundären Berechnungs- bzw. Optimierungsschritt des zweiten Brillenglases, das heißt zumindest einer Fläche oder eines Flächenbereichs des zweiten Brillenglases, (daher auch als zweiter sekundärer Berechnungs- bzw. Optimierungsschritt bezeichnet) in Abhängigkeit von dem im ersten primären Berechungs- bzw. Optimierungsschritt ermittelten ersten Brillenglas. Damit wird insbesondere die Startvorgabe für das zweite Brillenglas unter Berücksichtigung des im ersten primären Berechnungs- bzw. Optimierungsschritt ermittelten ersten Brillenglases abgeändert und optimiert. Vorzugsweise erfolgt die Berechnung bzw. Optimierung des zweiten Brillenglases in analoger Weise zur Berechnung bzw. Optimierung des ersten Brillenglases, wobei das im ersten primären Berechungs- bzw. Optimierungsschritt ermittelte erste Brillenglas entsprechend bei der Berechnung bzw. Optimierung des zweiten Brillenglases in analoger Weise berücksichtigt wird, wie die Startvorgabe des zweiten Brillenglases bei der Berechnung bzw. Optimierung des ersten Brillenglases. Dabei wird vorzugsweise eine zweite sekundäre Zielfunktion minimiert.

Bei der Optimierung bewertet die erste primäre bzw. zweite sekundäre Zielfunktion insbesondere lokale Werte der Fehlrefraktion des ersten bzw. zweiten Brillenglases in einer Vielzahl von Bewertungsstellen des jeweiligen Brillenglases, also für eine Vielzahl verschiedener Blickrichtungen des zugehörigen Auges in der bestimmten Gebrauchssituation. Dabei wird vorzugsweise zu jeder Bewertungsstelle des entsprechenden Brillenglases einerseits die der bestimmten Gebrauchssituation entsprechende Blickrichtung des jeweiligen Auges ermittelt, andererseits wird vorzugsweise für jede Bewertungsstelle eine der ermittelten Blickrichtung entsprechende, aber für die Gebrauchssituation mit Bezug auf eine korrespondierende Augenbewegung des anderen Auges auf Basis einer Vorgabe des zweiten Brillenglases bzw. des ermittelten ersten Brillenglases korrigierte bzw. transformierte astigmatische Refraktion des ersten bzw. zweiten Auges berücksichtigt. Dies führt zu einer besonders guten binokularen Verträglichkeit eines damit optimierten und hergestellten Brillenglaspaares insbesondere für die Nutzung in der Nähe.

Somit wird insbesondere eine Optimierung bzw. Herstellung zumindest eines Brillenglases, vorzugsweise eines Brillenglaspaares durch eine sukzessive monokulare Berechnung bzw. Optimierung einzelner Brillengläser erreicht, wobei im jeweiligen monokularen Berechnungs- bzw. Optimierungsschritt (primärer und/oder sekundärer Berechnungs- bzw. Optimierungsschritt) für eines der beiden Brillengläser das andere Brillenglas berücksichtigt, aber festgehalten wird bis insbesondere eine erwünschte bzw. vorgegebene Konvergenz eines monokularen Optimierungsverfahrens, insbesondere im ersten primären Berechnungs- bzw. Optimierungsschritt, für das eine Brillenglas erreicht ist. Damit wird eine sehr effiziente und schnelle Herstellung eines Brillenglases, insbesondere eines Brillenglaspaares, erreicht. Dabei werden vorzugsweise für beide Brillengläser des Brillenglaspaares die zylindrischen Verordnungen berücksichtigt. Obgleich das Verfahren sowohl zur Optimierung und Herstellung eines ersten Brillenglases als auch zur Optimierung und Herstellung eines Brillenglaspaares vorzugsweise die gekoppelten Freiheitsgrade von zwei Gläsern abändert und dabei binokulares Sehen verbessert, liegt der Aufwand, insbesondere der numerische Aufwand des Verfahrens nur bei dem Aufwand weniger monokularer Optimierungen.

Für die jeweilige Nummerierung bzw. Bezeichnung einzelner Berechnungs- bzw. Optimierungsschritte, jeweiliger Achsen, Richtungen und Winkel werden in der vorliegenden Beschreibungen der Erfindung und ihrer bevorzugten Ausführungsformen folgende Konventionen verwendet, die zur leichteren Verständnis hier im Überblick zusammengefasst sind:

- Vektoren und Richtungen sind fett gedruckt, während Skalare und Indizes normal (d.h. nicht fett) dargestellt sind.

- Die sich auf das **erste** Auge, Brillenglas oder Basiskoordinatensystem beziehenden Verfahrensschritte, Achsen, Richtungen, Winkel, Refraktionsgrößen, usw. werden entweder mit dem Zusatz "des ersten Auges" bzw. "des ersten Brillenglases" oder mit der vorangestellten Bezeichnung "erster" bzw. "erste" bzw. "erstes" versehen. In der mathematischen Darstellung erhalten sie einen hochgestellten Index "1" (in Klammern).

- Die sich auf das **zweite** Auge, Brillenglas oder Basiskoordinatensystem beziehenden Verfahrensschritte, Achsen, Richtungen, Winkel, Refraktionsgrößen, usw. werden entweder mit dem Zusatz "des zweiten Auges" bzw. "des zweiten Brillenglases" oder mit der vorangestellten Bezeichnung "zweiter" bzw. "zweiter" bzw. "zweites" versehen. In der mathematischen Darstellung erhalten sie einen hochgestellten Index "2" (in Klammern).

- Die sukzessiv durchgeführten Berechnungs- bzw. Optimierungsschritte erhalten den jeweiligen Zusatz

    -- **"primär"** zur Bezeichnung eines ersten von mehreren aufeinanderfolgenden Schritten (Berechnungs- bzw. Optimierungsschritten) gemäß der Erfindung. Die in diesem Schritt ermittelten bzw. berücksichtigten Größen (insbesondere Achsen, Richtungen, Winkel, usw.) insbesondere soweit sich ihr Wert im jeweiligen Schritt von dem entsprechenden Wert in einem späteren Schritt unterscheiden könnte, werden ebenfalls mit der Bezeichnung "primär" versehen. In der mathematischen Darstellung erhalten sie einen hochgestellten Index "p" (in Klammern).
    -- **"sekundär"** zur Bezeichnung eines weiteren bzw. zweiten, nach dem ersten, insbesondere im Anschluss an den ersten, also "primären" Schritt ausgeführten Schritt (Berechnungs- bzw. Optimierungsschritt) gemäß der Erfindung. Die in diesem Schritt ermittelten bzw. berücksichtigten Größen (Achsen, Richtungen, Winkel, usw.) insbesondere soweit sich ihr Wert im jeweiligen Schritt von dem entsprechenden Wert in einem anderen (z.B. frühren oder späteren) Schritt unterscheiden könnte, werden ebenfalls mit der Bezeichnung "sekundär" versehen. In der mathematischen Darstellung erhalten sie einen hochgestellten Index "s" (in Klammern).
    -- In analoger Weise können weitere Schritte und Größen die

Bezeichnungen **"tertiär", "quartär",** usw. mit entsprechenden, hochgestellten Indizes "t" bzw. "q" tragen.

Soweit es im Einzelfall nicht darauf ankommt, auf welches Auge bzw. Brillenglas oder auf welchen von mehreren Berechnungs- bzw. Optimierungsschritten sich eine weitere Charakterisierung einer Größe im Sinne einer bevorzugten Ausführungsform der vorliegenden Erfindung bezieht, d.h. falls eine solche Charakterisierung gleichermaßen auf jedes Auge bzw. Brillenglas oder auf jeden Berechnungs- bzw. Optimierungsschritt anwendbar ist, kann bei der entsprechenden Größe die genannte Bezeichnung bzw. der entsprechende Index auch weggelassen werden.

- Die jeweils als korrespondierende Blickrichtung bezeichnete Richtung trägt einen tief gestellten Index "k".

Vorzugsweise wird in der ersten primären Zielfunktion für die zumindest eine primäre Bewertungsstelle $i_b^{(1,p)}$ des ersten Brillenglases die Korrektion einer ersten primären transformierten astigmatischen Refraktion durch das erste Brillenglas in der bestimmten Gebrauchssituation derart berücksichtigt, dass die erste primäre transformierte astigmatische Refraktion in Bezug auf die primäre Blickrichtung des ersten Auges den ersten Zylinderwert und eine erste primäre Zylinderkorrektionsachse $\alpha_K^{(1,p)}$ (d.h. Zylinderachse der Augenrefraktion bei Stellung des ersten Auges in der ersten primären Blickrichtung) aufweist bzw. durch den ersten Zylinderwert und eine erste primäre Zylinderkorrektionsachse $\alpha_K^{(1,p)}$ festgelegt ist, wobei die erste primäre Zylinderkorrektionsachse $\alpha_K^{(1,p)}$ mit einer sowohl zur Referenzblickrichtung $-\mathbf{e}_z^{(1)}$ des ersten Auges als auch zur primären Blickrichtung $-\mathbf{e}_\zeta^{(1,p)}$ des ersten Auges senkrechten ersten primären Torsionsreferenzachse $\mathbf{e}_L^{(1,p)}$ einen ersten primären Korrektionstorsionswinkel $\psi_K^{(1,p)}$ einschließt, der von einem ersten primären Referenztorsionswinkel $\psi_0^{(1,p)}$ zwischen der ersten Zylinderreferenzachse $\alpha_0^{(1)}$ und der ersten primären Torsionsreferenzachse $\mathbf{e}_L^{(1,p)}$ um einen ersten primären Torsionskorrekturwinkel $\psi_\Delta^{(1,p)}\left(\mathbf{e}_{\zeta,k}^{(2,p)}\right)$ abweicht, welcher zumindest von der ermittelten korrespondierenden primären Blickrichtung $-\mathbf{e}_{\zeta,k}^{(2,p)}$ des zweiten Auges abhängt, d.h. für zumindest zwei verschiedene korrespondierende primäre Blickrichtungen $-\mathbf{e}_{\zeta,k}^{(2,p)}$ des zweiten Auges, wie sie sich beispielsweise für verschiedene Startvorgabe des zweiten Brillenglases und/oder für verschiedene Gebrauchssituationen ergeben könnten, verschiedene Werte aufweist.

Bei der Optimierung bewertet die erste primäre Zielfunktion insbesondere lokale Werte der Fehlrefraktion des ersten Brillenglases in einer Vielzahl von Bewertungsstellen des Brillenglases, also für eine Vielzahl verschiedener Blickrichtungen des ersten Auges in der bestimmten Gebrauchssituation. Dabei wird vorzugsweise zu jeder Bewertungsstelle des ersten Brillenglases einerseits die der bestimmten Gebrauchssituation entsprechende Blickrichtung des ersten Auges ermittelt, andererseits wird vorzugsweise für jede Bewertungsstelle eine der ermittelten Blickrichtung entsprechende, aber für die Gebrauchssituation mit Bezug auf das zweite Auge korrigierte Achslage der astigmatischen Refraktion des ersten Auges berücksichtigt. Dabei wird ein Modell für die Torsionseinstellung des ersten Auges zugrundegelegt, das außerdem von der Blickrichtung des zweiten Auges abhängt. Damit wird insbesondere eine Verbesserung der optischen Eigenschaften des Brillenglases im Nahbereich erreicht, bei dem der Einfluss des zweiten Auges auf die Torsionsbewegung des ersten Auges besonders groß ist. Es wird damit im Nahbereich insbesondere auch das binokulare Sehen verbessert.

Soweit in einer bevorzugten Ausführungsform der zweite sekundäre Berechnungs- bzw. Optimierungsschritt analog zum ersten primären Berechnungs- bzw. Optimierungsschritt ausgeführt wird, umfasst der zweite sekundäre Berechnungs- bzw. Optimierungsschritt damit vorzugsweise:

-- Ermitteln einer sekundären Blickrichtung $-\mathbf{e}_\zeta^{(2,s)}$ des zweiten Auges (daher auch als zweite sekundäre Blickrichtung $-\mathbf{e}_\zeta^{(2,s)}$ bezeichnet für zumindest eine sekundäre Bewertungsstelle $i_b^{(2,s)}$ des zweiten Brillenglases (daher auch als zweite sekundäre Bewertungsstelle $i_b^{(2,s)}$ bezeichnet);

-- Ermitteln einer in der bestimmten Gebrauchssituation zur sekundären Blickrichtung $-\mathbf{e}_\zeta^{(2,s)}$ des zweiten Auges korrespondierenden sekundären Blickrichtung $-\mathbf{e}_{\zeta,k}^{(1,s)}$ des ersten Auges (daher auch als korrespondierende erste sekundäre Blickrichtung $-\mathbf{e}_{\zeta,k}^{(1,s)}$ bezeichnet) des Brillenträgers in Abhängigkeit von dem im ersten primären Berechungs- bzw. Optimierungsschritt ermittelten ersten Brillenglas; und

-- Minimieren einer sekundären Zielfunktion für zumindest eine Fläche des zweiten Brillenglases (daher auch als zweite sekundäre Zielfunktion bezeichnet), wobei in der zweiten sekundären Zielfunktion für die zumindest eine sekundäre Bewertungsstelle $i_b^{(2,s)}$ des zweiten Brillenglases eine Korrektion einer zweiten sekundären transformierten astigmatischen Refraktion durch das zweite Brillenglas in der bestimmten Gebrauchssituation derart berücksichtigt wird, dass die zweite sekundäre transformierte astigmatische Refraktion in Bezug auf die sekundäre Blickrichtung des zweiten Auges den zweiten Zylinderwert und eine zweite sekundäre Zylinderkorrektionsachse $\alpha_K^{(2,s)}$ (d.h. Zylinderachse der Augenrefraktion bei Stellung des zweiten Auges in der zweiten sekundären Blickrichtung) aufweist bzw. durch den zweiten Zylinderwert und eine zweite sekundäre Zylinderkorrektionsachse $\alpha_K^{(2,s)}$ festgelegt ist, wobei die zweite sekundäre Zylinderkorrektionsachse $\alpha_K^{(2,s)}$ mit einer sowohl zur Referenzblickrichtung $-\mathbf{e}_z^{(2)}$ des zweiten Auges als auch zur sekundären Blickrichtung $-\mathbf{e}_\zeta^{(2,s)}$ des zweiten Auges senkrechten zweiten sekundären Torsionsreferenzachse $\mathbf{e}_L^{(2,s)}$ einen zweiten sekundären Korrektionstorsionswinkel $\psi_K^{(2,s)}$ einschließt, der von einem zweiten sekundären Referenztorsionswinkel $\psi_0^{(2,s)}$ zwischen der zweiten Zylinderreferenzachse $\alpha_0^{(2)}$ und der zweiten sekundären Torsionsreferenzachse $\mathbf{e}_L^{(2,s)}$ um einen zweiten sekundären Torsionskorrekturwinkel $\psi_\Delta^{(2,s)}\left(\mathbf{e}_{\zeta,k}^{(1,s)}\right)$ abweicht, welcher zumindest von der ermittelten korrespondierenden sekundären Blickrichtung $-\mathbf{e}_{\zeta,k}^{(1,s)}$ des ersten Auges abhängt, d.h. für zumindest zwei verschiedene korrespondierende sekundäre Blickrichtungen $-\mathbf{e}_{\zeta,k}^{(1,s)}$ des ersten Auges, wie sie sich beispielsweise für verschiedene im ersten primären Berechnungs- bzw. Optimierungsschritt ermittelte erste Brillengläser und/oder für verschiedene Gebrauchssituationen ergeben könnten, verschiedene Werte aufweist.

In einer anderen bevorzugten Ausführungsform umfasst der zweite sekundäre Berechnungs- bzw. Optimierungsschritt des zweiten Brillenglases ein Kopieren zumindest einer im ersten primären Berechnungs- bzw. Optimierungsschritt ermittelten Fläche des ersten Brillenglases. Dabei wird das zweite Brillenglas oder zumindest eine Fläche des zweiten Brillenglases insbesondere durch Kopieren und Spiegeln beispielsweise von Pfeilhöhen oder anderen Koeffizienten zur Beschreibung des ermittelten ersten Brillenglases bzw. der zumindest einen Fläche des ersten Brillenglases ermittelt. Dies ist insbesondere dann besonders vorteilhaft, wenn die Refraktionen der beiden Augen des Brillenträgers symmetrisch bzw. spiegelsymmetrisch sind bzw. symmetrische bzw. spiegelsymmetrische Defizite aufweisen. Ein solches Kopieren und Spiegeln kann insbesondere als letzter Berechungs- bzw. Optimierungsschritt von einer Vielzahl von sukzessiv ausgeführten Berechnungs- bzw. Optimierungsschritten durchgeführt werden.

In einer weiteren bevorzugten Ausführungsform umfasst das Verfahren:

- einen primären Berechnungs- bzw. Optimierungsschritt des zweiten Brillenglases (daher auch als zweiter primärer Berechnungs- bzw. Optimierungsschritt bezeichnet) in Abhängigkeit von Startvorgaben für das erste Brillenglas; und
- einen sekundären Berechnungs- bzw. Optimierungsschritt des ersten Brillenglases (daher auch als erster sekundärer Berechnungs- bzw. Optimierungsschritt bezeichnet) in Abhängigkeit von dem im zweiten primären Berechungs- bzw. Optimierungsschritt ermittelten ersten Brillenglas.

Besonders bevorzugt umfasst der zweite primäre Berechnungs- bzw. Optimierungsschritt:

-- Ermitteln einer primären Blickrichtung $-\mathbf{e}_\zeta^{(2,p)}$ des zweiten Auges (daher auch als zweite primäre Blickrichtung $-\mathbf{e}_\zeta^{(2,p)}$ bezeichnet) für zumindest eine primäre Bewertungsstelle $i_b^{(2,p)}$ des zweiten Brillenglases (daher auch als zweite primäre Bewertungsstelle $i_b^{(2,p)}$ bezeichnet); und

-- Ermitteln einer in der bestimmten Gebrauchssituation zur primären Blickrichtung $-\mathbf{e}_\zeta^{(2,p)}$ des zweiten Auges

korrespondierenden primären Blickrichtung $-\mathbf{e}_{\zeta,k}^{(1,p)}$ des ersten Auges (daher auch als korrespondierende erste primäre Blickrichtung $-\mathbf{e}_{\zeta,k}^{(1,p)}$ bezeichnet) des Brillenträgers in Abhängigkeit von Startvorgaben für das erste Brillenglas. Es wird also ein erstes, insbesondere noch nicht gemäß der vorliegenden Erfindung optimiertes Brillenglas als Startvorgabe verwendet, um auf Basis dieses vorgegebenen ersten Brillenglases für die bestimmte Gebrauchssituation die zur ermittelten primären Blickrichtung des zweiten Auges korrespondierende primäre Blickrichtung des ersten Auges zu ermitteln. Vorzugsweise geschieht dies unter Verwendung eines Ray-Tracing-Verfahrens. Damit wird insbesondere die lokale, also blickrichtungsabhängige prismatische Wirkung des ersten und/oder zweiten Brillenglases in der bestimmten Gebrauchssituation für die Berechnung des zweiten Brillenglases berücksichtigt.

Vorzugsweise umfasst der zweite primäre Berechnungs- bzw. Optimierungsschritt außerdem ein Minimieren einer primären Zielfunktion für zumindest eine Fläche des zweiten Brillenglases (daher auch als zweite primäre Zielfunktion bezeichnet), wobei in der zweiten primären Zielfunktion für die zumindest eine primäre Bewertungsstelle $i_b^{(2,p)}$ des zweiten Brillenglases eine Korrektion einer zweiten primären transformierten astigmatischen Refraktion durch das zweite Brillenglas in der bestimmten Gebrauchssituation derart berücksichtigt wird, dass die zweite primäre transformierte astigmatische Refraktion in Bezug auf die primäre Blickrichtung des zweiten Auges den zweiten Zylinderwert und eine zweite primäre Zylinderkorrektionsachse $\alpha_K^{(2,p)}$ (d.h. Zylinderachse der Augenrefraktion bei Stellung des zweiten Auges in der zweiten primären Blickrichtung) aufweist bzw. durch den zweiten Zylinderwert und eine zweite primäre Zylinderkorrektionsachse $\alpha_K^{(2,p)}$ festgelegt ist, wobei die zweite primäre Zylinderkorrektionsachse $\alpha_K^{(2,p)}$ mit einer sowohl zur Referenzblickrichtung $-\mathbf{e}_z^{(2)}$ des zweiten Auges als auch zur primären Blickrichtung $-\mathbf{e}_\zeta^{(2,p)}$ des zweiten Auges senkrechten zweiten primären Torsionsreferenzachse $\mathbf{e}_L^{(2,p)}$ einen zweiten primären Korrektionstorsionswinkel $\psi_K^{(2,p)}$ einschließt, der von einem zweiten primären Referenztorsionswinkel $\psi_0^{(2,p)}$ zwischen der zweiten Zylinderreferenzachse $\alpha_0^{(2)}$ und der zweiten primären Torsionsreferenzachse $\mathbf{e}_L^{(2,p)}$ um einen zweiten primären Torsionskorrekturwinkel $\psi_\Delta^{(2,p)}\left(\mathbf{e}_{\zeta,k}^{(1,p)}\right)$ abweicht, welcher zumindest von der ermittelten korrespondierenden primären Blickrichtung $-\mathbf{e}_{\zeta,k}^{(1,p)}$ des ersten Auges abhängt, d.h. für zumindest zwei verschiedene korrespondierende primäre Blickrichtungen $-\mathbf{e}_{\zeta,k}^{(1,p)}$ des ersten Auges, wie sie sich beispielsweise für verschiedene Startvorgabe des ersten Brillenglases und/oder für verschiedene Gebrauchssituationen ergeben könnten, verschiedene Werte aufweist.

Alternativ oder zusätzlich umfasst der erste sekundäre Berechnungs- bzw. Optimierungsschritt vorzugsweise:

-- Ermitteln einer sekundären Blickrichtung $-\mathbf{e}_\zeta^{(1,s)}$ des ersten Auges (daher auch als erste sekundäre Blickrichtung $-\mathbf{e}_\zeta^{(1,s)}$ bezeichnet) für zumindest eine sekundäre Bewertungsstelle $i_b^{(1,s)}$ des ersten Brillenglases (daher auch als erste sekundäre Bewertungsstelle $i_b^{(1,s)}$ bezeichnet);

-- Ermitteln einer in der bestimmten Gebrauchssituation zur sekundären Blickrichtung $-\mathbf{e}_\zeta^{(1,s)}$ des ersten Auges korrespondierenden sekundären Blickrichtung $-\mathbf{e}_{\zeta,k}^{(2,s)}$ des zweiten Auges (daher auch als korrespondierende zweite sekundäre Blickrichtung $-\mathbf{e}_{\zeta,k}^{(2,s)}$ bezeichnet) des Brillenträgers in Abhängigkeit von dem im zweiten primären Berechungs- bzw. Optimierungsschritt ermittelten zweiten Brillenglas; und

-- Minimieren einer sekundären Zielfunktion für zumindest eine Fläche des ersten Brillenglases (daher auch als erste sekundäre Zielfunktion bezeichnet), wobei in der ersten sekundären Zielfunktion für die zumindest eine sekundäre Bewertungsstelle $i_b^{(1,s)}$ des ersten Brillenglases eine Korrektion einer ersten sekundären transformierten astigmatischen Refraktion durch das erste Brillenglas in der bestimmten Gebrauchssituation derart berücksichtigt wird, dass die erste sekundäre transformierte astigmatische Refraktion in Bezug auf die sekundäre Blickrichtung des ersten Auges den ersten Zylinderwert und eine erste sekundäre Zylinderkorrektionsachse $\alpha_K^{(1,s)}$ (d.h. Zylinderachse der Augenrefraktion bei Stellung des ersten Auges in der ersten sekundären Blickrichtung) aufweist bzw. durch den ersten Zylinderwert und eine erste sekundäre Zylinderkorrektionsachse $\alpha_K^{(1,s)}$ festgelegt ist, wobei die erste sekundäre Zylinderkorrektionsachse $\alpha_K^{(1,s)}$ mit einer sowohl zur Referenzblickrichtung $-\mathbf{e}_z^{(1)}$ des ersten Auges als auch zur sekundären Blickrichtung $-\mathbf{e}_\zeta^{(1,s)}$ des ersten Auges senkrechten ersten sekundären Torsionsreferenzachse $\mathbf{e}_L^{(1,s)}$ einen ersten sekundären Korrektionstorsionswinkel $\psi_K^{(1,s)}$ einschließt, der von einem ersten sekundären Referenztorsionswinkel $\psi_0^{(1,s)}$ zwischen der ersten Zylinderreferenzachse $\alpha_0^{(1)}$ und der ersten sekundären Torsionsreferenzachse $\mathbf{e}_L^{(1,s)}$ um einen ersten sekundären Torsionskorrekturwinkel $\psi_\Delta^{(1,s)}\!\left(\mathbf{e}_{\zeta,k}^{(2,s)}\right)$ abweicht, welcher zumindest von der ermittelten korrespondierenden sekundären Blickrichtung $-\mathbf{e}_{\zeta,k}^{(2,s)}$ des zweiten Auges abhängt, d.h. für zumindest zwei verschiedene korrespondierende sekundäre Blickrichtungen $-\mathbf{e}_{\zeta,k}^{(2,s)}$ des zweiten Auges, wie sie sich beispielsweise für verschiedene im zweiten primären Berechnungs- bzw. Optimierungsschritt ermittelten zweiten Brillengläser und/oder für verschiedene Gebrauchssituationen ergeben könnten, verschiedene Werte aufweist.

Vorzugsweise erfolgt die Berücksichtigung einer Augentorsion, insbesondere des ersten Auges, durch einen Torsionskorrekturwinkel $\psi_\Delta^{(1)}\!\left(\mathbf{e}_\zeta^{(2)}\right)$ , insbesondere den ersten primären Torsionskorrekturwinkel $\psi_\Delta^{(1,p)}\!\left(\mathbf{e}_{\zeta,k}^{(2,p)}\right)$, in der Zielfunktion eines Brillenglases, insbesondere in der ersten primären Zielfunktion. Damit lässt sich für das Brillenglaspaar eine besonders effiziente und genaue Optimierung für die bestimmte Gebrauchssituation erreichen. Ein Minimieren der Zielfunktion erfolgt dabei vorzugsweise durch ein Variieren zumindest einer Fläche des jeweiligen Brillenglases und ein Auswerten der optischen Eigenschaften des Brillenglases in der bestimmten Gebrauchssituation bis der Wert der Zielfunktion unter einen vorgegebenen Grenzwert gesunken ist oder bis sich der Wert der Zielfunktion zwischen aufeinanderfolgenden Auswerteschritten bzw. Rekursionsschritten nicht mehr oder um weniger als einen vorgegebenen Grenzwert ändert. Ein solcher Grenzwert kann als Abbruchkriterium für den Berechnungs- bzw. Optimierungsschritt festgelegt werden.

Bei einer vorgegebenen Gebrauchsstellung des ersten Brillenglases bzw. des Brillenglaspaares bzw. einer Brille für einen Brillenträger, d.h. bei vorgegebener Position des Brillenglases bzw. der Brillengläser vor den Augen des Brillenträgers und einem vorgegebenen Objektabstand, ergibt sich für viele Objektpunkte ein korrespondierendes Paar von Blickrichtungen des rechten und linken Auges, welche im allgemeinen nicht symmetrisch angeordnet sind und sich bei Blickbewegungen in Abhängigkeit von der Objektposition und abhängig vom ersten Brillenglas und evtl. einem zweiten Brillenglas verändern. Damit hängt nicht nur jede einzelne Blickrichtung, sondern insbesondere auch die Relation der beiden Blickrichtungen zueinander von der Gebrauchssituation und dem ersten und zweiten Brillenglas ab. Durch die bevorzugte Berücksichtigung des Einflusses der Gebrauchssituation auf die Torsion des ersten Auges auf Basis der in der Gebrauchssituation ermittelten korrespondierenden Blickrichtungen ist somit eine verbesserte Korrektion astigmatischer Refraktionen über einen weiten Nutzungsbereich eines Brillengases, insbesondere auch im Nahbereich erreichbar.

Die Gebrauchssituation legt dabei eine Positionierung des Brillenglases bzw. der Brillengläser vor den Augen des Brillenträgers und ein Objektabstandsmodell fest. Damit werden als Gebrauchssituation insbesondere Gebrauchsdaten bezüglich einer Positionierung der Brillengläser für einen Brillenträger und bezüglich einer Sehaufgabe des Brillenträgers erfasst oder bereitgestellt. Solche Gebrauchsdaten umfassen vorzugsweise Fassungsdaten, insbesondere bezüglich eines Kastenmaßes der Fassungsscheiben und/oder der Brückenweite und/oder eines Fassungsscheibenwinkels und/oder einer Vorneigung usw. der Brille. In einer bevorzugten Ausführungsform umfassen die Gebrauchsdaten bezüglich einer Sehaufgabe eine Vorgabe über hauptsächlich genutzte Blickwinkelbereiche und/oder hauptsächlich genutzte Objektentfernungen.

In jedem Fall legt die bestimmte Gebrauchssituation für eine Vielzahl von Blickrichtungen zumindest eines Auges des Brillenträgers die Position eines zugehörigen Objektpunkts derart eindeutig fest, dass damit auch der Sehstrahl des anderen Auges bei Betrachtung desselben Objektpunkts (in Abhängigkeit von der optischen Wirkung des zugehörigen Brillenglases) eindeutig festgelegt ist. Die beiden zu einem Objektpunkt gehörenden Sehstrahlen (für das rechte und linke Auge) werden dabei als korrespondierende Sehstrahlen bezeichnet. Entsprechende Durchtrittpunkte der korrespondierenden Sehstrahlen durch die beiden Brillengläser werden als korrespondierende Durchblickpunkte bezeichnet. Dabei kann jeder Durchblickpunkt auf der Vorder- und/oder Rückfläche eines Brillenglases eine Bewertungsstelle für das Brillenglas repräsentieren. Aufgrund der eindeutigen Zuordnung der Sehstrahlen und Objektpunkte zu den Durchblickstellen durch das entsprechend Brillenglas könnte die jeweilige Bewertungsstelle auch durch den entsprechenden Sehstrahl bzw. die Blickrichtung und/oder den Objektpunkt repräsentiert werden. In einer bevorzugten Ausführungsform werden die Bewertungsstellen eines Brillenglases durch zwei Koordinaten eines in Bezug auf das Brillenglas festgelegten Koordinatensystems dargestellt. Vorzugsweise wird dazu ein kartesisches x-y-z-Koordinatensystem festgelegt, dessen Ursprung beispielsweise im geometrischen Mittelpunkt (des ungerandeten bzw. rohrunden ersten oder zweiten Brillenglases) bzw. im Glasmittelpunkt des ersten bzw. zweiten Brillenglases insbesondere auf dessen Vorderfläche befindet, wobei sich die y-Achse in vertikaler Richtung in der Gebrauchsstellung bzw. Gebrauchssituation erstreckt und die z-Achse zum Auge hin weist. Damit lassen sich die Bewertungsstellen insbesondere durch die x-y-Koordinaten der Durchblickspunkte repräsentieren. Paare von Bewertungsstellen des rechten und linken Brillenglases, die korrespondierende Durchblickpunkte repräsentieren, werden als korrespondierende Bewertungsstellen bezeichnet. Die korrespondierenden Bewertungsstellen beziehen sich dabei auf einen gemeinsamen Objektpunkt, welcher von beiden Augen gleichzeitig betrachtet wird, weshalb die korrespondierenden Bewertungsstellen von der bestimmten Gebrauchssituation abhängig sind.

Je nach gewünschter Anwendung oder Zielsetzung kann das erste bzw. zweite Brillenglas bzw. das zu optimierende Brillenglaspaar bzw. können die Brillengläser des Brillenglaspaares für eine vorgegebene oder vorgebbare Gebrauchssituation eines durchschnittlichen oder eines individuell bestimmten Brillenträgers hergestellt bzw. optimiert werden.

Eine durchschnittliche Gebrauchssituation (wie in DIN 58 208 Teil 2 definiert) kann durch:

- Parameter eines Standardauges, wie z.B. dem sogenannten Gullstrand-Auge eines Brillenträgers (Augendrehpunkt, Eintrittspupille, und/oder Hauptebene, etc.);
- Parameter einer Standardgebrauchsstellung bzw. Anordnung des Brillenglaspaares vor den Augen des Brillenträgers (Fassungsscheibenwinkel, Vorneigung, Hornhautscheitelabstand, etc.); und/oder
- Parameter eines Standardobjektmodells bzw. Standardobjektentfernung

charakterisiert werden.

Die Gebrauchsposition kann beispielsweise anhand einer standardisierten Gebrauchsposition festgelegt werden. Bei Verwendung der Brillenfassung bzw. der Brille gemäß einer standardisierten Gebrauchsstellung beträgt der Augendrehpunktabstand etwa 27,4 mm oder etwa 27,9 mm oder etwa 28,5 mm oder etwa 28,8 mm, die Vorneigung, d.h. der pantoskopische Winkel beträgt etwa 8°, der Fassungsscheibenwinkel beträgt etwa 0°, die Pupillendistanz beträgt etwa 63 mm, der Hornhautscheitelabstand beträgt etwa 15 mm, die Objektentfernung im Fernbezugspunkt beträgt etwa 0 dpt und die Objektentfernung im Nahbezugspunkt beträgt etwa -2,5 dpt.

Insbesondere beträgt bei einer Verwendung der Brillenfassung bzw. der Brille gemäß einer standardisierten Gebrauchsstellung der Augendrehpunktabstand etwa 26,5 mm, die Vorneigung, d.h. der pantoskopische Winkel etwa 9°, der Fassungsscheibenwinkel etwa 5°, die Pupillendistanz etwa 64 mm und der Hornhautscheitelabstand beträgt etwa 13 mm.

Alternativ beträgt bei einer Verwendung der Brillenfassung bzw. der Brille gemäß einer standardisierten Gebrauchsstellung der Augendrehpunktabstand etwa 28,5 mm, die Vorneigung, d.h. der pantoskopische Winkel etwa 7°, der Fassungsscheibenwinkel etwa 0°, die Pupillendistanz etwa 63 mm und der Hornhautscheitelabstand beträgt etwa 15 mm.

Alternativ beträgt bei einer Verwendung der Brillenfassung bzw. der Brille gemäß einer standardisierten Gebrauchsstellung der Augendrehpunktabstand etwa 25 mm, die Vorneigung, d.h. der pantoskopische Winkel etwa 8°, der Fassungsscheibenwinkel etwa 5°, die Pupillendistanz etwa 64 mm und der Hornhautscheitelabstand beträgt etwa 13 mm.

Alternativ beträgt bei einer Verwendung der Brillenfassung bzw. der Brille gemäß einer standardisierten Gebrauchsstellung der Augendrehpunktabstand etwa 27,5 mm, die Vorneigung, d.h. der pantoskopische Winkel etwa 11°, der Fassungsscheibenwinkel etwa 0°, die Pupillendistanz etwa 65 mm und der Hornhautscheitelabstand beträgt etwa 14 mm.

Die folgenden numerischen Parameter charakterisieren beispielsweise eine durchschnittliche Gebrauchssituation:

Hornhautscheitelabstand (HSA) = 15,00 mm;
Vorneigung = 8,0 Grad;
Fassungsscheibenwinkel = 0,0 Grad;
Pupillendistanz = 63,0 mm;
Augendrehpunktabstand e = 28,5mm;
Objektabstandmodell: unendlicher Objektabstand im oberen Abschnitt des Brillenglases, welcher fließend in einen Objektabstand von -2,6 dpt bei x = 0 mm, y = -20 mm übergeht.

Alternativ können aber auch individuelle Parameter des Auges bzw. der Augen eines bestimmten Brillenträgers (Augendrehpunkt, Eintrittspupille, und/oder Hauptebene, etc.), der individuellen Gebrauchsstellung bzw. Anordnung vor den Augen des Brillenträgers (Fassungsscheibenwinkel, Vorneigung, Hornhautscheitelabstand, etc.) und/oder des individuellen Objektentfernungsmodells berücksichtigt werden.

Für schräge bzw. diagonale Blickrichtungen des ersten bzw. zweiten Auges wird dabei beispielsweise ein festes verkipptes Koordinatensystem beschrieben, in dem die Wellenfront dargestellt wird, und das in eine geeignete Verbindung mit dem Basiskoordinatensystem in Geradeausblickrichtung gebracht wird, auf welches sich vorzugsweise die Refraktionsdaten beziehen.

Vorzugsweise wird dieser Koordinatenübergang z.B. durch Helmholtz-Koordinaten $(\varphi, \vartheta, \psi,)$ geeignet beschrieben. In anderen bevorzugten Ausführungsformen könnte auch eine andere Darstellung, wie z.B. Fick-Koordinaten oder Euler-Winkel, verwendet werden. Im Folgenden wird eine bevorzugte Verwendung der Helmholtz-Koordinaten beispielhaft beschrieben. Dies kann für jedes Auge separat erfolgen, was durch einen hochgestellten Index "(1)" für das erste Auge bzw. "(2)" für das zweite Auge oder in einigen Beispielen auch durch einen hochgestellten Index "(l)" für das linke Auge bzw. "(r)" für das rechte Auge zum Ausdruck gebracht wird. Soweit nicht ausdrücklich zwischen dem ersten und dem zweiten Auge unterschieden wird, kann dieser Index der Einfachheit halber auch weggelassen werden.

Damit umfasst das Ermitteln der primären Blickrichtung $-\mathbf{e}_\zeta^{(1,p)}$ des ersten Auges vorzugsweise ein Ermitteln eines primären ersten Helmholtz-Winkels $\vartheta^{(1,p)}$ des ersten Auges und eines primären zweiten Helmholtz-Winkels $\varphi^{(1,p)}$ des ersten Auges für die zumindest eine erste primäre Bewertungsstelle $i_b^{(1,p)}$ des ersten Brillenglases derart, dass die Referenzblickrichtung $-\mathbf{e}_z^{(1)}$ des ersten Auges durch eine Kombination einer ersten Rotation des ersten Auges um eine zur Referenzblickrichtung $-\mathbf{e}_z^{(1)}$ des ersten Auges senkrechte, horizontale erste Drehachse $\mathbf{e}_x^{(1)}$ (insbesondere durch den Augendrehpunkt) des ersten Auges (erste Basisachse des ersten Auges) um den primären ersten Helmholtz-Winkel $\vartheta^{(1,p)}$ des ersten Auges und einer zweiten Rotation des ersten Auges um eine primäre zweite Drehachse $\mathbf{e}_{y,H}^{(1,p)}$ des ersten Auges um den primären zweiten Helmholtz-Winkel $\varphi^{(1,p)}$ des ersten Auges in die primäre Blickrichtung $-\mathbf{e}_\zeta^{(1,p)}$ des ersten Auges übergeht, wobei die primäre zweite Drehachse $\mathbf{e}_{y,H}^{(1)}$ des ersten Auges eine gegenüber einer zur Referenzblickrichtung $-\mathbf{e}_z^{(1)}$ des ersten Auges und zur ersten Drehachse $\mathbf{e}_x^{(1)}$ des ersten Auges senkrechten Achse $\mathbf{e}_y^{(1)}$ (insbesondere durch den Augendrehpunkt; zweite Basisachse des ersten Auges) um die erste Drehachse $\mathbf{e}_x^{(1)}$ des ersten Auges um den primären ersten Helmholtz-Winkel $\vartheta^{(1,p)}$ des ersten Auges rotierte Achse ist.

Vorzugsweise umfasst das Ermitteln der korrespondierenden primären Blickrichtung $-\mathbf{e}_{\zeta,k}^{(2,p)}$ des zweiten Auges ein Ermitteln eines korrespondierenden primären ersten Helmholtz-Winkels $\vartheta_k^{(2,p)}$ des zweiten Auges und eines korrespondierenden primären zweiten Helmholtz-Winkels $\varphi_k^{(2,p)}$ des zweiten Auges (insbesondere in Bezug auf eine Referenzblickrichtung $-\mathbf{e}_z^{(2)}$ des zweiten Auges) derart, dass die Referenzblickrichtung $-\mathbf{e}_z^{(2)}$ des zweiten Auges durch eine Kombination einer ersten Rotation des zweiten Auges um eine zur Referenzblickrichtung $-\mathbf{e}_z^{(2)}$ des zweiten

Auges senkrechte, horizontale erste Drehachse $\mathbf{e}_x^{(2)}$ (insbesondere durch den Augendrehpunkt) des zweiten Auges (erste Basisachse des zweiten Auges) um den korrespondierenden primären ersten Helmholtz-Winkel $\vartheta_k^{(2,p)}$ des zweiten Auges und einer zweiten Rotation des zweiten Auges um eine korrespondierende primäre zweite Drehachse $\mathbf{e}_{y,H,k}^{(2,p)}$ des zweiten Auges um den korrespondierenden primären zweiten Helmholtz-Winkel $\varphi_k^{(2,p)}$ des zweiten Auges in die korrespondierende primäre Blickrichtung $-\mathbf{e}_{\zeta,k}^{(2,p)}$ des zweiten Auges übergeht, wobei die korrespondierende primäre zweite Drehachse $\mathbf{e}_{y,H,k}^{(2,p)}$ des zweiten Auges eine gegenüber einer zur Referenzblickrichtung $-\mathbf{e}_z^{(2)}$ des zweiten Auges und zur ersten Drehachse $\mathbf{e}_x^{(2)}$ des zweiten Auges senkrechten Achse $\mathbf{e}_y^{(2)}$ (insbesondere durch Augendrehpunkt; zweite Basisachse des zweiten Auges) um die erste Drehachse $\mathbf{e}_x^{(2)}$ des zweiten Auges um den korrespondierenden primären ersten Helmholtz-Winkel $\vartheta_k^{(2,p)}$ des zweiten Auges rotierte Achse ist.

Diese für die primäre Blickrichtung des ersten Auges und die korrespondierende primäre Blickrichtung des zweiten Auges beschriebene Ermittlung von Helmhoftz-Winkeln ist in analoger Weise auch auf primäre Blickrichtungen des zweiten Auges sowie sekundäre, tertiäre, usw. Blickrichtungen des ersten und/oder zweiten Auges sowie die dazu korrespondierenden Blickrichtungen anwendbar. Damit umfasst das Ermitteln einer Blickrichtung $-\mathbf{e}_\zeta^{(i)}$ bzw. $-\mathbf{e}_{\zeta,k}^{(i)}$ eines Auges, insbesondere des ersten (i=1) und/oder zweiten (i=2) Auges, vorzugsweise ein Ermitteln eines ersten Helmholtz-Winkels $\vartheta^{(i)}$ des Auges und eines zweiten Helmholtz-Winkels $\varphi^{(i)}$ des Auges. Diese Winkel werden insbesondere derart ermittelt, dass die Referenzblickrichtung $-\mathbf{e}_z^{(i)}$ des Auges durch eine Kombination

-   einer ersten Rotation des Auges um eine erste Drehachse $\mathbf{e}_x^{(i)}$ (erste Basisachse des Auges) um den ersten Helmholtz-Winkel $\vartheta^{(i)}$ und

-   einer zweiten Rotation des Auges um eine zweite Drehachse $\mathbf{e}_{y,H}^{(i)}$ um den zweiten Helmholtz-Winkel $\varphi^{(i)}$

in die entsprechende Blickrichtung $-\mathbf{e}_\zeta^{(i)}$ bzw. $-\mathbf{e}_{\zeta,k}^{(i)}$ des Auges übergeht.

Dabei steht die erste Drehachse $\mathbf{e}_x^{(i)}$ zur Referenzblickrichtung $-\mathbf{e}_z^{(i)}$ des Auges senkrecht und verläuft in der bestimmten Gebrauchssituation (insbesondere bei der gewohnt geraden Kopfhaltung des Brillenträgers) horizontal durch den Augendrehpunkt des Auges. Die zweite Drehachse $\mathbf{e}_{y,H}^{(i)}$ des Auges ist dabei als eine Achse festgelegt, die sich aus einer zweiten Basisachse $\mathbf{e}_y^{(i)}$ des Auges durch Rotation um die erste Drehachse $\mathbf{e}_x^{(i)}$ des Auges um den ersten Helmholtz-Winkel $\vartheta^{(i)}$ des Auges ergibt, d.h. dass die um die erste Drehachse $\mathbf{e}_x^{(i)}$ des Auges um den ersten Helmholtz-Winkel $\vartheta^{(i)}$ des Auges gedrehte zweite Basisachse $\mathbf{e}_y^{(i)}$ mit der zweiten Drehachse $\mathbf{e}_{y,H}^{(i)}$ zusammenfällt. Die zweite Basisachse $\mathbf{e}_y^{(i)}$ des Auges wiederum steht sowohl zur Referenzblickrichtung $-\mathbf{e}_z^{(i)}$ des Auges als auch zur ersten Drehachse $\mathbf{e}_x^{(i)}$ des Auges senkrecht.

Es wird also vorzugsweise neben dem ortfesten Basiskoordinatensystem $(\mathbf{e}_x, \mathbf{e}_y, \mathbf{e}_z)$ ein augenfestes Koordinatensystem bzw. ein mitbewegtes Dreibein $(\mathbf{e}_{x,H}^{(i)}, \mathbf{e}_{y,H}^{(i)}, \mathbf{e}_{z,H}^{(i)})$ definiert, das aus den Basisvektoren des Basiskoordinatensystems durch Anwendung der Helmholz-Matrix $\mathbf{H}$ hervorgeht:

$$e_{x,H} = H(\vartheta, \varphi, \psi) \cdot e_x \qquad e_{y,H} = H(\vartheta, \varphi, \psi) \cdot e_y \qquad e_{z,H} = H(\vartheta, \varphi, \psi) \cdot e_z \qquad (1)$$

mit

$$H(\vartheta, \varphi, \psi) := H_x(\vartheta) H_y(-\varphi) H_z(\psi) \qquad (2)$$

wobei

$$H_x(\vartheta) := \begin{pmatrix} 1 & 0 & 0 \\ 0 & \cos\vartheta & -\sin\vartheta \\ 0 & \sin\vartheta & \cos\vartheta \end{pmatrix}$$

$$H_y(\varphi) := \begin{pmatrix} \cos\varphi & 0 & \sin\varphi \\ 0 & 1 & 0 \\ -\sin\varphi & 0 & \cos\varphi \end{pmatrix} \qquad (3)$$

$$H_x(\vartheta) := \begin{pmatrix} \cos\psi & -\sin\psi & 0 \\ \sin\psi & \cos\psi & 0 \\ 0 & 0 & 1 \end{pmatrix}$$

Die Winkel $\varphi$ und $\vartheta$ legen die Blickrichtung fest, während der Winkel $\psi$ die Torsionseinstellung des Auges beschreibt. Der Vektor $HS = e_\zeta^{(i)}$ (i=1,2) bezeichnet in der Definition des mitbewegten Dreibeins den Vektor des augenseitigen Hauptstrahls für das erste (i=1) bzw. zweite Auge (i=2). Da er mit der Blickrichtung fest gekoppelt ist, lassen sich aus dem Vektor HS die beiden Blickwinkel $\varphi$ und $\vartheta$ rekonstruieren nach

$$\varphi = -\arcsin HS_x, \qquad -\frac{\pi}{2} < \varphi < \frac{\pi}{2}$$
$$\vartheta = -\arctan\frac{HS_y}{HS_z}, \qquad -\frac{\pi}{2} < \vartheta < \frac{\pi}{2} \qquad (4)$$

Der dritte Winkel $\psi$ lässt sich hingegen nicht aus der Blickrichtung ableiten, sondern entsteht stattdessen durch eine geeignete Torsionseinstellung des Auges. Hierfür gibt es verschiedene physiologische Modelle. Beispielsweise wird ein Modell, bei dem gefordert wird, dass die Endposition des Auges dadurch festgelegt wird, dass das Auge aus der Nullblickrichtung durch Rotation um die Torsionsreferenzachse $e_L$ in die endgültige Position gebracht wird, wobei die Torsionsreferenzachse $e_L$ dadurch ausgezeichnet ist, dass sie ganz in einer Ebene liegt, die senkrecht zur Nullblickrichtung steht, als Listing'sches Modell oder Listing'sche Regel "L1" bzw. Listing'sche Regel für die Ferne bezeichnet, da sie nur für den Blick in die Ferne ein gute Näherung bietet. Insbesondere ist die Torsionsreferenzachse $e_L$ in Helmholtz-Koordinaten durch

$$e_L = \frac{1}{1 + \tan\frac{\varphi}{2}\tan\frac{\vartheta}{2}\tan\frac{\psi}{2}} \begin{pmatrix} \tan\frac{\vartheta}{2} - \tan\frac{\psi}{2}\tan\frac{\varphi}{2} \\ -\tan\frac{\varphi}{2} - \tan\frac{\vartheta}{2}\tan\frac{\psi}{2} \\ \tan\frac{\psi}{2} - \tan\frac{\varphi}{2}\tan\frac{\vartheta}{2} \end{pmatrix} \qquad (5)$$

gegeben, wobei die letzte Komponente der Achse $e_L$ verschwindet, also

$$\tan\frac{\psi}{2} - \tan\frac{\varphi}{2}\tan\frac{\vartheta}{2} = 0 \quad \Leftrightarrow \quad \psi_{Helmholtz}(\varphi,\vartheta) = 2\arctan\left(\tan\frac{\varphi}{2}\tan\frac{\vartheta}{2}\right) \qquad (6)$$

d.h. der Torsionswinkel $\psi$ lässt sich gemäß der Listing'schen Regel L1 als Funktion der Blickwinkel auffassen.

Vorzugsweise hängt der erste primäre Torsionskorrekturwinkel $\psi_\Delta^{(1,p)}\left(e_\zeta^{(1,p)}, e_{\zeta,k}^{(2,p)}\right)$ sowohl von der ermittelten primären Blickrichtung $-e_\zeta^{(1,p)}$ des ersten Auges als auch von der ermittelten korrespondierenden primären Blickrichtung $-e_{\zeta,k}^{(2,p)}$ des zweiten Auges ab. In analoger Weise hängt auch der zweite primäre Torsionskorrekturwinkel und/oder der erste und/oder zweite sekundäre und/oder tertiäre usw. Torsionskorrekturwinkel sowohl von der ermittelten Blickrichtung des jeweiligen Auges als auch von der ermittelten korrespondierenden Blickrichtung des jeweils anderen Auges ab. Insbesondere hängt der Torsionskorrekturwinkel vorzugsweise von den ersten und zweiten Helmholtzwinkeln des ersten und zweiten Auges ab.

Besonders bevorzugt gilt für den ersten Torsionskorrekturwinkel $\psi_\Delta^{(1)}$:

$$\psi_\Delta^{(1)} = \arctan\left(\tan\left(\frac{\vartheta_k^{(2)}}{2}\right)\cdot\tan\left(\frac{\varphi_k^{(2)}}{2}\right)\right) - \arctan\left(\tan\left(\frac{\vartheta^{(1)}}{2}\right)\cdot\tan\left(\frac{\varphi^{(1)}}{2}\right)\right) \qquad (7)$$

Dieser Wert ergibt sich insbesondere in der Helmholtz-Darstellung durch Bildung des Mittelwerts aus den Helmholtz-Torsionswinkels des ersten und zweiten Auges:

$$L2: \quad \psi_{korrigiert}^{(1)} = \psi_{korrigiert}^{(2)} = \frac{\psi_{Helmholtz}(\varphi^{(1)}, \vartheta^{(1)}) + \psi_{Helmholtz}(\varphi_k^{(2)}, \vartheta_k^{(2)})}{2} \qquad (8)$$

Vorzugsweise gilt alternativ oder zusätzlich für den zweiten Torsionskorrekturwinkel $\psi_\Delta^{(2)}$ in analoger Weise:

$$\psi_\Delta^{(2)} = \arctan\left(\tan\left(\frac{\vartheta_k^{(1)}}{2}\right)\cdot\tan\left(\frac{\varphi_k^{(1)}}{2}\right)\right) - \arctan\left(\tan\left(\frac{\vartheta^{(2)}}{2}\right)\cdot\tan\left(\frac{\varphi^{(2)}}{2}\right)\right).$$

Damit lässt sich bei einem sehr guten Optimierungsergebnis eine sehr schnelle Optimierung erreichen. In einer anderen, bevorzugten Ausführungsform gilt für den ersten Torsionskorrekturwinkel $\psi_\Delta^{(1)}$:

$$\psi_\Delta^{(1)} = 2 \cdot \arctan\left(\tan\left(\frac{\vartheta^{(1)} + \vartheta_k^{(2)}}{4}\right) \cdot \tan\left(\frac{\varphi^{(1)} + \varphi_k^{(2)}}{4}\right)\right) - 2 \cdot \arctan\left(\tan\left(\frac{\vartheta^{(1)}}{2}\right) \cdot \tan\left(\frac{\varphi^{(1)}}{2}\right)\right) \qquad (9).$$

Alternativ oder zusätzlich gilt für den zweiten Torsionskorrekturwinkel $\psi_\Delta^{(2)}$ vorzugsweise in analoger Weise:

$$\psi_\Delta^{(2)} = 2 \cdot \arctan\left(\tan\left(\frac{\vartheta^{(2)} + \vartheta_k^{(1)}}{4}\right) \cdot \tan\left(\frac{\varphi^{(2)} + \varphi_k^{(1)}}{4}\right)\right) - 2 \cdot \arctan\left(\tan\left(\frac{\vartheta^{(2)}}{2}\right) \cdot \tan\left(\frac{\varphi^{(2)}}{2}\right)\right).$$

Dieser Wert stellt eine weitere Vereinfachung und Beschleunigung der Berechnung dar und bietet eine sehr gute Näherung von Gl (8). Für verschiedene vertikale Prismendifferenzen unterscheiden sich die Ergebnisse der Formeln (8) und (9) um maximal 0,2° oder um maximal 2% des Absolutwertes der Torsionskorrektur gegenüber der gewöhnlichen Listing'schen Regel L1, wenn Blickwinkel bis zu 50° in Betracht gezogen werden. Falls die vertikale Prismendifferenz nicht über 3 cm/m liegt, so liegt der Approximationsfehler von Gl. (9) gegenüber Gl. (8) bei maximal 0,4°, aber auch nur bei extremen Blickwinkeln.

Gleichung (9) ergibt sich insbesondere aus dem Modell des Zyklopen-Auges durch Mittelung der Helmholtz-Winkel gemäß

$$\varphi^{(Z)} = \frac{\varphi + \varphi_k}{2} \quad , \quad \vartheta^{(Z)} = \frac{\vartheta + \vartheta_k}{2}$$

Und Auswertung von Gl. (8) für die gemittelten Werte:

$$L2: \qquad \psi_{korrigiert}^{(1)} = \psi_{korrigiert}^{(2)} \approx \psi_{Helmholtz}(\varphi^{(Z)}, \vartheta^{(Z)})$$

Die beschriebenen Modifikationen der Listing'schen Regeln L1 für die Ferne werden im folgenden auch als Listing'sche Regeln L2 für die Nähe bezeichnet.

Damit lassen sich Brillengläser ohne hohen technischen Aufwand optimieren, weil die Helmholtz-Torsionen einfach berechenbar sind. Außerdem ist die Erfindung ohne jede Einschränkung an die vertikalen Blickwinkel für links und rechts anwendbar.

In einer bevorzugten Ausführungsform wird während eines Berechnungs- bzw. Optimierungsschritts eines Brillenglases das andere Glas festgehalten. Bei der Auswertung der Zielfunktion zu jedem Punkt bzw. zu jeder Bewertungsstelle in der Zielfunktion des einen Glases wird der korrespondierende Durchblickpunkt des festgehaltenen Glases berechnet, damit daraus die Helmholtz-Winkel bestimmt werden können. Für die Berechnung von Soll-Ist-Abweichungen des Astigmatismus in der Zielfunktion wird dabei die Torsionsstellung des jeweiligen Auges vorzugsweise nach Gl. (8). oder Gl. (9) bestimmt.

Vorzugsweise umfasst das Verfahren ein Bereitstellen einer ersten bzw. zweiten Startfläche für die zumindest eine Fläche des ersten und/oder zweiten Brillenglases, wobei die Startfläche durch Minimieren einer monokularen Zielfunktion bestimmt ist, welche nicht von der korrespondierenden Blickrichtung $-e_{\xi,k}$ des anderen Auges abhängt. In einer bevorzugten Ausführungsform ist dabei der Torsionskorrekturwinkel gleich Null. In einer anderen bevorzugten Ausführungsform ist die Helrnholtztorsion gleich Null.

Vorzugsweise umfasst das Verfahren ein Festlegen zumindest eines Torsionskorrekturbereichs, insbesondere eines ersten und/oder zweiten Torsionskorrekturbereichs, des ersten bzw. zweiten Brillenglases, insbesondere des zu optimierenden und herzustellenden Brillenglaspaares, welcher eine Vielzahl von ersten bzw. zweiten Bewertungsstellen $i_b$ des jeweiligen Brillenglases umfasst, wobei das Ermitteln der ersten bzw. zweiten Blickrichtung -$\mathbf{e}_\xi$ für jede Bewertungsstelle $i$ des ersten bzw. zweiten Brillenglases und das Ermitteln der korrespondierenden Blickrichtung -$\mathbf{e}_{\xi,k}$ des jeweils anderen Auges zumindest für jede Bewertungsstelle $i_b$ des entsprechenden Torsionskorrekturbereichs erfolgt, und wobei in der Zielfunktion zumindest für jede *Bewertungsstelle $i_b$* des entsprechenden Torsionskorrekturbereichs eine Korrektion einer jeweiligen transformierten astigmatischen Refraktion durch das jeweilige Brillenglas in der bestimmten Gebrauchssituation derart berücksichtigt wird, dass der jeweilige Torsionskorrekturwinkel $\psi_\Delta(\mathbf{e}_{\xi,k})$ von der ermittelten korrespondierenden Blickrichtung -$\mathbf{e}_{\xi,k}$ abhängt.

Vorzugsweise umfasst der Torsionskorrekturbereich des ersten und/oder zweiten Brillenglases einen Nahbereich des Brillenglases zumindest teilweise. Besonders bevorzugt umfasst der Torsionskorrekturbereich einen Nahbezugspunkt des Brillenglases. Dabei umfasst der Torsionskorrekturbereich vorzugsweise einen Fernbereich des Brillenglases zumindest teilweise nicht. In einer bevorzugten Ausführungsform wird in der Zielfunktion für jede Bewertungsstelle $i$ des Brillenglases, welche nicht vom Torsionskorrekturbereich umfasst ist, eine Korrektion einer transformierten astigmatischen Refraktion durch das Brillenglas in der bestimmten Gebrauchssituation derart berücksichtigt, dass der Korrektionstorsionswinkel $\psi_k$ mit dem jeweiligen Referenztorsionswinkel $\psi_0$ übereinstimmt. In dieser Ausführungsform wird also außerhalb des festgelegten Torsionskorrekturbereichs auf Basis der Listing'schen Regel optimiert, wonach insbesondere eine Berücksichtigung der korrespondierenden Blickrichtung nicht erforderlich ist. Dies ist vor allem in einem Fernteil bzw. Fernbereich des Brillenglases bzw. Brillenglaspaares vorteilhaft, wo der Einfluss der Blickrichtung des zweiten Auges auf die Torsionslage des ersten Auges gering ist. Dadurch kann trotz Erreichens einer guten Korrektionsqualität des Brillenglaspaares die Optimierung und Herstellung schnell und effizient erfolgen, da der Rechenaufwand gering gehalten wird. Dabei hängt die Zielfunktion für Bewertungsstellen außerhalb des Torsionskorrekturbereichs und insbesondere zumindest teilweise für Bewertungsstellen des Fernbereichs nicht von der dazu korrespondierenden Blickrichtung des anderen Auges ab. Dies vereinfacht den bzw. die Berechnungs- bzw. Optimierungsschritt(e) und führt zu einer schnelleren Optimierung und Herstellung des Brillenglaspaares. Insbesondere ist für weit entfernte Objektpunkte ein Einfluss des zweiten Auges auf die Torsion des ersten Auges für die Fusion der Bilder für ein binokulares Sehen geringer als für weniger weit entfernte bzw. nahe Objektpunkte, weshalb im Fernbereich vorzugsweise zumindest teilweise auf eine binokulare Torsionskorrektur zugunsten einer schnelleren Optimierung und Herstellung des Brillenglaspaares verzichtet wird.

Insbesondere betrifft die Erfindung ein Verfahren zur Optimierung und Herstellung eines Brillenglaspaares bzw. eines Brillenglases für ein Paar von Brillengläsern zur Korrektion von Anisometropie. Insbesondere bei der Korrektion von Anisometropie treten vor allem bei nicht zentraler Blickrichtung oft deutliche prismatische Differenzen zwischen dem rechten und linken Brillenglas auf, was für diese Blickrichtungen bzw. die zugehörigen Bewertungsstellen einen deutlichen Einfluss auf die Konvergenzbewegung der Augen und damit auf die Torsionsstellung in der jeweiligen Bewertungsstelle hat. Erfindungsgemäß lässt sich somit insbesondere für solche Brillen eine deutliche Verbesserung der Abbildungsqualität bewirken.

Vorzugsweise wird für die zumindest eine Bewertungsstelle $i_b$ des ersten und/oder zweiten Brillenglases die in der bestimmten Gebrauchssituation zur ersten und/oder zweiten Blickrichtung -$\mathbf{e}_\xi$ korrespondierende Blickrichtung -$\mathbf{e}_{\xi,k}$ mittels Ray-Tracing unter Annahme der Orthotropie ermittelt.

Außerdem betrifft die Erfindung eine Verwendung eines gemäß einem der oben beschriebenen Verfahren hergestellten Brillenglases bzw. Brillenglaspaares in einer Brille zur Korrektion von Anisometropie.

Außerdem stellt die Erfindung ein Computerprogrammerzeugnis bereit, welches Programmteile enthält, welche ausgelegt sind, wenn geladen und ausgeführt auf einem Computer, ein Verfahren zur Optimierung zumindest eines ersten Brillenglases für eine bestimmte Gebrauchssituation zur Korrektion zumindest einer ersten astigmatischen Refraktion eines ersten Auges eines Brillenträgers, welche in einer Referenzblickrichtung $-\mathbf{e}_\zeta^{(1)}$ des ersten Auges eine erste Zylinderreferenzachse $\mathbf{a}_0^{(1)}$ aufweist, durchzuführen, wobei das Verfahren zumindest einen ersten primären Berechnungs- bzw. Optimierungsschritt des ersten Brillenglases umfasst, welcher umfasst:

-

Ermitteln einer primären Blickrichtung $-\mathbf{e}_\zeta^{(1,p)}$ des ersten Auges für zumindest eine primäre Bewertungsstelle $i_b^{(1,p)}$ des ersten Brillenglases;

-

Ermitteln einer in der bestimmten Gebrauchssituation zur primären Blickrichtung $-e_{\zeta}^{(1,p)}$ des ersten Auges korrespondierenden primären Blickrichtung $-e_{\zeta,k}^{(2,p)}$ eines zweiten Auges des Brillenträgers, insbesondere in Abhängigkeit von Startvorgaben für ein zweites Brillenglas, welches insbesondere zur Benutzung mit dem ersten Brillenglas in einem Brillenglaspaar vorgesehen ist; und

- Minimieren einer ersten primären Zielfunktion für zumindest eine Fläche des ersten Brillenglases, wobei in der ersten primären Zielfunktion für die zumindest eine primäre Bewertungsstelle $i_b^{(1,p)}$ des ersten Brillenglases eine Korrektion einer ersten primären transformierten astigmatischen Refraktion durch das erste Brillenglas in der bestimmten Gebrauchssituation derart berücksichtigt wird, dass die erste primäre transformierte astigmatische Refraktion von der ermittelten korrespondierenden primären Blickrichtung $-e_{\zeta,k}^{(2,p)}$ des zweiten Auges abhängt.

Vorzugsweise umfasst das Computerprogrammerzeugnis Programmteile, welche ausgelegt sind, wenn geladen und ausgeführt auf einem Computer, ein Verfahren gemäß der vorliegenden Erfindung oder einer bevorzugten Ausführungsform davon durchzuführen.

Außerdem stellt die Erfindung ein Speichermedium mit einem darauf gespeicherten Computerprogramm bereit, wobei das Computerprogramm ausgelegt ist, wenn geladen und ausgeführt auf einem Computer, ein Verfahren zur Optimierung zumindest eines ersten Brillenglases für eine bestimmte Gebrauchssituation zur Korrektion zumindest einer ersten astigmatischen Refraktion eines ersten Auges eines Brillenträgers, welche in einer Referenzblickrichtung $-e_z^{(1)}$ des ersten Auges eine erste Zylinderreferenzachse $a_0^{(1)}$ aufweist, durchzuführen, wobei das Verfahren zumindest einen ersten primären Berechnungs- bzw. Optimierungsschritt des ersten Brillenglases umfasst, welcher umfasst:

-

Ermitteln einer primären Blickrichtung $-e_{\zeta}^{(1,p)}$ des ersten Auges für zumindest eine primäre Bewertungsstelle $i_b^{(1,p)}$ des ersten Brillenglases;

-

Ermitteln einer in der bestimmten Gebrauchssituation zur primären Blickrichtung $-e_{\zeta}^{(1,p)}$ des ersten Auges korrespondierenden primären Blickrichtung $-e_{\zeta,k}^{(2,p)}$ eines zweiten Auges des Brillenträgers insbesondere in Abhängigkeit von Startvorgaben für ein zweites Brillenglas, welches insbesondere zur Benutzung mit dem ersten Brillenglas in einem Brillenglaspaar vorgesehen ist; und

- Minimieren einer ersten primären Zielfunktion für zumindest eine Fläche des ersten Brillenglases, wobei in der ersten primären Zielfunktion für die zumindest eine primäre Bewertungsstelle $i_b^{(1,p)}$ des ersten Brillenglases eine Korrektion einer ersten primären transformierten astigmatischen Refraktion durch das erste Brillenglas in der bestimmten Gebrauchssituation derart berücksichtigt wird, dass die erste primäre transformierte astigmatische Refraktion von der ermittelten korrespondierenden primären Blickrichtung $-e_{\zeta,k}^{(2,p)}$ des zweiten Auges abhängt.

Vorzugsweise ist auf dem Speichermedium Computerprogrammcode gespeichert, der ausgelegt ist, wenn geladen und ausgeführt auf einem Computer, ein Verfahren gemäß der vorliegenden Erfindung oder einer bevorzugten Ausführungsform davon durchzuführen.

Schließlich stellt die Erfindung eine Vorrichtung zur Herstellung zumindest eines ersten Brillenglases, insbesondere eines Brillenglaspaares, bereit, wobei die Vorrichtung Erfassungsmittel bzw. eine Erfassungseinheit zum Erfassen von Zieldaten eines Brillenglaspaares und Berechnungs- und Optimierungsmittel bzw. eine Berechnungs- und Optimierungseinheit zum Berechnen und Optimieren zumindest eines ersten Brillenglases umfasst. Insbesondere ist die Erfassungseinheit bzw. sind die Erfassungsmittel ausgelegt, Rezeptdaten, wie z.B. eine erste astigmatische Refraktion eines ersten Auges eines Brillenträgers, welche in einer Referenzblickrichtung $-e_z^{(1)}$ des ersten Auges einen ersten Zylinderwert und eine erste Zylinderreferenzachse $\alpha_0^{(1)}$ aufweist, und/oder eine zweite astigmatische Refraktion eines zweiten Auges des Brillenträgers, welche in einer Referenzblickrichtung $-\mathbf{e}_z^{(2)}$ des zweiten Auges einen zweiten Zylinderwert und eine zweite Zylinderreferenzachse $\alpha_0^{(2)}$ aufweist, zu erfassen. Vorzugsweise sind die Erfassungsmittel außerdem ausgelegt die bestimmte Gebrauchssituation zumindest teilweise zu erfassen bzw. festzulegen.

Die Berechnungs- und Optimierungsmittel sind dabei ausgelegt zum Berechnen und Optimieren zumindest eines ersten Brillenglases für die bestimmte Gebrauchssituation zur Korrektion zumindest der ersten astigmatischen Refraktion des ersten Auges des Brillenträgers, wobei das Berechnen und Optimieren derart erfolgt, dass es zumindest einen ersten primären Berechnungs- bzw. Optimierungsschritt des ersten Brillenglases umfasst, welcher umfasst:

-

Ermitteln einer primären Blickrichtung $-\mathbf{e}_\zeta^{(1,p)}$ des ersten Auges für zumindest eine primäre Bewertungsstelle $i_b^{(1,p)}$ des ersten Brillenglases;

-

Ermitteln einer in der bestimmten Gebrauchssituation zur primären Blickrichtung $-\mathbf{e}_\zeta^{(1,p)}$ des ersten Auges korrespondierenden primären Blickrichtung $-\mathbf{e}_{\zeta,k}^{(2,p)}$ eines zweiten Auges des Brillenträgers, insbesondere in Abhängigkeit von Startvorgaben für ein zweites Brillenglas, welches insbesondere zur Benutzung mit dem ersten Brillenglas in einem Brillenglaspaar vorgesehen ist; und

-- Minimieren einer ersten primären Zielfunktion für zumindest eine Fläche des ersten Brillenglases, wobei in der ersten primären Zielfunktion für die zumindest eine primäre Bewertungsstelle $i_b^{(1,p)}$ des ersten Brillenglases eine Korrektion einer ersten primären transformierten astigmatischen Refraktion durch das erste Brillenglas in der bestimmten Gebrauchssituation derart berücksichtigt wird, dass die erste primäre transformierte astigmatische Refraktion von der ermittelten korrespondierenden primären Blickrichtung $-\mathbf{e}_{\zeta,k}^{(2,p)}$ des zweiten Auges abhängt.

Vorzugsweise ist die Vorrichtung ausgelegt, ein Verfahren gemäß der vorliegenden Erfindung oder einer bevorzugten Ausführungsform davon durchzuführen.

Als Zielfunktion, insbesondere als erste und/oder zweite primäre und/oder sekundäre Zielfunktion, wird insbesondere eine Funktion $F$ mit folgendem funktionalen Zusammenhang zur sphärischen Wirkung S, zum Betrag der zylindrischen Wirkung $Z$ und zur Achslage des Zylinders $\alpha$ (auch als "SZA"-Kombination bezeichnet) berücksichtigt und minimiert:

$$F = \sum_{i=1}^m \left[ g_{i,S\Delta} \left( S_{\Delta,i} - S_{\Delta,i,Soll} \right)^2 + g_{i,Z\Delta} \left( Z_{\Delta,i} - Z_{\Delta,i,Soll} \right)^2 + ... \right].$$

Dabei werden in der Zielfunktion $F$ an den Bewertungsstellen $i$ des jeweiligen Brillenglases zumindest die tatsächlichen Refraktionsdefizite der sphärischen Wirkung $S_{\Delta,i}$ und der zylindrischen Wirkung $Z_{\Delta,i}$ sowie Sollvorgaben für die Refraktionsdefizite der sphärischen Wirkung $S_{\Delta,i,Soll}$ und der zylindrischen Wirkung $Z_{\Delta,i,Soll}$ berücksichtigt. Die jeweiligen Refraktionsdefizite an den jeweiligen Bewertungsstellen werden vorzugsweise mit Gewichtungsfaktoren $g_{i,S\Delta}$ bzw. $g_{i,Z\Delta}$ berücksichtigt. Dabei bilden die Sollvorgaben für die Refraktionsdefizite der sphärischen Wirkung $S_{\Delta,i,Soll}$ und/oder der zylindrischen Wirkung $Z_{\Delta,i,Soll}$ insbesondere zusammen mit den Gewichtungsfaktoren $g_{i,S\Delta}$ bzw. $g_{i,Z\Delta}$ das sogenannte Brillenglasdesign. Darüber hinaus können insbesondere auch weitere Residuen, insbesondere weitere zu optimierende Größen, wie z.B. Koma und/oder sphärische Aberration und/oder Prisma und/oder Vergrößerung und/oder anamorphotische Verzerrung, usw., berücksichtigt werden, was insbesondere durch den Ausdruck "+..." angedeutet ist.

Betrachtet man bei der Brillenglasdurchrechnung die Strahldurchrechnung durch einen bestimmten Durchblickspunkt *i*, also eine bestimmte Bewertungsstelle des entsprechenden Brillenglases, dann hat die Wellenfront an der Scheitelpunktkugel eine bestimmte SZA-Kombination. Ziel bei einer Brillenglasoptimierung ist, dass diese SZA-Kombination möglichst gut mit der SZA-Kombination der Refraktionsbestimmung bzw. im Sinne der vorliegenden Erfindung mit einer von der jeweiligen Blickrichtung abhängigen transformierten SZA-Kombination übereinstimmt. Da dies in der Regel nicht simultan an allen Durchblickspunkten *i* gleichzeitig gelingen kann, wird eine Zielfunktion aufgestellt, deren Minimierung einen möglichst passenden Kompromiss über alle Bewertungsstellen bzw. Durchblickspunkte *i* ergibt.

Eine bevorzugte prinzipielle Vorgehensweise zur Bestimmung des Refraktionsdefizits ist beispielsweise in Diepes H., Blendowske R. "Optik und Technik der Brille", Optische Fachveröffentlichung GmbH, Heidelberg (2002), insbesondere auf Seite 481 ff. beschrieben. Dazu betrachtet man die sogenannte Brechwertmatrix oder Vergenzmatrix **S**, welche wie folgt mit den Werten für die sphärischen Wirkung *S*, den Betrag der zylindrischen Wirkung *Z* und der Achslage des Zylinders $\alpha$ zusammenhängt:

$$\mathbf{S} = \begin{pmatrix} S_{xx} & S_{xy} \\ S_{xy} & S_{yy} \end{pmatrix} = \begin{pmatrix} \left(S + \dfrac{Z}{2}\right) - \dfrac{Z}{2}\cos 2\alpha & -\dfrac{Z}{2}\sin 2\alpha \\ -\dfrac{Z}{2}\sin 2\alpha & \left(S + \dfrac{Z}{2}\right) + \dfrac{Z}{2}\cos 2\alpha \end{pmatrix}$$

$$\Leftrightarrow \quad \begin{aligned} Z &= \sqrt{\left(S_{xx} - S_{yy}\right)^2 + 4S_{xy}^2} \\ S &= \frac{1}{2}\left(S_{xx} + S_{yy} - Z\right) \\ \tan\alpha &= \frac{S - S_{xx}}{S_{xy}} \end{aligned}$$

Die Vergenzmatrix **S** wird dabei einerseits für die SZA-Werte $S_{SK}$, $Z_{SK}$, $\alpha_{SK}$ an der Scheitelpunktpunktkugel und andererseits für die aus der Refraktionsbestimmung für das zumindest eine Auge des Brillenträgers erfindungsgemäß transformierten SZA-Werte $S_{Ref}$, $Z_{Ref}$, $\alpha_{Ref}$ bestimmt. Dadurch ergibt sich $\mathbf{S}_{SK}$ bzw. $\mathbf{S}_{Ref}$. Dabei beschreibt anschaulich gesprochen $\mathbf{S}_{SK}$ gewissermaßen die lokale Wirkung des Brillenglases, während $\mathbf{S}_{Ref}$ die für den Brillenträger in Idealfall gewünschte Wirkung beschreibt. Erfindungsgemäß werden dabei nicht unmittelbar die aus der Refraktionsbestimmung für den Brillenträger ermittelten SZA-Werte zur Bestimmung von $\mathbf{S}_{ref}$ verwendet, sondern die erfindungsgemäß für die jeweilige Blickrichtung des einen Auges in Abhängigkeit von der korrespondierenden Blickrichtung des anderen Auges transformierten SZA-Werte, also insbesondere die transformierte astigmatische Refraktion. Die Transformation betrifft dabei insbesondere die blickwinkelabhängige Bestimmung bzw. Festlegung des Winkels $\alpha_{Ref}$, welcher in der transformierten astigmatischen Refraktion die Lage der Zylinderkorrektionsachse beschreibt.

Für eine ideale Abbildung sollte gefordert werden, dass $\mathbf{S}_{SK}$ und $\mathbf{S}_{Ref}$ übereinstimmen, was aber nicht für alle Bewertungsstellen eines Brillenglases gleichzeitig erfüllbar ist. Somit bleibt als Differenzmatrix:

$$\begin{aligned} \mathbf{S}_\Delta &= \mathbf{S}_{SK} - \mathbf{S}_{Ref} \\ &= \begin{pmatrix} S_{SK,xx} & S_{SK,xy} \\ S_{SK,xy} & S_{SK,yy} \end{pmatrix} - \begin{pmatrix} S_{Ref,xx} & S_{Ref,xy} \\ S_{Ref,xy} & S_{Ref,yy} \end{pmatrix} \\ &= \begin{pmatrix} S_{SK,xx} - S_{Ref,xx} & S_{SK,xy} - S_{Ref,xy} \\ S_{SK,xy} - S_{Ref,xy} & S_{SK,yy} - S_{Ref,yy} \end{pmatrix} \end{aligned}$$

welche in der Regel von Null verschieden ist. Entsprechend der oben dargestellten Definition der Vergenzmatrix, werden der Differenzmatrix $\mathbf{S}_\Delta$ entsprechende SZA-Werte als Refraktionsdefizit zugeordnet:

$$Z_\Delta = \sqrt{\left(\left(S_{SK,xx} - S_{Ref,xx}\right) - \left(S_{SK,yy} - S_{Ref,yy}\right)\right)^2 + 4\left(S_{SK,xy} - S_{Ref,xy}\right)^2}$$

$$S_\Delta = \frac{1}{2}\left(\left(S_{SK,xx} - S_{Ref,xx}\right) + \left(S_{SK,yy} - S_{Ref,yy}\right) - Z_\Delta\right)$$

$$\tan \alpha_\Delta = \frac{S_\Delta - \left(S_{SK,xx} - S_{Ref,xx}\right)}{S_{SK,xy} - S_{Ref,xy}}$$

Da die Unschärfe der Abbildung bei nicht verschwindendem $S_\Delta$ insbesondere nur von $S_\Delta$ und $Z_\Delta$ abhängt (insbesondere aber nicht von $\alpha_\Delta$), wird eine Optimierung vorzugsweise nur nach $S_\Delta$ und $Z_\Delta$ durchgeführt. Die Achslage der Wellenfront und der transformierten Refraktion fließen dabei aber sehr wohl beide ein. Man beachte, dass, falls $S_\Delta$ = 0 und $Z_\Delta$ = 0 ist, gleichzeitig folgt, dass $S_{SK} = S_{Ref}$, $Z_{SK} = Z_{Ref}$ und $\alpha_{SK} = \alpha_{Ref}$ ist. Explizit gilt:

$$Z_\Delta = \sqrt{Z_{SK}^2 + Z_{Ref}^2 - 2 Z_{SK} Z_{Ref} \cos\left(2\left(\alpha_{SK} - \alpha_{Ref}\right)\right)}$$

$$S_\Delta = S_{SK} - S_{Ref} + \frac{1}{2}\left(\left(Z_{SK} - Z_{Ref}\right) - Z_\Delta\right)$$

Dies zeigt, wie die Achslage in die Größen $S_\Delta$ und $Z_\Delta$ eingeht. Eine Änderung der Achslage um den Winkel $\delta\alpha_{SK}$ bewirkt, dass $\cos(2(\alpha_{SK} + \delta\alpha_{SK} - \alpha_{Ref}))$ statt $\cos(2(\alpha_{SK} - \alpha_{Ref}))$ unter der Wurzel steht. Die Optimierung wird mit der Zielfunktion dadurch durchgeführt, dass vorzugsweise an jedem Durchblickspunkt $i$ jeder der Größen $S_{\Delta,i}$, $Z_{\Delta,i}$ ein Sollwert $S_{\Delta,i,Soll}$ bzw. $Z_{\Delta,i,Soll}$ zugeordnet wird und anschließend die Zielfunktion minimiert wird. Für weitere Hintergründe zum Verständnis dieser Vorgehensweise wird auf den Artikel von W. Becken, A. Seidemann, H. Altheimer, G. Esser und D. Uttenweiler "Brillengläser im Sport: Optimierung der Abbildungseigenschaften unter physiologischen Aspekten", Z. Med. Phys. 17 (2007), 56-66 hingewiesen.

Die Erfindung wird nachfolgend anhand von bevorzugten Ausführungsformen unter Bezugnahme auf die beigefügten Zeichnungen beschrieben. Dabei zeigen:

Fig. 1          eine Darstellung von Helmholtz-Koordinaten gemäß einer bevorzugten Ausführungsform der Erfindung;

Fig. 2A und 2B          schematische Darstellungen eines Augenpaars mit paralleler (Fig. 2A) bzw. konvergenter (Fig. 2B) erster und zweiter Blickrichtung;

Fig. 3          eine schematische Darstellung des Ablaufs eines Verfahrens zur Optimierung und Herstellung eines Brillenglaspaares gemäß einer ersten bevorzugten Ausführungsform der vorliegenden Erfindung;

Fig. 4          eine schematische Darstellung eines Verfahrnes gemäß einer zweiten bevorzugten Ausführungsform der vorliegenden Erfindung;

Fig. 5          eine schematische Darstellung eines Verfahrens gemäß einer dritten bevorzugten Ausführungsform der vorliegenden Erfindung;

Fig. 6A und 6B          Isoastigmatismuslinien der Fehlrefraktion eines ohne Berücksichtigung der Blickrichtung des anderen Auges optimierten Brillenglases bei einer Bewertung ohne (Fig. 6A) bzw. mit (Fig.6B) Berücksichtigung der Blickrichtung des anderen Auges;

Fig. 6C und 6D          Verlauf der Fehlrefraktion in Bezug auf die Brechkraft (linke Kurve) und den Astigmatismus (rechte Kurve) entlang der Hauptlinie von Fig. 6A (Fig. 6C) bzw. von Fig. 6B (Fig. 6D);

| Fig. 7A und 7B | Isoastigmatismuslinien der Fehlrefraktion eines ohne Berücksichtigung der Blickrichtung des anderen Auges optimierten Brillenglases (Fig. 7A) bzw. eines Brillenglases eines unter Berücksichtigung der Blickrichtung des anderen Auges erfindungsgemäß optimierten Brillenglaspaares (Fig. 7B) jeweils bei einer Bewertung mit Berücksichtigung der Blickrichtung des anderen Auges; |
| --- | --- |
| Fig. 7C und 7D | Verlauf der Fehlrefraktion in Bezug auf die Brechkraft (linke Kurve) und den Astigmatismus (rechte Kurve) entlang der Hauptlinie von Fig. 7A (Fig. 7C) bzw. von Fig. 7B (Fig. 7D); |
| Fig. 8 | eine Tabelle der Pfeilhöhen der Rückfläche des Brillenglases gemäß der bevorzugten Ausführungsform von Fig. 7; und |
| Fig. 9 | eine schematische Darstellung eines Beispiels einer bevorzugten Vorrichtung zum Optimieren bzw. Herstellen eines Brillenglases bzw. Brillenglaspaares gemäß der vorliegenden Erfindung. |

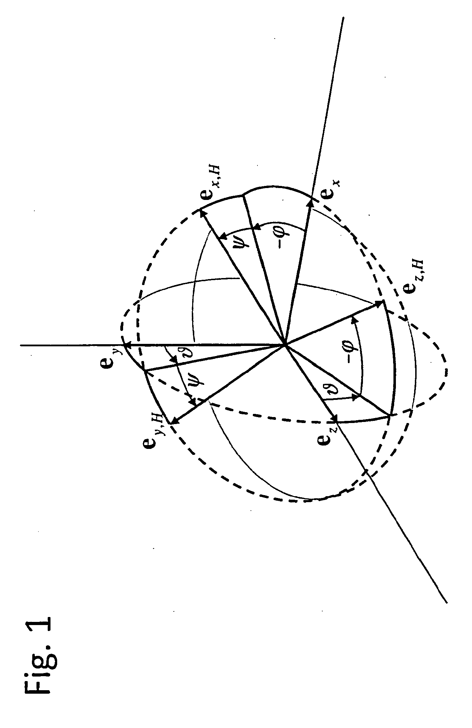

**Fig. 1** zeigt eine graphische Definition von Helmholtz-Koordinaten zur Optimierung eines Brillenglases bzw. Brillenglaspaares gemäß einer bevorzugten Ausführungsform der Erfindung. Dabei könnten für jedes der beiden Augen eigene Helmholtz-Koordinaten eingeführt werden. In Fig. 1 ist dies nur für ein Auge beispielhaft dargestellt. Das augenfeste Dreibein ($\mathbf{e}_{x,H},\mathbf{e}_{y,H},\mathbf{e}_{z,H}$) des Auges geht dabei aus dem raumfesten Dreibein ($\mathbf{e}_x,\mathbf{e}_y,\mathbf{e}_z$) durch folgende Schritte hervor:

1. Rotation um die x-Achse um den Winkel $\vartheta$ (erster Helmholtz-Winkel)
2. Rotation um die neue y-Achse um den Winkel - $\varphi$ (zweiter Helmholtz-Winkel)
3. Rotation um die neue z-Achse um den Winkel $\psi$ der Torsion

Vorzugsweise beschreibt die z-Achse $\mathbf{e}_z$ die Richtung des augenseitigen Hauptstrahls in der Referenzblickrichtung, während die gedrehte z-Achse die Richtung des augenseitigen Hauptstrahls in der ersten bzw. zweiten Blickrichtung repräsentiert.

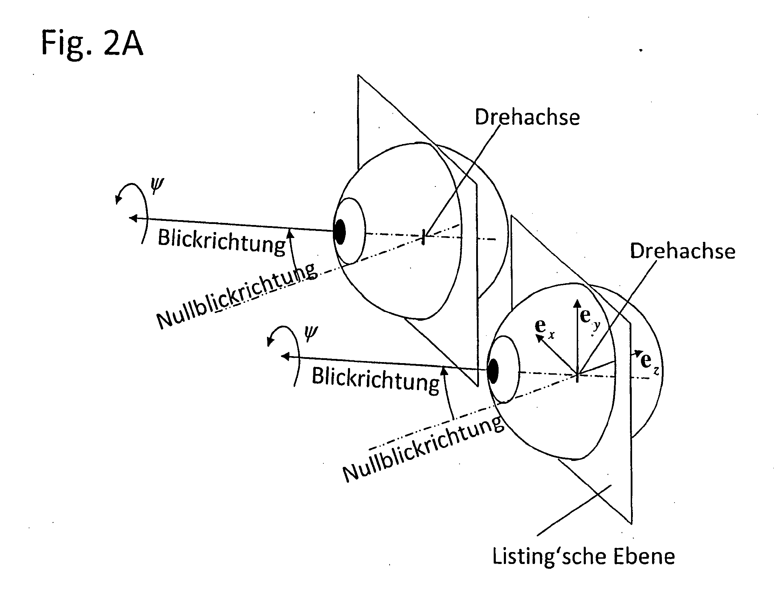

**Fig. 2A** veranschaulicht die Listing'sche Regel beim Blick in die Ferne. Beide Augen besitzen dieselben Blickwinkel $\vartheta$ und $\varphi$, und infolgedessen auch denselben Torsionswinkel $\psi_{Helmholtz}(\varphi, \vartheta)$ in der Helmholtz-Darstellung nach Gl. (6). Die Helmholtz-Koordinaten beziehen sich auf das raumfeste Dreibein ($\mathbf{e}_x,\mathbf{e}_y,\mathbf{e}_z$), das auch in Fig. 2A eingezeichnet ist.

Insbesondere für manche Blickrichtungen könnte es dazu kommen, dass die Torsionswinkel $\psi^{(l)}$ und $\psi^{(r)}$ in der Helmholzdarstellung für die beiden Augen verschieden sind, so dass die Einzelbilder nicht mehr auf korrespondierenden Netzhautstellen, sondern auf disparaten Netzhautstellen entstehen, die verdreht zueinander sind. Dadurch kommt es zu einem binokularen Doppelbild und Fusionsstörungen. Dieses Problem tritt insbesondere dann auf, wenn die augenseitigen Hauptstrahlen für das linke bzw. das rechte Auge verscheiden sind. Dies ist entweder bei einer Konvergenzbewegung der Fall oder kann auch durch Prismen im Glas hervorgerufen werden, die für beide Augen im benutzten Strahlengang verschieden sind.

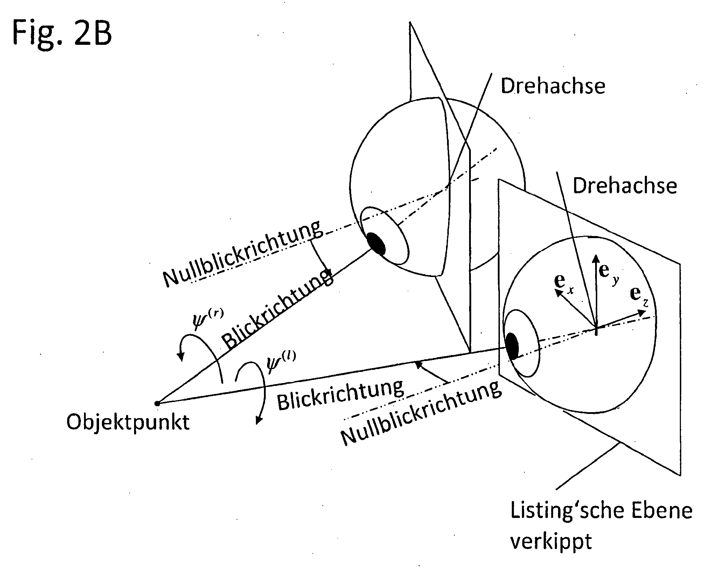

Die Torsionsbewegung der Augen weicht in einem solchen Fall von den Vorgaben der Listing'schen Regel L1 ab, wie z.B. in Fig. 2B dargestellt. Fig. 2B veranschaulicht eine Abwandlung der Listing'schen Regel gemäß einer bevorzugten Ausführungsform der Erfindung. Die Augen konvergieren, und daher besitzt das linke Auge ein anderes Blickwinkelpaar ($\varphi^{(l)},\vartheta^{(l)}$) als das rechte Auge, das durch ($\varphi^{(r)},\vartheta^{(r)}$) beschrieben wird. Entsprechend sind auch die Torsionswinkel $\psi_{Helmholtz}(\varphi,\vartheta)$ nach Gl. (6) verschieden, $\psi^{(l)}\neq\psi^{(r)}$. Die Helmholtz-Koordinaten beziehen sich auf das raumfeste Dreibein ($\mathbf{e}_x,\mathbf{e}_y,\mathbf{e}_z$), das auch in Fig.2B eingezeichnet ist.

Die Notwendigkeit dieser Torsionskorrektur wird auch als "Listing'sche Regel für die Nähe" oder "Listing'sche Regel 2 (L2)" bezeichnet. Die Größenordnung dieser Korrektur beträgt je nach Blickrichtung bis zu 4° bei einem Konvergenzwinkel von 30° und fällt erwartungsgemäß auf Null ab, falls der Konvergenzwinkel gegen Null geht (Blick nach Unendlich). Gemäß der vorliegenden Erfindung wird eine besonders effiziente und flexible Optimierung eines Brillenglaspaares für die Korrektion einer astigmatischen Refraktion unter Berücksichtigung dieser Winkelkorrektur bereitgestellt, welche insbesondere für den Blick in der Nähe und für prismatische Differenzen zweier Brillengläser einer Brille große Verbesserungen bietet.

Im folgenden Beispiel wird ein Objektpunkt betrachtet, der vom linken Auge aus gesehen in der schräg nach rechts unten verlaufenden Blickrichtung mit den Helmholtz-Blickwinkeln ($\varphi^{(l)},\vartheta^{(l)}$)=(25.0°,-30.0°) liegt und der vom linken Augendrehpunkt einen (negativ zu messenden) Objektabstand von $a1^{(l)}$=-400.0mm besitzen soll. Um seine kartesischen Koordinaten in dem in Fig. 2B eingezeichneten Koordinatensystem anzugeben, betrachten wir Gl. (2) für das linke Auge (der Index "(l)" bezeichnet dabei die Koordinaten des linken Auges). Die z-Achse des linken Auges, also $\mathbf{e}_{z,H}^{(l)}$, lautet

in raumfesten Koordinaten

$$\mathbf{e}_{z,H}^{(l)} = \mathbf{H}(\vartheta^{(l)},\varphi^{(l)},\psi^{(l)}) \cdot \mathbf{e}_z = \mathbf{H}(\vartheta,\varphi,\psi) \cdot \begin{pmatrix} 0 \\ 0 \\ 1 \end{pmatrix} = \begin{pmatrix} -\sin\varphi^{(l)} \\ -\cos\varphi^{(l)}\sin\vartheta^{(l)} \\ \cos\varphi^{(l)}\cos\vartheta^{(l)} \end{pmatrix} = \begin{pmatrix} -0.42262 \\ 0.45315 \\ 0.78489 \end{pmatrix}, \qquad (11)$$

und damit ist der Objektpunkt selbst gegeben durch

$$(x,y,z) = a1^{(l)}\mathbf{e}_{z,H}^{(l)} = -400\text{mm} \cdot \begin{pmatrix} -0.42262 \\ 0.45315 \\ 0.78489 \end{pmatrix} = \begin{pmatrix} 169.05 \\ -181.26 \\ -313.95 \end{pmatrix}\text{mm} \qquad (12)$$

Nun sei außerdem das rechte Auge in der Entfernung der Pupillendistanz PD=64.0mm vom linken Auge entfernt. Man kann dann nachrechnen, dass der Objektpunkt vom rechten Auge um a1$^{(r)}$=-377.44mm entfernt ist und in der Richtung der Blickwinkel $(\varphi^{(r)},\vartheta^{(r)})$ = (16.16°,-30.0°) gesehen wird, denn der Objektpunkt hat vom rechten Auge aus gerechnet die absoluten Koordinaten

$$(x,y,z) = PD \cdot \mathbf{e}_x + a1^{(r)}\mathbf{e}_z^{Auge,(r)} = PD \cdot \mathbf{e}_x + a1^{(r)} \begin{pmatrix} -\sin\varphi^{(r)} \\ -\cos\varphi^{(r)}\sin\vartheta^{(r)} \\ \cos\varphi^{(r)}\cos\vartheta^{(r)} \end{pmatrix}, \qquad (13)$$

$$= 64.0\text{mm} \cdot \begin{pmatrix} 1 \\ 0 \\ 0 \end{pmatrix} - 377.44\text{mm} \cdot \begin{pmatrix} -0.27832 \\ 0.48024 \\ 0.83181 \end{pmatrix} = \begin{pmatrix} 169.05 \\ -181.26 \\ -313.95 \end{pmatrix}\text{mm}$$

was mit dem vom linken Auge aus gerechneten Punkt in Gl. (12) übereinstimmt.
Zur Anwendung der Listing-Regel hat man nun nach den Gln. (6,7) für die beiden Augen

$$\psi^{(l)} = -6.80°, \qquad\qquad \psi^{(r)} = -4.357° . \qquad\qquad (14)$$

Die Listing-Regel für die Nähe sagt nun aus, dass beide Augen statt der in Gl. (14) verschiedenen angegebenen Winkel den Mittelwert aus Gl. (8), also

$$L2: \qquad \psi_{korrigiert}^{(l)} = \psi_{korrigiert}^{(r)} = \frac{(-6.80°)+(-4.357°)}{2} = -5.578° \qquad (15)$$

als Torsionsstellung annehmen, das ist gegenüber der Listing-Regel für die Ferne eine Korrektur von 1.221°.
Bei diesem vereinfachten Beispiel sind die Einflüsse der Brillengläser beispielsweise durch ihre lokalen prismatischen Effekte nicht dargestellt. In einem bevorzugten Verfahren werden diese bei einer Ermittlung der jeweiligen Blickrichtungen, insbesondere bei der Ermittlung der korrespondierenden Blickrichtungen beispielsweise durch ein Ray-Tracing-Verfahren berücksichtigt.

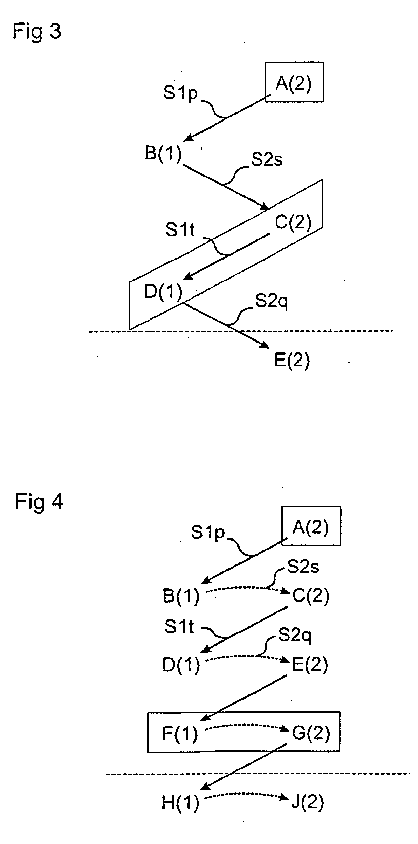

**Fig. 3** zeigt eine schematische Darstellung des Ablaufs eines Verfahrens zur Optimierung und Herstellung eines Brillenglaspaares gemäß einer ersten bevorzugten Ausführungsform der vorliegenden Erfindung. Dabei wird von einem Startglas A(2) in Form von Startvorgaben für das zweite Glas, beispielsweise das rechte Glas, ausgegangen.

Vorzugsweise ist dieses zweite Brillenglas A(2) bereits monokular zur Korrektion der Augenrefraktion des zweiten Auges des Brillenträgers optimiert. In einem ersten primären Berechnungs- bzw. Optimierungsschritt S1p wird nun das erste Brillenglas durch Minimieren einer ersten primären Zielfunktion optimiert, während das zweite Brillenglas A(2) festgehalten wird. In diesem Schritt S1p werden also nur die Parameter bzw. Freiheitsgrade des ersten Brillenglases variiert, um zu einem primär optimierten ersten Brillenglas B(1) zu kommen. Der numerische Aufwand ist somit vergleichbar mit einer herkömmlichen monokularen Optimierung. Durch die erfindungsgemäße Berücksichtung der Vorgaben des zweiten Brillenglases, insbesondere der prismatischen Wirkungen des zweiten Brillenglases, auf Grundlage der Ermittlung korrespondierender Blickrichtungen wird allerdings eine deutliche Verbesserung der binokularen Eigenschaften des Brillenglaspaares erreicht. Als Startglas für einen Variations- bzw. Optimierungsprozess des ersten Brillenglases im ersten primären Berechnungs- bzw. Optimierungsschritt könnte ein monokular optimiertes Glas dienen.

In der in Fig. 3 dargestellten, bevorzugten Ausführungsform wird in einem zweiten sekundären Berechnungs- bzw. Optimierungsschritt S2s ausgehend von dem im ersten primären Berechnungs- bzw. Optimierungsschritt S1p ermittelten ersten Brillenglas B(1) durch Minimieren einer zweiten sekundären Zielfunktion für das zweite Brillenglas ein sekundär optimiertes zweites Brillenglas C(2) ermittelt. Das primär optimierte erste Brillenglas B(1) wird dabei festgehalten. Damit ist der numerische Aufwand für den zweiten sekundären Berechnungs- bzw. Optimierungsschritt S2s wiederum vergleichbar mit einer herkömmlichen monokularen Optimierung. Als Startglas für einen Variations- bzw. Optimierungsprozess des zweiten Brillenglases im zweiten sekundären Berechnungs- bzw. Optimierungsschritt S2s könnte die Startvorgabe A(2) dienen, wobei das Glas nach einem geeigneten Algorithmus solange variiert wird, bis die zweite sekundäre Zielfunktion die gewünschte Konvergenz erreicht hat.

In Fig. 3 sind in analoger Weise als weitere Berechungs- bzw. Optimierungsschritte ein erster tertiärer Berechnungs- bzw. Optimierungsschritt S1t zum Ermitteln eines tertiär optimierten ersten Brillenglases D(1) abhängig vom sekundär optimierten zweiten Brillenglas C(2) und ein zweiter quartärer Berechnungs- bzw. Optimierungsschritt S2q zum Ermitteln eines quartär optimierten zweiten Brillenglases E(2) abhängig vom tertiär optimierten ersten Brillenglases D(1) dargestellt. Vorzugsweise wird mit zunehmender Anzahl dieser nacheinander ausgeführten Berechungs- bzw. Optimierungsschritte eine bessere binokulare Optimierung des Brillenglaspaares erreicht. Vorzugsweise wird ein Abbruchkriterium festgelegt, um damit festzulegen wann die Optimierung als ausreichend gut angesehen wird. In Fig. 3 wird die Optimierung beispielhaft nach dem ersten tertiären Berechnungs- bzw. Optimierungsschritt beendet. Die Brillenglaspaar [D(1);C(2)] bestehend aus dem tertiär optimierten ersten Brillenglas D(1) und dem sekundär optimierten zweiten Brillenglas C(2) wird anschließend für den Brillenträger gefertigt bzw. hergestellt.

**Fig. 4** zeigt eine schematische Darstellung eines Verfahrnes gemäß einer zweiten bevorzugten Ausführungsform der vorliegenden Erfindung. Dabei wird vorzugsweise ein erster primärer Berechnungs- bzw. Optimierungsschritt analog zu der in Fig. 3 gezeigten Ausführungsform durchgeführt. Gemäß der bevorzugten Ausführungsform von Fig. 4 umfasst ein zweiter sekundärer Berechnungs- bzw. Optimierungsschritt S2s allerdings ein Kopieren und Spiegeln des primär optimierten ersten Brillenglases B(1), um das sekundär optimierte zweite Brillenglas C(2) zu erhalten. Ein nachfolgender erster tertiärer Berechnungs- bzw. Optimierungsschritt S1t könnte wieder analog zu der in Fig. 3 dargestellten Ausführungsform durch Minimieren einer ersten tertiären Zielfunktion für zumindest eine Fläche des ersten Brillenglases unter Berücksichtigung des durch Kopieren und Spiegeln erhaltenen zweiten Brillenglases C(2) durchgeführt werden, um ein tertiär optimiertes erstes Brillenglas D(1) zu erhalten. Ein nachfolgender zweiter quartärer Berechnungs- bzw. Optimierungsschritt wird wiederum durch Kopieren und Spiegeln erreicht. Diese Ausführungsform eines erfindungsgemäßen Verfahrens ist besonders bevorzugt, wenn die Verordnungen des Brillenträgers für beide Augen symmetrisch bzw. spiegelsymmetrisch zueinander sind (Isometropie). Damit lässt sich eine besonders effiziente und schnelle Optimierung und Herstellung eines Brillenglaspaares [F(1);G(2)] mit geringem Rechenaufwand erreichen.

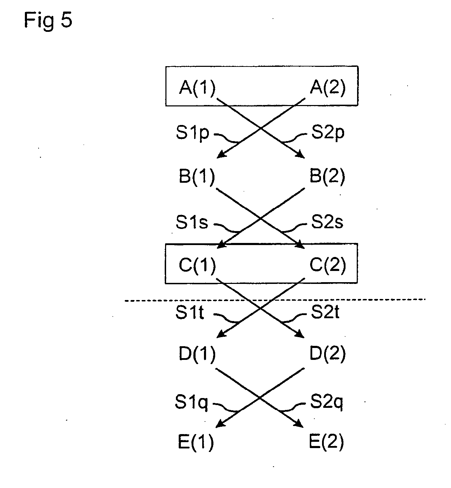

**Fig. 5** zeigt eine schematische Darstellung eines Verfahrens gemäß einer dritten bevorzugten Ausführungsform der vorliegenden Erfindung. Dabei wird vorzugsweise zusätzlich zu einem Startglas A(2) in Form von Startvorgaben für das zweite Glas, auch ein Startglas A(1) in Form von Startvorgaben für das erste Glas bereitgestellt. Vorzugsweise ist dieses erste Brillenglas A(1) bereits monokular zur Korrektion der Augenrefraktion des ersten Auges des Brillenträgers optimiert. Analog und evtl. sogar gleichzeitig zum ersten primären Berechnungs- bzw. Optimierungsschritt S1p, wie mit Bezug auf Fig. 3 bereits beschrieben, umfasst das hier gezeigte bevorzugte Verfahren einen zweiten primären Berechnungs- und Optimierungsschritt S2p, in dem das zweite Brillenglas durch Minimieren einer zweiten primären Zielfunktion optimiert wird, während das erste Brillenglas A(1) festgehalten wird. In diesem Schritt S2p werden also nur die Parameter bzw. Freiheitsgrade des zweiten Brillenglases variiert, um zu einem primär optimierten ersten Brillenglas B(1) zu kommen. Als Ausgangpunkt bzw. Startglas für einen Variations- bzw. Optimierungsprozess des zweiten Brillenglases im zweiten primären Berechnungs- bzw. Optimierungsschritt S2p könnte dabei das Glas A(2) dienen. Entsprechend könnte als Ausgangpunkt bzw. Startglas für einen Variations- bzw. Optimierungsprozess des ersten Brillenglases im ersten primären Berechnungs- bzw. Optimierungsschritt S1p das Glas A(1) dienen.

In einem ersten sekundären Berechnungs- bzw. Optimierungsschritt S1s wird ausgehend von dem im zweiten primären Berechnungs- bzw. Optimierungsschritt S2p ermittelten zweiten Brillenglas B(2) durch Minimieren einer ersten sekundären Zielfunktion für das erste Brillenglas ein sekundär optimiertes erstes Brillenglas C(1) ermittelt. Das primär optimierte zweite Brillenglas B(2) wird dabei festgehalten. In analoger Weise können weitere erste bzw. zweite tertiäre, quartäre, usw. Berechnungs- bzw. Optimierungsschritte durchgeführt werden, bis die jeweiligen Berechnungs- bzw. Optimierungsergebnisse D(1), D(2), E(1), E(2), usw. eine ausreichende Konvergenz erreicht haben. In der beispielhaften, bevorzugten Ausführungsform von Fig. 5 wird die Iteration nach Ermittlung des Brillenglaspaares [C(1);C(2)] abgebrochen und dieses Brillenglaspaar für den Brillenträger gefertigt bzw. hergestellt.

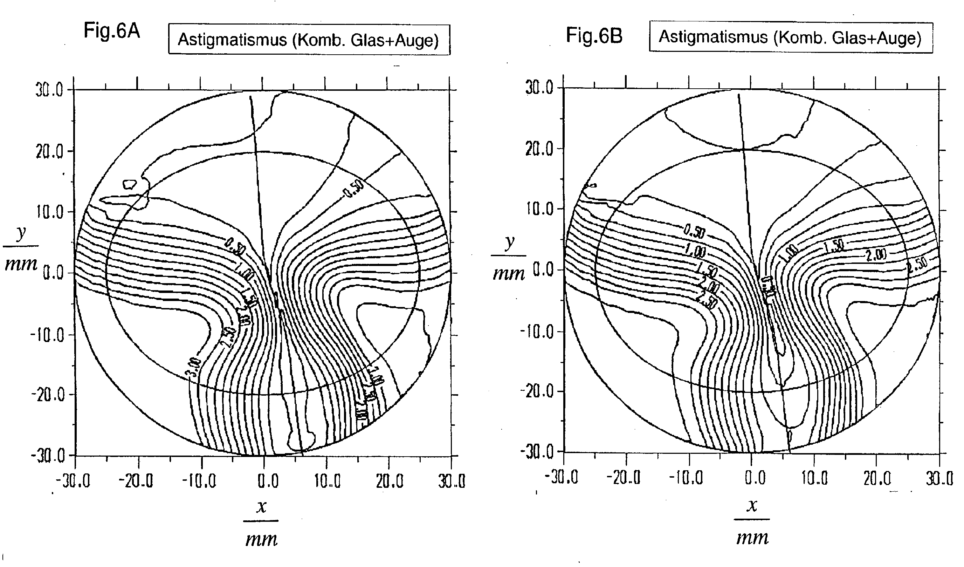

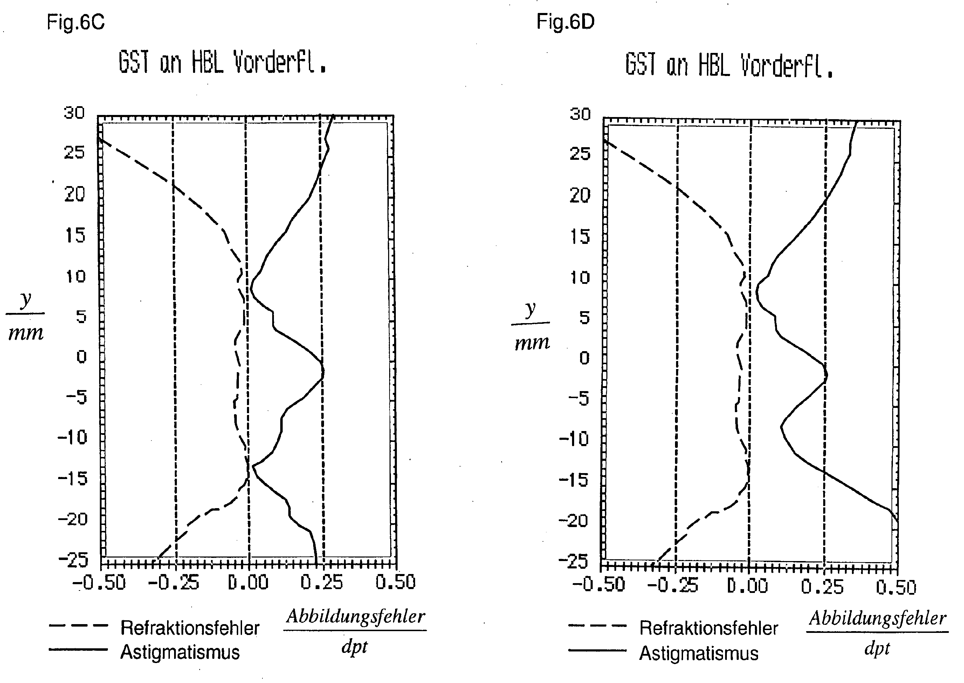

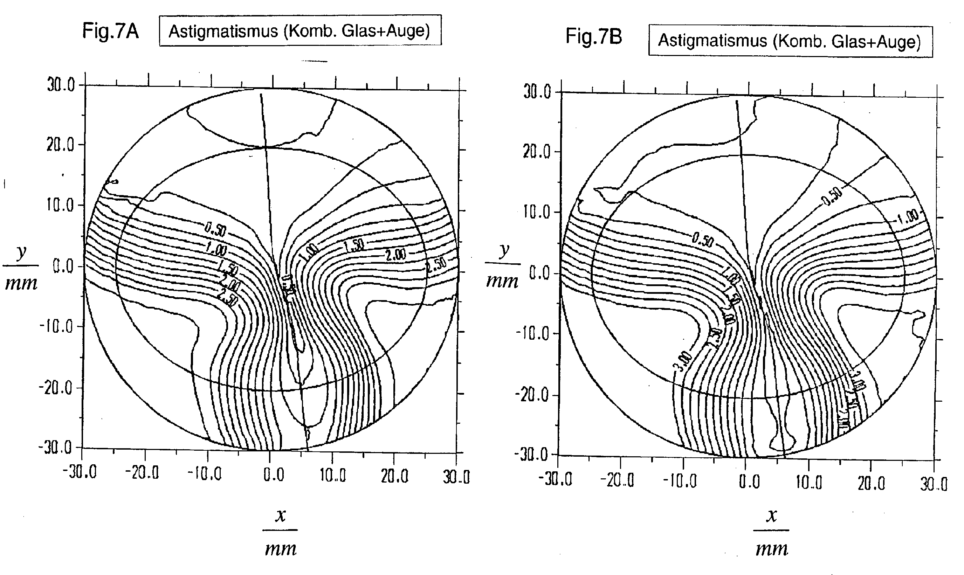

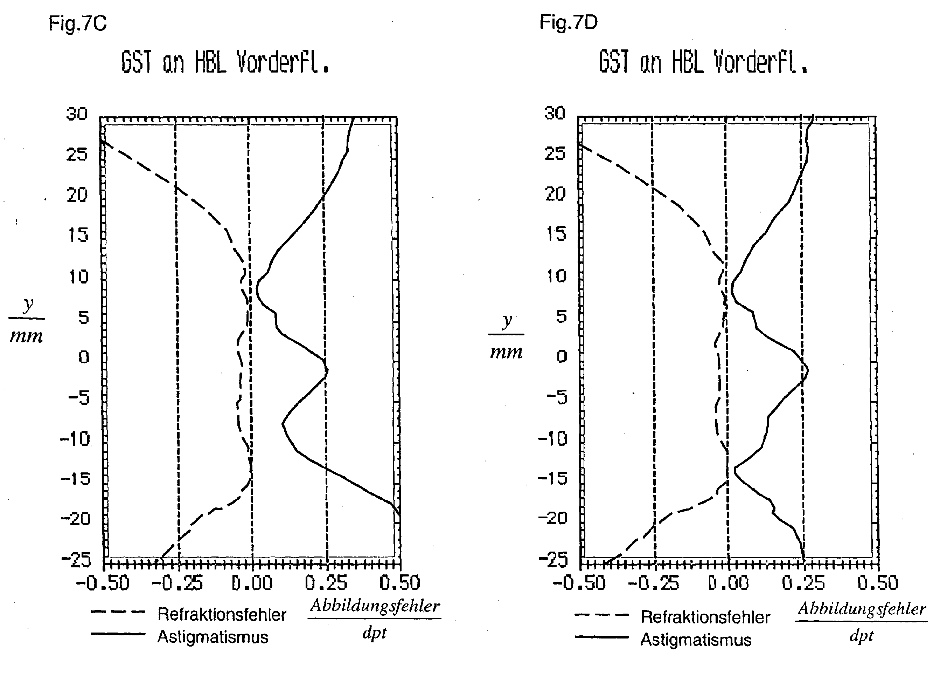

In **Fig. 6** und **Fig. 7** werden nun zwei verschiedene Brillengläser verglichen, wobei das erste (Fig. 6, Fig. 7A, Fig. 7C) nach der Listing'schen Regel L1 optimiert wurde und das zweite (Fig. 7B, Fig. 7D) ein Resultat eines ersten primären Berechnungs- bzw. Optimierungsschritts eines Verfahrens gemäß einer bevorzugten Ausführungsform der vorliegenden Erfindung unter Berücksichtigung der Listing'schen Regel L2 darstellt. Für die Optimierung und Herstellung der gezeigten Gläser werden als Verordnungswerte Sph = 2,0dpt, Zyl = 4,0dpt, A30° (Achslage = 30°), Add = 3,0 (Addition), Pr=0,0 (Prisma) angegeben. Insbesondere bei der Wahl eines hohen Zylinders und einer hohen Addition (und einem somit hohen Konvergenzwinkel) sind die Auswirkungen einer Torsionskorrektur besonders auffällig. Bei Berücksichtigung einer Abhängigkeit der Torsionsstellung des einen Auges von der Blickrichtung des anderen Auges in der Beschreibung der physiologischen Realität ergeben sich folgende Vergleiche:

a) Wechsel des Modells bei der Beurteilung: Man nimmt ein- und dasselbe L1-optimierte Glas. Es wird aber dann einmal mit dem Modell L1 (Fig. 6A, 6C) und andererseits mit dem Modell L2 (Fig. 6B, 6D) betrachtet bzw. beurteilt. Dabei ergibt sich, dass das mit L2 beurteilte L1-optimierte Glas etwas schlechter aussieht, d.h. höhere Astigmatismuswerte besitzt, als würde es mit L2 beurteilt.

b) Wechsel des Modells bei der Optimierung: Man betrachtet nun die zwei oben genannten verschiedenen L1- und L2- optimierten Gläser. Zur Beurteilung benutzt man aber in beiden Fällen das als zutreffend angenommene Modell L2 (Fig. 6B, 6D, 7B, 7D).

In Fig. 6 ist der Vergleich a) dargestellt, Links oben ist die gewöhnliche Astigmatismusbeurteilung dargestellt, die für ein L2-optimiertes Glas erscheint, falls L1 auch tatsächlich die Physiologie realistisch wiedergibt. Links unten ist der dazugehörige Brechkraft- und Astigmatismusverlauf an der Hauptlinie gezeigt. Falls aber alternativ die physiologische Realität durch L2 wiedergegeben wird, dann sieht dasselbe Glas ganz anders aus (siehe Fig. 6B und 6D), und zwar umso stärker, je stärker die Konvergenz ist. Da diese im Nahteil besonders zunimmt, wird dort der Fehlzylinder aufgrund der unpassenden Achslage besonders groß, und entsprechend wäre das Nahteil viel enger, als man nach L1 annehmen würde. Die Zunahme des Fehlzylinders mit zunehmender Blicksenkung ist entlang der Hauptlinie besonders gut zu erkenne, wohingegen der Verlauf des mittleren Brechwertes unverändert gegenüber der L1-Betrachtung erscheint (dies ist natürlich auch exakt zu erwarten, da bei ein- und demselben Glas eine geänderte relative Achslage der gekreuzten Zylinder von Glas und Auge keine Auswirkung auf den mittleren Brechwert hat).

Optimiert man nun nach dem Modell L2, so entsteht ein neues Glas, das in Fig. 7B und 7D dargestellt ist. In Fig. 7A und 7C sind zum Vergleich noch einmal die gleichen Graphiken gezeigt wie in Fig. 6B und 6D für das mit L2 betrachtete L1-optimierte Glas. Man erkennt, dass der L2-beurteilte Astigmatismus an der Hauptlinie durch die L2-Optimierung wieder einen ähnlich guten Verlauf annimmt wie der L1-beurteilte Wert des L1-Glases.

Die Tatsache, dass die gezeigten Vergleiche auch im Fernteil leichte Unterschiede aufweisen, rührt von den leichten prismatischen Differenzen her, die aufgrund des Wirkungsverlaufs in realen Strahlengängen auftreten.

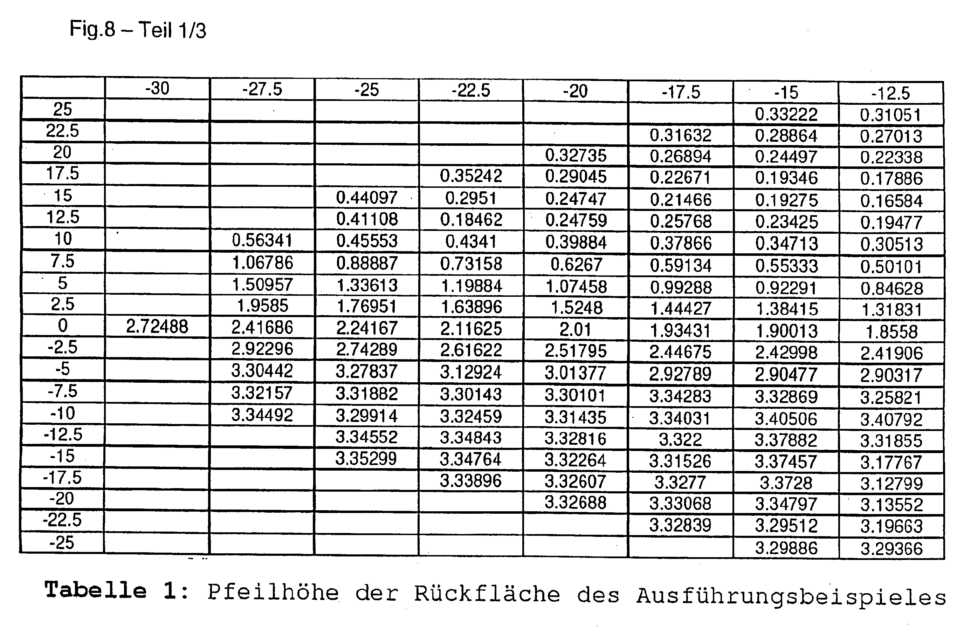

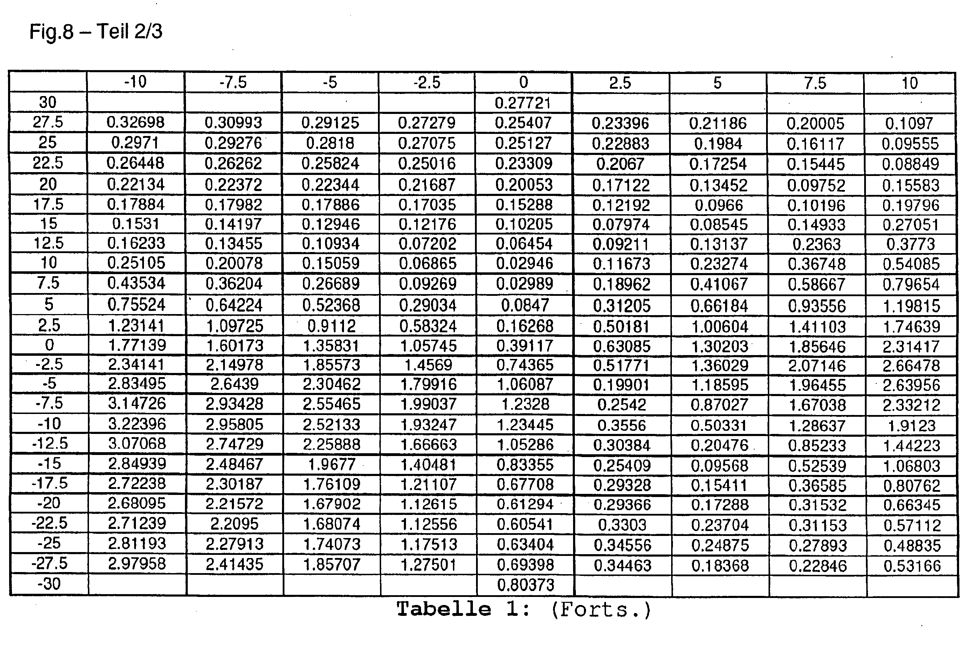

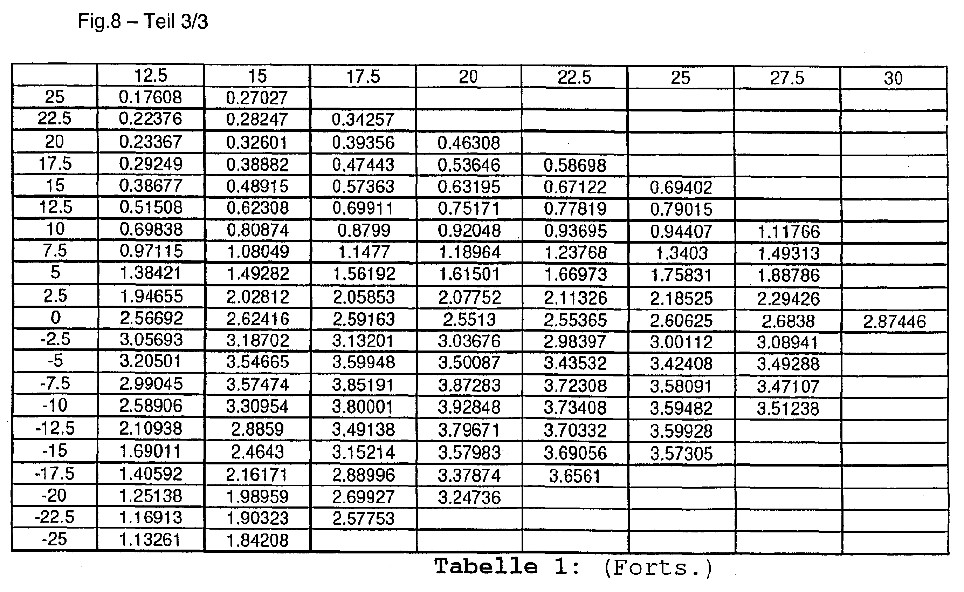

Das in den Abbildungen gezeigte Ausführungsbeispiel nach L2 ist in der Tabelle von **Fig. 8** für ein Glas offengelegt, das zu der rechts und links gleichen Verordnung Sph 2.0 dpt gehört. Dargestellt ist die optimierte Rückfläche, die Vorderfläche ist sphärisch mit einer Basiskurve von 8,5 dpt. Das Material besitzt eine Brechungsindex von n = 1,597, das Glas besitzt eine Mittendicke von 7,16 mm. Durch die Lage des Prismenbezugspunktes bei (x,y) = (0,0) in den in der Tabelle (jeweils erste Zeile bzw. Spalte jedes Tabellenabschnitts) angegebenen Koordinaten wird die Konstruktion des Glases eindeutig nachvollziehbar.

Das dargestellte Brillenglas ergibt sich dabei insbesondere nach einem einzigen primären Berechnungs- bzw. Optimierungsschritt. Für eine bevorzugte Optimierung und Herstellung eines Brillenglaspaares wird anschließend dieses Brillenglas festgehalten und das andere Brillenglas in entsprechender Weise in einem sekundären Berechungs- bzw. Optimierungsschritt ermittelt. Alternativ könnte das andere Brillenglas auch durch Kopien und Spiegeln des gezeigten Brillenglases ermittelt werden. In einer bevorzugten Ausführungsform wird dann wiederum dieses andere Brillenglas in der im sekundären Berechnungs- bzw. Optimierungsschritt oder durch Kopieren und Spiegeln erhaltenen Form festgehalten und in einem tertiären Berechnungs- bzw. Optimierungsschritt das eine (zuerst optimierte) Brillenglas weiter optimiert. Diese Berechnungs- bzw. Optimierungsschritte werden vorzugsweise so lange iteriert bzw. wiederholt bis eine ausreichende Konvergenz des Berechnungs- bzw. Optimierungsverfahrens erreicht ist, d.h. die Abweichungen aufeinanderfolgender Optimierungsergebnisse der einzelnen Brillengläser klein genug, insbesondere kleiner als eine vorgegebenen Grenze sind.

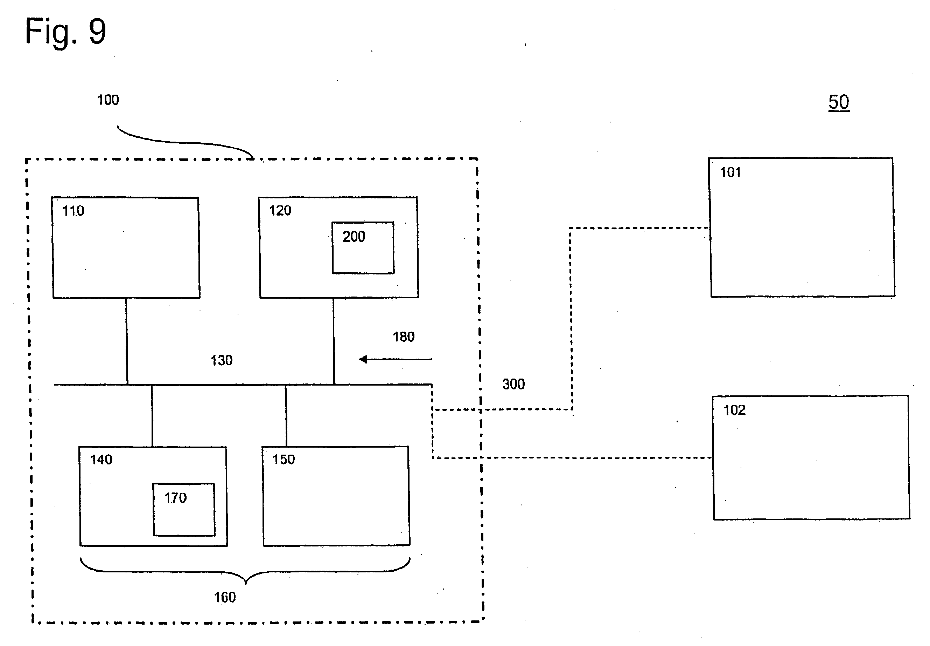

Wie schematisch in **Fig. 9** dargestellt ist, wird ferner ein Computerprogrammerzeugnis (d.h. ein in der Patentanspruchskategorie einer Vorrichtung beanspruchtes Computerprogramm) 200 bereitgestellt, welches derart ausgelegt ist, dass es - wenn geladen und ausgeführt auf einem geeigneten Computer 100 bzw. Netzwerk - ein Verfahren zur Optimierung bzw. Herstellung eines Brillenglaspaares zur Benutzung in einer Brille für eine bestimmte Gebrauchssituation durchführen kann. Das Computerprogrammerzeugnis 200 kann auf einem körperlichen Speichermedium bzw. Programmträger 120 gespeichert werden bzw. sein. Das Computerprogrammerzeugnis kann ferner als Programmsignal vorliegen.

Unter Bezugnahme auf Fig. 9 wird nachfolgend eine mögliche Computer- bzw. Netzwerkarchitektur beschrieben. Der Prozessor 110 des Computers 100 ist beispielsweise ein Zentralprozessor (CPU), ein Mikrocontroller (MCU), oder ein digitaler Signalprozessor (DSP). Der Speicher 120 symbolisiert Elemente, die Daten und Befehle entweder zeitlich begrenzt oder dauerhaft speichern. Obwohl zum besseren Verständnis der Speicher 120 als Teil des Computers 100 gezeigt ist, kann die Speicherfunktion an anderen Stellen, z.B. im Prozessor selbst (z.B. Cache, Register) und/oder auch im Netzwerk 300, beispielsweise in den Computern 101/102 implementiert werden. Der Speicher 120 kann ein Read-Only-Memory (ROM), ein Random-Access-Memory (RAM), ein programmierbares oder nichtprogrammierbares PROM oder ein Speicher mit anderen Zugriffsoptionen sein. Der Speicher 120 kann physisch auf einem computerlesbaren Programmträger, zum Beispiel auf:

(a) einem magnetischen Träger (Festplatte, Diskette, Magnetband);
(b) einem optischen Träger (CD-ROM, DVD);
(c) einem Halbleiterträger (DRAM, SRAM, EPROM, EEPROM);

implementiert bzw. gespeichert werden.

Wahlweise ist der Speicher 120 über verschiedene Medien verteilt. Teile des Speichers 120 können fest oder austauschbar angebracht sein. Zum Lesen und Schreiben benutzt der Computer 100 bekannte Mittel wie z.B. Diskettenlaufwerke, usw.

Der Speicher 120 speichert Unterstützungskomponenten wie zum Beispiel ein Bios (Basic Input Output System), ein Betriebssystem (OS), eine Programmbibliothek, einen Compiler, einen Interpreter und/oder ein Tabellen- bzw. Textverarbeitungsprogramm. Diese Komponenten sind zum besseren Verständnis nicht dargestellt. Unterstützungskomponenten sind kommerziell verfügbar und können auf dem Computer 100 von Fachleuten installiert bzw. in diesem implementiert werden.

Der Prozessor 110, der Speicher 120, die Eingabe- und die Ausgabevorrichtung sind über zumindest einen Bus 130 verbunden und/oder wahlweise über das (mono-, bi- bzw. multidirektionale) Netzwerk 300 (z.B. das Internet) angeschlossen bzw. stehen miteinander in Verbindung. Der Bus 130 sowie das Netzwerk 300 stellen logische und/oder physische Verbindungen dar, die sowohl Befehle als auch Datensignale übertragen. Die Signale innerhalb des Computers 100 sind überwiegend elektrische Signale, wohingegen die Signale im Netzwerk elektrische, magnetische und/oder optische Signale oder auch drahtlose Funksignale sein können.

Netzwerkumgebungen (wie das Netzwerk 300) sind in Büros, unternehmensweiten Computernetzwerken, Intranets und im Internet (d.h. World Wide Web) üblich. Die physische Entfernung zwischen den Computern im Netzwerk ist ohne Bedeutung. Das Netzwerk 300 kann ein drahtloses oder ein verdrahtetes Netzwerk sein. Als mögliche Beispiele für Implementierungen des Netzwerks 300 seien hier angeführt: ein lokales Netzwerk (LAN), ein kabelloses lokales Netzwerk (WLAN), ein Wide Area Network (WAN), ein ISDN-Netz, eine Infrarotverbindung (IR), eine Funkverbindung wie beispielsweise das Universal Mobile Telecommunication System (UMTS) oder eine Satellitenverbindung. Übertragungsprotokolle und Datenformate sind bekannt. Beispiele dafür sind: TCP/IP (Transmission Control Protocol/Internet Protocol), HTTP (Hypertext Transfer Protocol), URL (Unique Resource Locator), HTML (Hypertext Markup Language), XML (Extensible Markup Language), WML (Wireless Application Markup Language), Wireless Application Protocol (WAP) usw.

Die Eingabe- und Ausgabevorrichtungen können Teil einer Benutzerschnittstelle 160 sein. Die Eingabevorrichtung 140 steht für eine Vorrichtung, die Daten und Anweisungen zur Verarbeitung durch den Computer 100 bereitstellt. Beispielsweise ist die Eingabevorrichtung 140 eine Tastatur, eine Zeigevorrichtung (Maus, Trackball, Cursorpfeile), Mikrofon, Joystick, Scanner. Obwohl es sich bei den Beispielen allesamt um Vorrichtungen mit menschlicher Interaktion, vorzugsweise durch eine graphische Benutzerschnittstelle, handelt, kann die Vorrichtung 140 auch ohne menschliche Interaktion auskommen, wie zum Beispiel ein drahtloser Empfänger (z.B. mittels Satelliten- oder terrestrischer Antenne), ein Sensor (z.B. ein Thermometer), ein Zähler (z.B. ein Stückzahlzähler in einer Fabrik). Die Eingabevorrichtung 140 kann zum Lesen des Speichermediums bzw. Trägers 170 verwendet werden.

Die Ausgabevorrichtung 150 bezeichnet eine Vorrichtung, die Anweisungen und Daten anzeigt, die bereits verarbeitet wurden. Beispiele dafür sind ein Monitor oder eine anderer Anzeige (Kathodenstrahlröhre, Flachbildschirm, Flüssigkristallanzeige, Lautsprecher, Drucker, Vibrationsalarm). Ähnlich wie bei der Eingabevorrichtung 140 kommuniziert die Ausgabevorrichtung 150 bevorzugt mit dem Benutzer, vorzugsweise durch eine graphische Benutzerschnittstelle. Die Ausgabevorrichtung kann ebenfalls mit anderen Computern 101, 102, usw. kommunizieren.

Die Eingabevorrichtung 140 und die Ausgabevorrichtung 150 können in einer einzigen Vorrichtung kombiniert werden. Beide Vorrichtungen 140, 150 können wahlweise bereitgestellt werden.

Das Computerprogrammerzeugnis bzw. -produkt 200 umfasst Programminstruktionen und wahlweise Daten, die den Prozessor 110 unter anderem dazu veranlassen, die Verfahrensschritte des Verfahrens gemäß der Erfindung oder bevorzugte Ausführungsformen hiervon auszuführen. Mit anderen Worten definiert das Computerprogramm 200 die Funktion des Computers 100 und dessen Interaktion mit dem Netzwerksystem 300. Das Computerprogrammerzeugnis 200 kann beispielsweise als Quellcode in einer beliebigen Programmiersprache und/oder als Binärcode in kompilierter Form (d.h. maschinenlesbarer Form) vorliegen. Ein Fachmann ist in der Lage, das Computerprogrammerzeugnis 200 in Verbindung mit jeder der zuvor erläuterten Unterstützungskomponente (z.B. Compiler, Interpreter, Betriebssystem) zu benutzen.

Obwohl das Computerprogrammerzeugnis 200 als im Speicher 120 gespeichert dargestellt ist, kann das Computerprogrammerzeugnis 100 aber auch an beliebig anderer Stelle (z.B. auf dem Speichermedium bzw. Programmträger 170) gespeichert sein.

Das Speichermedium 170 ist beispielhaft als außerhalb des Computers 100 angeordnet dargestellt. Um das Computerprogrammerzeugnis 200 auf den Computer 100 zu übertragen, kann das Speichermedium 170 in das Eingabegerät 140 eingeführt werden. Das Speichermedium 170 kann als ein beliebiger, computerlesbarer Träger implementiert werden, wie zum Beispiel als eines der zuvor erläuterten Medien (vgl. Speicher 120). Das Programmsignal 180, welches vorzugsweise über das Netzwerk 300 zum Computer 100 übertragen wird, kann ebenfalls das Computerprogrammerzeugnis 200 beinhalten bzw. ein Teil hiervon sein.

Schnittstellen zum Koppeln der einzelnen Komponenten des Computersystems 50 sind ebenfalls bekannt. Zur Vereinfachung sind die Schnittstellen nicht dargestellt. Eine Schnittstelle kann beispielsweise eine serielle Schnittstelle, eine parallele Schnittstelle, ein Gameport, ein universeller serieller Bus (USB), ein internes oder externes Modem, ein Grafikadapter und/oder eine Soundkarte aufweisen.

Es ist insbesondere möglich, Rezeptdaten der Brillengläser vorzugsweise zusammen mit individuellen Daten des Brillenträgers (einschließlich der Daten der individuellen Gebrauchssituation) und/oder Daten des Brillenglases (Brechungsindex, Pfeilhöhen der Vorder- und Rückfläche) vorzugsweise per Datenfernübertragung an eine erfindungsgemäße Vorrichtung zur Herstellung eines Brillenglases zu übermitteln. Die Optimierung des Brillenglases erfolgt dann vorzugsweise aufgrund der übermittelten Rezeptdaten und individuellen Daten.

**Bezugszeichenliste**

| | |
|---|---|
| S1p, S2p | erster bzw. zweiter primärer Berechnungs- bzw. Optimierungsschritt |
| S1s, S2s | erster bzw. zweiter sekundärer Berechnungs- bzw. Optimierungsschritt |
| S1t, S2t | erster bzw. zweiter tertiärer Berechnungs- bzw. Optimierungsschritt |
| S1q, S2q | erster bzw. zweiter quartärer Berechnungs- bzw. Optimierungsschritt |
| A(1), A(2) | Startvorgabe für das erste bzw. zweite Brillenglas |
| B(1), B(2) | erstes bzw. zweites primär optimiertes Brillenglas |
| C(1), C(2) | erstes bzw. zweites sekundär optimiertes Brillenglas |
| D(1), D(2) | erstes bzw. zweites tertiär optimiertes Brillenglas |
| E(1), E(2) | erstes bzw. zweites quartär optimiertes Brillenglas |

| | |
|---|---|
| 50 | Computersystem |
| 100, 101, 102 | Computer |
| 110 | Prozessor |
| 120 | Speicher |
| 130 | Bus |
| 140 | Eingabevorrichtung |
| 150 | Ausgabevorrichtung |
| 160 | Benutzerschnittstelle |
| 170 | Speichermedium |
| 180 | Programmsignal |
| 200 | Computerprogrammerzeugnis |
| 300 | Netzwerk |

**Patentansprüche**

1.  Verfahren zur Optimierung und Herstellung zumindest eines ersten Brillenglases für eine bestimmte Gebrauchssituation zur Korrektion zumindest einer ersten astigmatischen Refraktion eines ersten Auges eines Brillenträgers, welche in einer Referenzblickrichtung $-\mathbf{e}_z^{(1)}$ des ersten Auges eine erste Zylinderreferenzachse $\boldsymbol{\alpha}_0^{(1)}$ aufweist, umfassend zumindest einen ersten primären Berechnungs- bzw. Optimierungsschritt des ersten Brillenglases, welcher umfasst:

   - Ermitteln einer primären Blickrichtung $-\mathbf{e}_\zeta^{(1,p)}$ des ersten Auges für zumindest eine primäre Bewertungsstelle $i_b^{(1,p)}$ des ersten Brillenglases;
   - Ermitteln einer in der bestimmten Gebrauchssituation zur primären Blickrichtung $-\mathbf{e}_\zeta^{(1,p)}$ des ersten Auges korrespondierenden primären Blickrichtung $-\mathbf{e}_{\zeta,k}^{(2,p)}$ eines zweiten Auges des Brillenträgers; und
   - Minimieren einer ersten primären Zielfunktion für zumindest eine Fläche des ersten Brillenglases, wobei in der ersten primären Zielfunktion für die zumindest eine primäre Bewertungsstelle $i_b^{(1,p)}$ des ersten Brillenglases eine Korrektion einer ersten primären transformierten astigmatischen Refraktion durch das erste Brillenglas in der bestimmten Gebrauchssituation derart berücksichtigt wird, dass die orientierung der zylinderachse der ersten primären transformierten astigmatischen Refraktion von der ermittelten korrespondierenden primären Blickrichtung $-\mathbf{e}_{\zeta,k}^{(2,p)}$ des zweiten Auges abhängt.

2.  Verfahren nach Anspruch 1, wobei in der ersten primären Zielfunktion für die zumindest eine primäre Bewertungsstelle $i_b^{(1,p)}$ des ersten Brillenglases die Korrektion einer ersten primären transformierten astigmatischen Refraktion durch das erste Brillenglas in der bestimmten Gebrauchssituation derart berücksichtigt wird, dass die erste primäre transformierte astigmatische Refraktion eine erste primäre Zylinderkorrektionsachse $\boldsymbol{\alpha}_K^{(1,p)}$ aufweist, welche mit einer sowohl zur Referenzblickrichtung $-\mathbf{e}_z^{(1)}$ des ersten Auges als auch zur primären Blickrichtung $-\mathbf{e}_\zeta^{(1,p)}$ des ersten Auges senkrechten ersten primären Torsionsreferenzachse $\mathbf{e}_L^{(1,p)}$ einen ersten primären Korrektionstorsionswinkel $\psi_K^{(1,p)}$ einschließt, der von einem ersten primären Referenztorsionswinkel $\psi_0^{(1,p)}$ zwischen der ersten Zylinderreferenzachse $\boldsymbol{\alpha}_0^{(1)}$ und der ersten primären Torsionsreferenzachse $\mathbf{e}_L^{(1,p)}$ um einen ersten primären Torsionskorrekturwinkel $\psi_\Delta^{(1,p)}\!\left(\mathbf{e}_{\zeta,k}^{(2,p)}\right)$ abweicht, welcher zumindest von der ermittelten korrespondierenden primären Blickrichtung $-\mathbf{e}_{\zeta,k}^{(2,p)}$ des zweiten Auges abhängt.

3.  Verfahren nach Anspruch 1 oder 2 zur Optimierung und Herstellung des ersten Brillenglases für ein Brillenglaspaar zur Benutzung zusammen mit einem zweiten Brillenglas des Brillenglaspaares in einer Brille für die bestimmte Gebrauchssituation, wobei das Ermitteln der korrespondierenden primären Blickrichtung $-\mathbf{e}_{\zeta,k}^{(2,p)}$ des zweiten Auges ein Ermitteln einer in der bestimmten Gebrauchssituation zur primären Bewertungsstelle $i_b^{(1,p)}$ des ersten Brillenglases korrespondierenden primären Bewertungsstelle $i_b^{(2,p)}$ des zweiten Brillenglases unter Berücksichtigung einer prismatischen Wirkung des zu optimierenden ersten Brillenglases und/oder des zweiten Brillenglases in der primären Bewertungsstelle des ersten bzw. zweiten Brillenglases in der bestimmten Gebrauchssituation umfasst, und wobei das Verfahren umfasst: ein Erfassen einer zweiten Zylinderreferenzachse $\boldsymbol{\alpha}_0^{(2)}$ einer zweiten

astigmatischen Refraktion des zweiten Auges in einer Referenzblickrichtung $-\mathbf{e}_z^{(2)}$ des zweiten Auges, wobei die erste primäre Zielfunktion für die zumindest eine Fläche des ersten Brillenglases von einer Korrektion einer zweiten primären transformierten astigmatischen Refraktion durch das zweite Brillenglas in der bestimmten Gebrauchssituation abhängt, wobei die zweite primäre transformierte astigmatische Refraktion eine zweite primäre Zylinderkorrektionsachse $\boldsymbol{\alpha}_K^{(2,p)}$ aufweist, welche mit einer sowohl zur Referenzblickrichtung $-\mathbf{e}_z^{(2)}$ des zweiten Auges als auch zur korrespondierenden primären Blickrichtung $-\mathbf{e}_{\zeta,k}^{(2,p)}$ des zweiten Auges senkrechten zweiten primären Torsionsreferenzachse $\mathbf{e}_L^{(2,p)}$ einen zweiten primären Korrektionstorsionswinkel $\psi_K^{(2,p)}$ einschließt, der von einem zweiten primären Referenztorsionswinkel $\psi_0^{(2,p)}$ zwischen der zweiten Zylinderreferenzachse $\boldsymbol{\alpha}_0^{(2)}$ und der zweiten primären Torsionsreferenzachse $\mathbf{e}_L^{(2,p)}$ um einen zweiten primären Torsionskorrekturwinkel $\psi_\Delta^{(2,p)}\!\left(\mathbf{e}_\zeta^{(1,p)}\right)$ abweicht, welcher zumindest von der ermittelten primären Blickrichtung $-\mathbf{e}_\zeta^{(1,p)}$ des ersten Auges abhängt.

4.  Verfahren nach einem der vorangegangen Ansprüche zur Optimierung und Herstellung des ersten Brillenglases für ein Brillenglaspaar zur Korrektion von Anisometropie.

5.  Verfahren zur Optimierung und Herstellung eines Brillenglaspaares ([D(1);C(2)]) für eine bestimmte Gebrauchssituation zur Korrektion einer ersten astigmatischen Refraktion eines ersten Auges eines Brillenträgers, welche in einer Referenzblickrichtung $-\mathbf{e}_z^{(1)}$ des ersten Auges eine erste Zylinderreferenzachse $\boldsymbol{\alpha}_0^{(1)}$ aufweist, mittels eines ersten Brillenglases des Brillenglaspaares und einer zweiten astigmatischen Refraktion eines zweiten Auges des Brillenträgers, welche in einer Referenzblickrichtung $-\mathbf{e}_z^{(2)}$ des zweiten Auges eine zweite Zylinderreferenzachse $\boldsymbol{\alpha}_0^{(2)}$ aufweist, mittels eines zweiten Brillenglases des Brillenglaspaares, umfassend:

- ein Verfahren zur Optimierung und Herstellung des ersten Brillenglases nach einem der vorangegangen Ansprüche; und
- einen zweiten sekundären Berechnungs- bzw. Optimierungsschritt des zweiten Brillenglases in Abhängigkeit von dem im ersten primären Berechungs- bzw. Optimierungsschritt ermittelten ersten Brillenglas,

wobei der zweite sekundäre Berechnungs- bzw. Optimierungsschritt umfasst:

-- Ermitteln einer sekundären Blickrichtung $-\mathbf{e}_\zeta^{(2,s)}$ des zweiten Auges für zumindest eine sekundäre Bewertungsstelle $i_b^{(2,s)}$ des zweiten Brillenglases;
-- Ermitteln einer in der bestimmten Gebrauchssituation zur sekundären Blickrichtung $-\mathbf{e}_\zeta^{(2,s)}$ des zweiten Auges korrespondierenden sekundären Blickrichtung $-\mathbf{e}_{\zeta,k}^{(1,s)}$ des ersten Auges des Brillenträgers in Abhängigkeit von dem im ersten primären Berechungs- bzw. Optimierungsschritt ermittelten ersten Brillenglas; und
-- Minimieren einer zweiten sekundären Zielfunktion für zumindest eine Fläche des zweiten Brillenglases, wobei in der zweiten sekundären Zielfunktion für die zumindest eine sekundäre Bewertungsstelle $i_b^{(2,s)}$ des zweiten Brillenglases eine Korrektion einer zweiten sekundären transformierten astigmatischen Refraktion durch das zweite Brillenglas in der bestimmten Gebrauchssituation derart berücksichtigt wird, dass die zweite sekundäre transformierte astigmatische Refraktion eine zweite sekundäre Zylinderkorrektionsachse $\boldsymbol{\alpha}_K^{(2,s)}$ aufweist, welche mit einer sowohl zur Referenzblickrichtung $-\mathbf{e}_z^{(2)}$ des zweiten Auges als auch zur sekundären Blick-

richtung $-\mathbf{e}_{\zeta}^{(2,s)}$ des zweiten Auges senkrechten zweiten sekundären Torsionsreferenzachse $\mathbf{e}_{L}^{(2,s)}$ einen zweiten sekundären Korrektionstorsionswinkel $\psi_{K}^{(2,s)}$ einschließt, der von einem zweiten sekundären Referenztorsionswinkel $\psi_{0}^{(2,s)}$ zwischen der zweiten Zylinderreferenzachse $\boldsymbol{\alpha}_{0}^{(2)}$ und der zweiten sekundären Torsionsreferenzachse $\mathbf{e}_{L}^{(2,s)}$ um einen zweiten sekundären Torsionskorrekturwinkel $\psi_{\Delta}^{(2,s)}\!\left(\mathbf{e}_{\zeta,k}^{(1,s)}\right)$ abweicht, welcher zumindest von der ermittelten korrespondierenden sekundären Blickrichtung $-\mathbf{e}_{\zeta,k}^{(1,s)}$ des ersten Auges abhängt.

6. Verfahren zur Optimierung und Herstellung eines Brillenglaspaares ([D(1);C(2)]) für eine bestimmte Gebrauchssituation zur Korrektur einer ersten astigmatischen Refraktion eines ersten Auges eines Brillenträgers, welche in einer Referenzblickrichtung $-\mathbf{e}_{z}^{(1)}$ des ersten Auges eine erste Zylinderreferenzachse $\boldsymbol{\alpha}_{0}^{(1)}$ aufweist, mittels eines ersten Brillenglases des Brillenglaspaares und einer zweiten astigmatischen Refraktion eines zweiten Auges des Brillenträgers, welche in einer Referenzblickrichtung $-\mathbf{e}_{z}^{(2)}$ des zweiten Auges eine zweite Zylinderreferenzachse $\boldsymbol{\alpha}_{0}^{(2)}$ aufweist, mittels eines zweiten Brillenglases des Brillenglaspaares, umfassend:

   - ein Verfahren zur Optimierung und Herstellung des ersten Brillenglases nach einem der vorangegangen Ansprüche; und
   - einen zweiten sekundären Berechnungs- bzw. Optimierungsschritt des zweiten Brillenglases in Abhängigkeit von dem im ersten primären Berechungs- bzw. Optimierungsschritt ermittelten ersten Brillenglas,

   wobei der zweite sekundäre Berechnungs- bzw. Optimierungsschritt des zweiten Brillenglases ein Kopieren zumindest einer im ersten primären Berechnungs- bzw. Optimierungsschritt ermittelten Fläche des ersten Brillenglases umfasst.

7. Verfahren nach Anspruch 5 oder 6, umfassend

   - einen zweiten primären Berechnungs- bzw. Optimierungsschritt des zweiten Brillenglases in Abhängigkeit von Startvorgaben für das erste Brillenglas; und
   - einen ersten sekundären Berechnungs- bzw. Optimierungsschritt des ersten Brillenglases in Abhängigkeit von dem im zweiten primären Berechungs- bzw. Optimierungsschritt ermittelten ersten Brillenglas,

   wobei der zweite primäre Berechnungs- bzw. Optimierungsschritt umfasst:

   -- Ermitteln einer primären Blickrichtung $-\mathbf{e}_{\zeta}^{(2,p)}$ des zweiten Auges für zumindest eine primäre Bewertungsstelle $i_{b}^{(2,p)}$ des zweiten Brillenglases;
   -- Ermitteln einer in der bestimmten Gebrauchssituation zur primären Blickrichtung $-\mathbf{e}_{\zeta}^{(2,p)}$ des zweiten Auges korrespondierenden primären Blickrichtung $-\mathbf{e}_{\zeta,k}^{(1,p)}$ des ersten Auges des Brillenträgers in Abhängigkeit von Startvorgaben für das erste Brillenglas; und
   -- Minimieren einer zweiten primären Zielfunktion für zumindest eine Fläche des zweiten Brillenglases, wobei in der zweiten primären Zielfunktion für die zumindest eine primäre Bewertungsstelle $i_{b}^{(2,p)}$ des zweiten Brillenglases eine Korrektur einer zweiten primären transformierten astigmatischen Refraktion durch das zweite Brillenglas in der bestimmten Gebrauchssituation derart berücksichtigt wird, dass die zweite primäre transformierte astigmatische Refraktion eine zweite primäre Zylinderkorrektionsachse $\boldsymbol{\alpha}_{K}^{(2,p)}$ aufweist, welche mit

einer sowohl zur Referenzblickrichtung $-\mathbf{e}_z^{(2)}$ des zweiten Auges als auch zur primären Blickrichtung $-\mathbf{e}_\zeta^{(2,p)}$ des zweiten Auges senkrechten zweiten primären Torsionsreferenzachse $\mathbf{e}_L^{(2,p)}$ einen zweiten primären Korrektionstorsionswinkel $\psi_K^{(2,p)}$ einschließt, der von einem zweiten primären Referenztorsionswinkel $\psi_0^{(2,p)}$ zwischen der zweiten Zylinderreferenzachse $\boldsymbol{\alpha}_0^{(2)}$ und der zweiten primären Torsionsreferenzachse $\mathbf{e}_L^{(2,p)}$ um einen zweiten primären Torsionskorrekturwinkel $\psi_\Delta^{(2,p)}\left(\mathbf{e}_{\zeta,k}^{(1,p)}\right)$ abweicht, welcher zumindest von der ermittelten korrespondierenden primären Blickrichtung $-\mathbf{e}_{\zeta,k}^{(1,p)}$ des ersten Auges abhängt; und/oder

wobei der erste sekundäre Berechnungs- bzw. Optimierungsschritt umfasst:

-- Ermitteln einer sekundären Blickrichtung $-\mathbf{e}_\zeta^{(1,s)}$ des ersten Auges für zumindest eine sekundäre Bewertungsstelle $i_b^{(1,s)}$ des ersten Brillenglases;

-- Ermitteln einer in der bestimmten Gebrauchssituation zur sekundären Blickrichtung $-\mathbf{e}_\zeta^{(1,s)}$ des ersten Auges korrespondierenden sekundären Blickrichtung $-\mathbf{e}_{\zeta,k}^{(2,s)}$ des zweiten Auges des Brillenträgers in Abhängigkeit von dem im zweiten primären Berechungs- bzw. Optimierungsschritt ermittelten zweiten Brillenglas; und

-- Minimieren einer ersten sekundären Zielfunktion für zumindest eine Fläche des ersten Brillenglases, wobei in der ersten sekundären Zielfunktion für die zumindest eine sekundäre Bewertungsstelle $i_b^{(1,s)}$ des ersten Brillenglases eine Korrektion einer ersten sekundären transformierten astigmatischen Refraktion durch das erste Brillenglas in der bestimmten Gebrauchssituation derart berücksichtigt wird, dass die erste sekundäre transformierte astigmatische Refraktion eine erste sekundäre Zylinderkorrektionsachse $\boldsymbol{\alpha}_K^{(1,s)}$ aufweist, welche mit einer sowohl zur Referenzblickrichtung $-\mathbf{e}_z^{(1)}$ des ersten Auges als auch zur sekundären Blickrichtung $-\mathbf{e}_\zeta^{(1,s)}$ des ersten Auges senkrechten ersten sekundären Torsionsreferenzachse $\mathbf{e}_L^{(1,s)}$ einen ersten sekundären Korrektionstorsionswinkel $\psi_K^{(1,s)}$ einschließt, der von einem ersten sekundären Referenztorsionswinkel $\psi_0^{(1,s)}$ zwischen der ersten Zylinderreferenzachse $\boldsymbol{\alpha}_0^{(1)}$ und der ersten sekundären Torsionsreferenzachse $\mathbf{e}_L^{(1,s)}$ um einen ersten sekundären Torsionskorrekturwinkel $\psi_\Delta^{(1,s)}\left(\mathbf{e}_{\zeta,k}^{(2,s)}\right)$ abweicht, welcher zumindest von der ermittelten korrespondierenden sekundären Blickrichtung $-\mathbf{e}_{\zeta,k}^{(2,s)}$ des zweiten Auges abhängt.

8. Verfahren nach einem der vorangegangenen Ansprüche, umfassend ein Bereitstellen einer ersten und/oder zweiten Startfläche für die zumindest eine Fläche des ersten bzw. zweiten Brillenglases, wobei die erste bzw. zweite Startfläche durch Minimieren einer monokularen Zielfunktion bestimmt ist, welche nicht von dem jeweils anderen Brillenglas abhängt.

9. Verfahren nach einem der vorangegangenen Ansprüche, umfassend ein Festlegen eines ersten und/oder zweiten Torsionskorrekturbereichs des ersten bzw. zweiten Brillenglases, welcher einen Nahbereich des ersten bzw. zweiten Brillenglases zumindest teilweise umfasst und welcher eine Vielzahl von ersten bzw. zweiten Bewertungsstellen $i_b$ des ersten bzw. zweiten Brillenglases umfasst, wobei das Ermitteln der ersten bzw. zweiten Blickrichtung $-\mathbf{e}_\zeta$ für jede Bewertungsstelle $i$ des erste bzw. zweiten Brillenglases und das Ermitteln der korrespondierenden Blickrichtung $-\mathbf{e}_{\zeta,k}$ des jeweils anderen Auges zumindest für jede Bewertungsstelle $i_b$ des ersten bzw. zweiten Torsionskorrekturbereichs erfolgt, und wobei in der Zielfunktion zumindest für jede Bewertungsstelle $i_b$ des ersten bzw. zweiten Torsionskorrekturbereichs eine Korrektion einer jeweiligen transformierten astigmatischen Refraktion durch das jeweilige Brillenglas in der bestimmten Gebrauchssituation derart berücksichtigt wird, dass der jeweilige Torsionskorrekturwinkel $\Psi_\Delta(\mathbf{e}_{\zeta,k})$ von der ermittelten korrespondierenden Blickrichtung $-\mathbf{e}_{\zeta,k}$ abhängt,

wobei in der ersten und/oder zweiten primären Zielfunktion für jede Bewertungsstelle $i^{(1)}$ des ersten bzw. zweiten Brillenglases, welche nicht vom ersten bzw. zweiten Torsionskorrekturbereich umfasst ist, eine Korrektion einer ersten bzw. zweiten transformierten astigmatischen Refraktion durch das erste bzw. zweite Brillenglas in der bestimmten Gebrauchssituation derart berücksichtigt wird, dass der erste bzw. zweite primäre Korrektionstorsionswinkel

$\psi_K^{(p)}$ mit dem ersten bzw. zweiten primären Referenztorsionswinkel $\psi_0^{(p)}$ übereinstimmt.

**10.** Verfahren nach einem der vorangegangenen Ansprüche, wobei das Ermitteln der primären Blickrichtung $-\mathbf{e}_\zeta^{(1,p)}$ des ersten Auges ein Ermitteln eines primären ersten Helmholtz-Winkels $\upsilon^{(1,p)}$ des ersten Auges und eines primären zweiten Helmholtz-Winkels $\varphi^{(1,p)}$ des ersten Auges für die zumindest eine erste primäre Bewertungsstelle $i_b^{(1,p)}$ des ersten Brillenglases derart umfasst, dass die Referenzblickrichtung $-\mathbf{e}_z^{(1)}$ des ersten Auges durch eine Kombination einer ersten Rotation des ersten Auges um eine zur Referenzblickrichtung $-\mathbf{e}_z^{(1)}$ des ersten Auges senkrechte, horizontale erste Drehachse $\mathbf{e}_x^{(1)}$ des ersten Auges um den primären ersten Helmholtz-Winkel $\vartheta^{(1,p)}$ des ersten Auges und einer zweiten Rotation des ersten Auges um eine primäre zweite Drehachse $\mathbf{e}_{y,H}^{(1,p)}$ des ersten Auges um den primären zweiten Helmholtz-Winkel $\varphi^{(1,p)}$ des ersten Auges in die primäre Blickrichtung $-\mathbf{e}_\zeta^{(1,p)}$ des ersten Auges übergeht, wobei die primäre zweite Drehachse $\mathbf{e}_{y,H}^{(1)}$ des ersten Auges eine gegenüber einer zur Referenzblickrichtung $-\mathbf{e}_z^{(1)}$ des ersten Auges und zur ersten Drehachse $\mathbf{e}_x^{(1)}$ des ersten Auges senkrechten Achse $\mathbf{e}_y^{(1)}$ um die erste Drehachse $\mathbf{e}_x^{(1)}$ des ersten Auges um den primären ersten Helmholtz-Winkel $\vartheta^{(1,p)}$ des ersten Auges rotierte Achse ist, und

wobei das Ermitteln der korrespondierenden primären Blickrichtung $-\mathbf{e}_{\zeta,k}^{(2,p)}$ des zweiten Auges ein Ermitteln eines korrespondierenden primären ersten Helmholtz-Winkels $\vartheta_k^{(2,p)}$ des zweiten Auges und eines korrespondierenden primären zweiten Helmholtz-Winkels $\varphi_k^{(2,p)}$ des zweiten Auges derart umfasst, dass die Referenzblickrichtung $-\mathbf{e}_z^{(2)}$ des zweiten Auges durch eine Kombination einer ersten Rotation des zweiten Auges um eine zur Referenzblickrichtung $-\mathbf{e}_z^{(2)}$ des zweiten Auges senkrechte, horizontale erste Drehachse $\mathbf{e}_x^{(2)}$ des zweiten Auges um den korrespondierenden primären ersten Helmholtz-Winkel $\vartheta_k^{(2,p)}$ des zweiten Auges und einer zweiten Rotation des zweiten Auges um eine korrespondierende primäre zweite Drehachse $\mathbf{e}_{y,H,k}^{(2,p)}$ des zweiten Auges um den korrespondierenden primären zweiten Helmholtz-Winkel $\varphi_k^{(2,p)}$ des zweiten Auges in die korrespondierende primäre Blickrichtung $-\mathbf{e}_{\zeta,k}^{(2,p)}$ des zweiten Auges übergeht, wobei die korrespondierende primäre zweite Drehachse $\mathbf{e}_{y,H,k}^{(2,p)}$ des zweiten Auges eine gegenüber einer zur Referenzblickrichtung $-\mathbf{e}_z^{(2)}$ des zweiten Auges und zur ersten Drehachse $\mathbf{e}_x^{(2)}$ des zweiten Auges senkrechten Achse $\mathbf{e}_y^{(2)}$ um die erste Drehachse $\mathbf{e}_x^{(2)}$ des zweiten Auges um den korrespondierenden primären ersten Helmholtz-Winkel $\vartheta_k^{(2,p)}$ des zweiten Auges rotierte Achse ist.

**11.** Verfahren nach einem der vorangegangenen Ansprüche in Verbindung mit Anspruch 2, wobei der erste primäre Torsionskorrekturwinkel $\psi_\Delta^{(1,p)}\left(\mathbf{e}_\zeta^{(1,p)}, \mathbf{e}_{\zeta,k}^{(2,p)}\right)$ sowohl von der ermittelten primären Blickrichtung $-\mathbf{e}_\zeta^{(1,p)}$ des ersten Auges als auch von der ermittelten korrespondierenden primären Blickrichtung $-\mathbf{e}_{\zeta,k}^{(2,p)}$ des zweiten Auges abhängt.

**12.** Verfahren nach Anspruch 11 soweit er sich auf Anspruch 10 bezieht, wobei für den ersten primären Torsionskorrekturwinkel $\psi_\Delta^{(1)}$ gilt:

$$\psi_\Delta^{(1)} = \arctan\left(\tan\left(\frac{\vartheta_k^{(2,p)}}{2}\right) \cdot \tan\left(\frac{\varphi_k^{(2,p)}}{2}\right)\right) - \arctan\left(\tan\left(\frac{\vartheta^{(1,p)}}{2}\right) \cdot \tan\left(\frac{\varphi^{(1,p)}}{2}\right)\right).$$

oder

$$\psi_\Delta^{(1)} = 2 \cdot \arctan\left(\tan\left(\frac{\vartheta^{(1,p)} + \vartheta_k^{(2,p)}}{4}\right) \cdot \tan\left(\frac{\varphi^{(1,p)} + \varphi_k^{(2,p)}}{4}\right)\right)$$
$$- 2 \cdot \arctan\left(\tan\left(\frac{\vartheta^{(1,p)}}{2}\right) \cdot \tan\left(\frac{\varphi^{(1,p)}}{2}\right)\right).$$

**13.** Verfahren nach einem der vorangegangenen Ansprüche, wobei für die zumindest eine primäre Bewertungsstelle $i_b^{(1,p)}$ des ersten Brillenglases die in der bestimmten Gebrauchssituation zur primären Blickrichtung $-\mathbf{e}_\zeta^{(1,p)}$ des ersten Auges korrespondierende primäre Blickrichtung $-\mathbf{e}_{\zeta,k}^{(2,p)}$ des zweiten Auges mittels Ray-Tracing unter Annahme der Orthotropie ermittelt wird.

**14.** Computerprogrammerzeugnis, welches Programmteile enthält, welche ausgelegt sind, wenn geladen und ausgeführt auf einem Computer, ein Verfahren zur Optimierung zumindest eines ersten Brillenglases für eine bestimmte Gebrauchssituation zur Korrektion zumindest einer ersten astigmatischen Refraktion eines ersten Auges eines Brillenträgers, welche in einer Referenzblickrichtung $-\mathbf{e}_z^{(1)}$ des ersten Auges eine erste Zylinderreferenzachse $\boldsymbol{\alpha}_0^{(1)}$ aufweist, durchzuführen, wobei das Verfahren zumindest einen ersten primären Berechnungs- bzw. Optimierungsschritt des ersten Brillenglases umfasst, welcher umfasst:

    - Ermitteln einer primären Blickrichtung $-\mathbf{e}_\zeta^{(1,p)}$ des ersten Auges für zumindest eine primäre Bewertungsstelle $i_b^{(1,p)}$ des ersten Brillenglases;

    - Ermitteln einer in der bestimmten Gebrauchssituation zur primären Blickrichtung $-\mathbf{e}_\zeta^{(1,p)}$ des ersten Auges korrespondierenden primären Blickrichtung $-\mathbf{e}_{\zeta,k}^{(2,p)}$ eines zweiten Auges des Brillenträgers; und

    - Minimieren einer ersten primären Zielfunktion für zumindest eine Fläche des ersten Brillenglases, wobei in der ersten primären Zielfunktion für die zumindest eine primäre Bewertungsstelle $i_b^{(1,p)}$ des ersten Brillenglases eine Korrektion einer ersten primären transformierten astigmatischen Refraktion durch das erste Brillenglas in der bestimmten Gebrauchssituation derart berücksichtigt wird, dass die orientierung der zylinderachse der ersten primären transformierten astigmatischen Refraktion von der ermittelten korrespondierenden primären Blickrichtung $-\mathbf{e}_{\zeta,k}^{(2,p)}$ des zweiten Auges abhängt.

**15.** Vorrichtung zur Herstellung zumindest eines ersten Brillenglases, wobei die Vorrichtung umfasst:

- Erfassungsmittel zum Erfassen von Zieldaten zumindest eines ersten Brillenglases;
- Berechnungs- und Optimierungsmittel zum Berechnen und Optimieren zumindest eines ersten Brillenglases für eine bestimmte Gebrauchssituation zur Korrektion zumindest einer ersten astigmatischen Refraktion eines ersten Auges eines Brillenträgers, welche in einer Referenzblickrichtung $-\mathbf{e}_{z}^{(1)}$ des ersten Auges eine erste Zylinderreferenzachse $\alpha_{0}^{(1)}$ aufweist, wobei das Berechnen und Optimieren derart erfolgt, dass es zumindest einen ersten primären Berechnungs- bzw. Optimierungsschritt des ersten Brillenglases umfasst, welcher umfasst:

- Ermitteln einer primären Blickrichtung $-\mathbf{e}_{\zeta}^{(1,p)}$ des ersten Auges für zumindest eine primäre Bewertungsstelle $i_{b}^{(1,p)}$ des ersten Brillenglases;
- Ermitteln einer in der bestimmten Gebrauchssituation zur primären Blickrichtung $-\mathbf{e}_{\zeta}^{(1,p)}$ des ersten Auges korrespondierenden primären Blickrichtung $-\mathbf{e}_{\zeta,k}^{(2,p)}$ eines zweiten Auges des Brillenträgers; und
- Minimieren einer ersten primären Zielfunktion für zumindest eine Fläche des ersten Brillenglases, wobei in der ersten primären Zielfunktion für die zumindest eine primäre Bewertungsstelle $i_{b}^{(1,p)}$ des ersten Brillenglases eine Korrektion einer ersten primären transformierten astigmatischen Refraktion durch das erste Brillenglas in der bestimmten Gebrauchssituation derart berücksichtigt wird, dass die orientierung der zylinderachse der ersten primären transformierten astigmatischen Refraktion von der ermittelten korrespondierenden primären Blickrichtung $-\mathbf{e}_{\zeta,k}^{(2,p)}$ des zweiten Auges abhängt.

**Claims**

**1.** Method for optimising and producing at least a first spectacle lens for a specific situation of wear for correcting at least a first astigmatic refraction of a first eye of a spectacle wearer, which in a reference direction of sight $-\mathbf{e}_{z}^{(1)}$ of the first eye has a first cylinder reference axis $\alpha_{0}^{(1)}$, the method comprising at least a first primary calculation and optimisation step for the first spectacle lens, which comprises:

- determining a primary direction of sight $-\mathbf{e}_{\zeta}^{(1,p)}$ of the first eye for at least one primary evaluation point $i_{b}^{(1,p)}$ of the first spectacle lens;
- determining a primary direction of sight $-\mathbf{e}_{\zeta,k}^{(2,p)}$ of a second eye of the spectacle wearer that corresponds to the primary direction of sight $-\mathbf{e}_{\zeta}^{(1,p)}$ of the first eye in the specific situation of wear; and
- minimising a first primary merit function for at least one surface of the first spectacle lens, wherein the first primary merit function for the at least one primary evaluation point $i_{b}^{(1,p)}$ of the first spectacle lens takes into account a correction of a first primary transformed astigmatic refraction by the first spectacle lens in the specific situation of wear such that the orientation of the cylinder axis of the first primary transformed astigmatic refraction depends on the determined corresponding primary direction of sight $-\mathbf{e}_{\zeta,k}^{(2,p)}$ of the second eye.

2. Method according to claim 1, wherein the first primary merit function for the at least one primary evaluation point $i_b^{(1,p)}$ of the first spectacle lens takes into account the correction of a first primary transformed astigmatic refraction by the first spectacle lens in the specific situation of wear such that the first primary transformed astigmatic refraction has a first primary cylinder correction axis $\alpha_K^{(1,p)}$ that encloses a first primary correction torsion angle $\psi_K^{(1,p)}$ with a first primary torsion reference axis $\mathbf{e}_L^{(1,p)}$, which is perpendicular to the reference direction of sight $-\mathbf{e}_z^{(1)}$ of the first eye and to the primary direction of sight $-\mathbf{e}_\zeta^{(1,p)}$ of the first eye, the first primary correction torsion angle deviating from a first primary reference torsion angle $\psi_0^{(1,p)}$ between the first cylinder reference axis $\alpha_0^{(1)}$ and the first primary torsion reference axis $\mathbf{e}_L^{(1,p)}$ by a first primary torsion correction angle $\psi_\Delta^{(1,p)}\left(\mathbf{e}_{\zeta,k}^{(2,p)}\right)$, which at least depends on the determined corresponding primary direction of sight $-\mathbf{e}_{\zeta,k}^{(2,p)}$ of the second eye.

3. Method according to either claim 1 or claim 2 for optimising and producing the first spectacle lens for a spectacle lens pair to be used together with a second spectacle lens of the spectacle lens pair in spectacles for the specific situation of wear, wherein determining the corresponding primary direction of sight $-\mathbf{e}_{\zeta,k}^{(2,p)}$ the second eye comprises determining a primary evaluation point $i_b^{(2,p)}$ of the second spectacle lens which corresponds to the primary evaluation point $i_b^{(1,p)}$ of the first spectacle lens in the specific situation of wear, taking into account a prismatic power of the first spectacle lens to be optimised and/or of the second spectacle lens in the primary evaluation point of the first or second spectacle lens respectively in the specific situation of wear, and wherein the method comprises: detecting a second cylinder reference axis $\alpha_0^{(2)}$ of a second astigmatic refraction of the second eye in a reference direction of sight $-\mathbf{e}_z^{(2)}$ of the second eye, wherein the first primary merit function for the at least one surface of the first spectacle lens depends on a correction of a second primary transformed astigmatic refraction by the second spectacle lens in the specific situation of wear, wherein the second primary transformed astigmatic refraction has a second primary cylinder correction axis $\alpha_K^{(2,p)}$, which encloses a second primary correction torsion angle $\psi_K^{(2,p)}$ with a second primary torsion reference axis $\mathbf{e}_L^{(2,p)}$, which is perpendicular to the reference direction of sight $-\mathbf{e}_z^{(2)}$ of the second eye and to the corresponding primary direction of sight $-\mathbf{e}_{\zeta,k}^{(2,p)}$ of the second eye, the second primary correction torsion angle deviating from a second primary reference torsion angle $\psi_0^{(2,p)}$ between the second cylinder reference axis $\alpha_0^{(2)}$ and the second primary torsion reference axis $\mathbf{e}_L^{(2,p)}$ by a second primary torsion correction angle $\psi_\Delta^{(2,p)}\left(\mathbf{e}_\zeta^{(1,p)}\right)$, which at least depends on the determined primary direction of sight $-\mathbf{e}_\zeta^{(1,p)}$ of the first eye.

4. Method according to any of the preceding claims for optimising and producing the first spectacle lens for a spectacle lens pair for correcting anisometropia.

5. Method for optimising and producing a spectacle lens pair ([D(1);C(2)]) for a specific situation of wear for correcting a first astigmatic refraction of a first eye of a spectacle wearer, which in a reference direction of sight $-\mathbf{e}_z^{(1)}$ of the

first eye has a first cylinder reference axis $\alpha_0^{(1)}$, by means of a first spectacle lens of the spectacle lens pair and a second astigmatic refraction of a second eye of the spectacle wearer, which in a reference direction of sight $-e_z^{(2)}$ of the second eye has a second cylinder reference axis $\alpha_0^{(2)}$, by means of a second spectacle lens of the spectacle lens pair, comprising:

- a method for optimising and producing the first spectacle lens according to any of the preceding claims; and
- a second secondary calculation and optimisation step for the second spectacle lens depending on the first spectacle lens determined in the first primary calculation and optimisation step,

wherein the second secondary calculation and optimisation step comprises:

-- determining a secondary direction of sight $-e_\zeta^{(2,s)}$ of the second eye for at least one secondary evaluation point $i_b^{(2,s)}$ of the second spectacle lens;

-- determining a secondary direction of sight $-e_{\zeta,k}^{(1,s)}$ of the first eye of the spectacle wearer that corresponds to the secondary direction of sight $-e_\zeta^{(2,s)}$ of the second eye in the specific situation of wear, depending on the first spectacle lens determined in the first primary calculation and optimisation step; and

-- minimising a second secondary merit function for at least one surface of the second spectacle lens, wherein the second secondary merit function for the at least one secondary evaluation point $i_b^{(2,s)}$ of the second spectacle lens takes into account a correction of a second secondary transformed astigmatic refraction by the second spectacle lens in the specific situation of wear such that the second secondary transformed astigmatic refraction has a second secondary cylinder correction axis $\alpha_K^{(2,s)}$, which encloses a second secondary correction torsion angle $\psi_K^{(2,s)}$ with a second secondary torsion reference axis $e_L^{(2,s)}$ that is perpendicular to the reference direction of sight $-e_z^{(2)}$ of the second eye and to the secondary direction of sight $-e_\zeta^{(2,s)}$ of the second eye, said second secondary correction torsion angle deviating from a second secondary reference torsion angle $\psi_0^{(2,s)}$ between the second cylinder reference axis $\alpha_0^{(2)}$ and the second secondary torsion reference axis $e_L^{(2,s)}$ by a second secondary torsion correction angle $\psi_\Delta^{(2,s)}\left(e_{\zeta,k}^{(1,s)}\right)$, which at least depends on the determined corresponding secondary direction of sight $-e_{\zeta,k}^{(1,s)}$ of the first eye.

6. Method for optimising and producing a spectacle lens pair ([D(1);C(2)]) for a specific situation of wear for correcting a first astigmatic refraction of a first eye of a spectacle wearer, which in a reference direction of sight $-e_z^{(1)}$ of the first eye has a first cylinder reference axis $\alpha_0^{(1)}$, by means of a first spectacle lens of the spectacle lens pair and a second astigmatic refraction of a second eye of the spectacle wearer, which in a reference direction of sight $-e_z^{(2)}$ of the second eye has a second cylinder reference axis $\alpha_0^{(2)}$, by means of a second spectacle lens of the spectacle lens pair, comprising:

- a method for optimising and producing the first spectacle lens according to any of the preceding claims; and
- a second secondary calculation and optimisation step for the second spectacle lens depending on the first spectacle lens determined in the first primary calculation and optimisation step,

wherein the second secondary calculation and optimisation step of the second spectacle lens comprises copying at least one surface of the first spectacle lens determined in the first primary calculation and optimisation step.

7. Method according to either claim 5 or claim 6, comprising:

    - a second primary calculation and optimisation step for the second spectacle lens depending on starting values for the first spectacle lens; and
    - a first secondary calculation and optimisation step for the first spectacle lens depending on the first spectacle lens determined in the second primary calculation and optimisation step,

wherein the second primary calculation and optimisation step comprises:

    -- determining a primary direction of sight $-\mathbf{e}_{\zeta}^{(2,p)}$ of the second eye for at least one primary evaluation point $i_b^{(2,p)}$ of the second spectacle lens;

    -- determining a primary direction of sight $-\mathbf{e}_{\zeta,k}^{(1,p)}$ of the first eye of the spectacle wearer that corresponds to the primary direction of sight $-\mathbf{e}_{\zeta}^{(2,p)}$ of the second eye in the specific situation of wear, depending on starting values for the first spectacle lens; and

    -- minimising the second primary merit function for at least one surface of the second spectacle lens, wherein the second primary merit function for the at least one primary evaluation point $i_b^{(2,p)}$ of the second spectacle lens takes into account a correction of a second primary transformed astigmatic refraction by the second spectacle lens in the specific situation of wear such that the second primary transformed astigmatic refraction has a second primary cylinder correction axis $\alpha_K^{(2,p)}$, which encloses a second primary correction torsion angle $\psi_K^{(2,p)}$ with a second primary torsion reference axis $\mathbf{e}_L^{(2,p)}$, which is perpendicular to the reference direction of sight $-\mathbf{e}_z^{(2)}$ of the second eye and to the primary direction of sight $-\mathbf{e}_{\zeta}^{(2,p)}$ of the second eye, the second primary correction torsion angle deviating from a second primary reference torsion angle $\psi_0^{(2,p)}$ between the second cylinder reference axis $\alpha_0^{(2)}$ and the second primary torsion reference axis $\mathbf{e}_L^{(2,p)}$ by a second primary torsion correction angle $\psi_\Delta^{(2,p)}\left(\mathbf{e}_{\zeta,k}^{(1,p)}\right)$, which at least depends on the determined corresponding primary direction of sight $-\mathbf{e}_{\zeta,k}^{(1,p)}$ of the first eye; and/or

wherein the first secondary calculation and optimisation step comprises:

    -- determining a secondary direction of sight $-\mathbf{e}_{\zeta}^{(1,s)}$ of the first eye for at least one secondary evaluation point $i_b^{(1,s)}$ of the first spectacle lens;

    -- determining a secondary direction of sight $-\mathbf{e}_{\zeta,k}^{(2,s)}$ of the second eye of the spectacle wearer that corresponds to the secondary direction of sight $-\mathbf{e}_{\zeta}^{(1,s)}$ of the first eye in the specific situation of wear, depending on the second spectacle lens determined in the second primary calculation and optimisation step; and

    -- minimising a first secondary merit function for at least one surface of the first spectacle lens, wherein the first secondary merit function for the at least one secondary evaluation point $i_b^{(1,s)}$ of the first spectacle lens takes into account a correction of a first secondary transformed astigmatic refraction by the first spectacle lens in the specific situation of wear such that the first secondary transformed astigmatic refraction has a first secondary

cylinder correction axis $\alpha_K^{(1,s)}$, which encloses a first secondary correction torsion angle $\psi_K^{(1,s)}$ with a first secondary torsion reference axis $\mathbf{e}_L^{(1,s)}$, which is perpendicular to the reference direction of sight $-\mathbf{e}_z^{(1)}$ of the first eye and to the secondary direction of sight $-\mathbf{e}_\zeta^{(1,s)}$ of the first eye, the first secondary correction torsion angle deviating from a first secondary reference torsion angle $\psi_0^{(1,s)}$ between the first cylinder reference axis $\alpha_0^{(1)}$ and the first secondary torsion reference axis $\mathbf{e}_L^{(1,s)}$ by a first secondary torsion correction angle $\psi_\Delta^{(1,s)}\left(\mathbf{e}_{\zeta,k}^{(2,s)}\right)$, which at least depends on the determined corresponding secondary direction of sight $-\mathbf{e}_{\zeta,k}^{(2,s)}$ of the second eye.

8. Method according to any of the preceding claims, comprising providing a first and/or second starting surface for the at least one surface of the first or second spectacle lens respectively, wherein the first or second starting surface is determined by minimising a monocular merit function that does not depend on the respective other spectacle lens.

9. Method according to any of the preceding claims, comprising specifying a first and/or second torsion correction region of the first or second spectacle lens respectively, the torsion correction region comprising a near region of the first or second spectacle lens respectively, at least in part, and comprising a plurality of first or second evaluation points $i_b$ of the first or second spectacle lens respectively,
wherein determining the first or second direction of sight $-\mathbf{e}_\zeta$ respectively, for each evaluation point $i$ of the first or second spectacle lens respectively, and determining the corresponding direction of sight $-\mathbf{e}_{\zeta,k}$ of the respective other eye is performed at least for each evaluation point $i_b$ of the first or second torsion correction region respectively, and wherein the merit function, at least for each evaluation point $i_b$ of the first or second torsion correction region respectively, takes into account a correction of a respective transformed astigmatic refraction by the respective spectacle lens in the specific situation of wear such that the respective torsion correction angle $\Psi_\Delta(\mathbf{e}_{\zeta,k})$ depends on the determined corresponding direction of sight $-\mathbf{e}_{\zeta,k}$, wherein the first and/or second primary merit function for each evaluation point $i^{(1)}$ of the first or second spectacle lens respectively, not comprised by the first or second torsion correction region respectively, takes into account a correction of a first or second transformed astigmatic refraction by the first or second spectacle lens respectively in the specific situation of wear such that the first or second primary correction torsion angle $\psi_K^{(p)}$ respectively matches the first or second primary reference torsion angle $\psi_0^{(p)}$ respectively.

10. Method according to any of the preceding claims, wherein determining the primary direction of sight $-\mathbf{e}_\zeta^{(1,p)}$ of the first eye comprises determining a primary first Helmholtz angle $\vartheta^{(1,p)}$ of the first eye and a primary second Helmholtz angle $\varphi^{(1,p)}$ of the first eye for the at least one first primary evaluation point $i_b^{(1,p)}$ of the first spectacle lens such that the reference direction of sight $-\mathbf{e}_z^{(1)}$ of the first eye transitions into the primary direction of sight $-\mathbf{e}_\zeta^{(1,p)}$ of the first eye by a combination of a first rotation of the first eye about a horizontal first rotation axis $\mathbf{e}_x^{(1)}$ of the first eye, which is perpendicular to the reference direction of sight $-\mathbf{e}_z^{(1)}$ of the first eye, by the primary first Helmholtz angle $\vartheta^{(1,p)}$ of the first eye, and of a second rotation of the first eye about a primary second rotation axis $\mathbf{e}_{y,H}^{(1,p)}$ of the first eye by the primary second Helmholtz angle $\varphi^{(1,p)}$ of the first eye, wherein the primary second rotation axis $\mathbf{e}_{y,H}^{(1)}$ of the first eye is an axis that is rotated about the first rotation axis $\mathbf{e}_x^{(1)}$ of the first eye by the primary first Helmholtz angle $\vartheta^{(1,p)}$ of the first eye with respect to an axis $\mathbf{e}_y^{(1)}$, which is perpendicular to the reference direction of sight $-\mathbf{e}_z^{(1)}$ of the first eye and to the first rotation axis $\mathbf{e}_x^{(1)}$ of the first eye, and

wherein determining the corresponding primary direction of sight $-\mathbf{e}_{\zeta,k}^{(2,p)}$ of the second eye comprises determining a corresponding primary first Helmholtz angle $\vartheta_k^{(2,p)}$ of the second eye and a corresponding primary second Helmholtz angle $\varphi_k^{(2,p)}$ of the second eye such that the reference direction of sight $-\mathbf{e}_z^{(2)}$ of the second eye transitions into the corresponding primary direction of sight $-\mathbf{e}_{\zeta,k}^{(2,p)}$ of the second eye by a combination of a first rotation of the second eye about a horizontal first rotation axis $\mathbf{e}_x^{(2)}$ of the second eye, which is perpendicular to the reference direction of sight $-\mathbf{e}_z^{(2)}$ of the second eye, by the corresponding primary first Helmholtz angle $\vartheta_k^{(2,p)}$ of the second eye, and of a second rotation of the second eye about a corresponding primary second rotation axis $\mathbf{e}_{y,H,k}^{(2,p)}$ of the second eye by the corresponding primary second Helmholtz angle $\varphi_k^{(2,p)}$ of the second eye, wherein the corresponding primary second rotation axis $\mathbf{e}_{y,H,k}^{(2,p)}$ of the second eye is an axis that is rotated about the first rotation axis $\mathbf{e}_x^{(2)}$ of the second eye by the corresponding primary first Helmholtz angle $\vartheta_k^{(2,p)}$ of the second eye with respect to an axis which is perpendicular to the reference direction of sight $-\mathbf{e}_z^{(2)}$ of the second eye and to the first rotation axis $\mathbf{e}_x^{(2)}$ of the second eye.

11. Method according to any of the preceding claims in conjunction with claim 2, wherein the first primary torsion correction angle $\psi_\Delta^{(1,p)}\!\left(\mathbf{e}_\zeta^{(1,p)},\mathbf{e}_{\zeta,k}^{(2,p)}\right)$ depends on the determined primary direction of sight $-\mathbf{e}_\zeta^{(1,p)}$ of the first eye and on the determined corresponding primary direction of sight $-\mathbf{e}_{\zeta,k}^{(2,p)}$ of the second eye.

12. Method according to claim 11 when dependent on claim 10, wherein it holds for the first primary torsion correction angle $\psi_\Delta^{(1)}$:

$$\psi_\Delta^{(1)} = \arctan\left(\tan\left(\frac{\vartheta_k^{(2,p)}}{2}\right)\cdot\tan\left(\frac{\varphi_k^{(2,p)}}{2}\right)\right) - \arctan\left(\tan\left(\frac{\vartheta^{(1,p)}}{2}\right)\cdot\tan\left(\frac{\varphi^{(1,p)}}{2}\right)\right).$$

or

$$\psi_\Delta^{(1)} = 2\cdot\arctan\left(\tan\left(\frac{\vartheta^{(1,p)}+\vartheta_k^{(2,p)}}{4}\right)\cdot\tan\left(\frac{\varphi^{(1,p)}+\varphi_k^{(2,p)}}{4}\right)\right)$$
$$- 2\cdot\arctan\left(\tan\left(\frac{\vartheta^{(1,p)}}{2}\right)\cdot\tan\left(\frac{\varphi^{(1,p)}}{2}\right)\right).$$

13. Method according to any of the preceding claims, wherein the primary direction of sight $-\mathbf{e}_{\zeta,k}^{(2,p)}$ of the second eye corresponding to the primary direction of sight $-\mathbf{e}_{\zeta}^{(1,p)}$ in the specific situation of wear is determined for the at least one primary evaluation point $i_b^{(1,p)}$ of the first spectacle lens by means of ray tracing assuming orthotropia.

14. Computer program product including program parts which, when loaded and executed on a computer, are configured to perform a method for optimising at least a first spectacle lens for a specific situation of wear for correcting at least a first astigmatic refraction of a first eye of a spectacle wearer, which has a first cylinder reference axis $\boldsymbol{\alpha}_0^{(1)}$ in a reference direction of sight $-\mathbf{e}_z^{(1)}$ of the first eye, wherein the method comprises at least a first primary calculation and optimisation step for the first spectacle lens, which comprises:

- determining a primary direction of sight $-\mathbf{e}_{\zeta}^{(1,p)}$ of the first eye for at least one primary evaluation point $i_b^{(1,p)}$ of the first spectacle lens;
- determining a primary direction of sight $-\mathbf{e}_{\zeta,k}^{(2,p)}$ of a second eye of the spectacle wearer that corresponds to the primary direction of sight $-\mathbf{e}_{\zeta}^{(1,p)}$ of the first eye in the specific situation of wear; and
- minimising a first primary merit function to produce at least one surface of the first spectacle lens, wherein the first primary merit function for the at least one primary evaluation point $i_b^{(1,p)}$ of the first spectacle lens takes into account a correction of a first primary transformed astigmatic refraction by the first spectacle lens in the specific situation of wear such that the orientation of the cylinder axis of the first primary transformed astigmatic refraction depends on the determined corresponding primary direction of sight $-\mathbf{e}_{\zeta,k}^{(2,p)}$ of the second eye.

15. Apparatus for producing at least a first spectacle lens, wherein the apparatus comprises:

- gathering means for gathering merit data of at least a first spectacle lens;
- calculation and optimisation means for calculating and optimising at least a first spectacle lens for a specific situation of wear for correcting at least a first astigmatic refraction of a first eye of a spectacle wearer, which has a first cylinder reference axis $\boldsymbol{\alpha}_0^{(1)}$ in a reference direction of sight $-\mathbf{e}_z^{(1)}$ of the first eye, wherein the calculation and optimisation is performed such that it comprises at least a first primary calculation and optimisation step for the first spectacle lens, which comprises:

- determining a primary direction of sight $-\mathbf{e}_{\zeta}^{(1,p)}$ of the first eye for at least one primary evaluation point $i_b^{(1,p)}$ of the first spectacle lens;
- determining a primary direction of sight $-\mathbf{e}_{\zeta,k}^{(2,p)}$ of a second eye of the spectacle wearer that corresponds to the primary direction of sight $-\mathbf{e}_{\zeta}^{(1,p)}$ of the first eye in the specific situation of wear; and
- minimising a first primary merit function for at least one surface of the first spectacle lens, wherein the first primary merit function for the at least one primary evaluation point $i_b^{(1,p)}$ of the first spectacle lens takes into account a correction of a first primary transformed astigmatic refraction by the first spectacle lens in the specific situation of wear such that the orientation of the cylinder axis of the first primary transformed astigmatic refraction depends on the determined corresponding primary direction of sight $-\mathbf{e}_{\zeta,k}^{(2,p)}$ of the second eye.

**Revendications**

1. Procédé d'optimisation et de fabrication d'au moins un premier verre de lunettes pour une situation d'utilisation définie pour la correction d'au moins une première réfraction astigmatique d'un premier oeil d'un porteur de lunettes, qui présente un premier axe de cylindre de référence $\alpha_0^{(1)}$ dans une ligne de vision de référence $-\mathbf{e}_z^{(1)}$ du premier oeil, comprenant au moins une étape primaire de calcul, respectivement d'optimisation, du premier verre de lunettes, qui comprend :

- l'établissement d'une ligne de vision primaire $-\mathbf{e}_\zeta^{(1,p)}$ du premier oeil pour au moins un point d'évaluation $i_b^{(1,p)}$ primaire du premier verre de lunettes ;
- l'établissement d'une ligne de vision primaire $-\mathbf{e}_{\zeta,k}^{(2,p)}$ d'un deuxième oeil du porteur de lunettes correspondant à la ligne de vision primaire $-\mathbf{e}_\zeta^{(1,p)}$ du premier oeil dans la situation d'utilisation définie ; et
- la minimisation d'une première fonction cible primaire pour au moins une surface du premier verre de lunettes, où, dans le cas de la première fonction cible primaire, pour au moins un point d'évaluation primaire $i_b^{(1,p)}$ du premier verre de lunettes, une correction d'une première réfraction astigmatique primaire transformée par le premier verre de lunettes dans la situation d'utilisation définie est considérée de sorte que l'orientation de l'axe de cylindre de la première réfraction astigmatique transformée primaire dépend de la ligne de vision $-\mathbf{e}_{\zeta,k}^{(2,p)}$ primaire correspondante établie du second oeil.

2. Procédé selon la revendication 1 où, dans la première fonction cible primaire pour au moins un premier point d'évaluation primaire $i_b^{(1,p)}$ du premier verre de lunettes, la correction d'une première réfraction astigmatique transformée primaire par le premier verre de lunettes dans la situation d'utilisation définie est considérée de telle façon que la première réfraction astigmatique transformée primaire présente un premier axe de cylindre de correction $\alpha_k^{(1,p)}$ primaire qui englobe à la fois un premier axe de torsion de référence primaire $\mathbf{e}_L^{(1,p)}$ vertical par rapport à la ligne de vision de référence $-\mathbf{e}_z^{(1)}$ du premier oeil et par rapport à la ligne de vision $-\mathbf{e}_\zeta^{(1,p)}$ du premier oeil, et un premier angle de torsion de correction $\Psi_k^{(1,p)}$ primaire, qui dévie d'un premier angle de torsion de référence $\Psi_0^{(1,p)}$ primaire entre le premier axe de cylindre de référence $\alpha_0^{(1)}$ et le premier axe de torsion de référence $\mathbf{e}_L^{(1,p)}$ primaire d'un premier angle de correction de torsion $\Psi_\Delta^{(1,p)}(\mathbf{e}_{\zeta,k}^{(2,p)})$ primaire, qui dépend au moins de la ligne de vision $-\mathbf{e}_{\zeta,k}^{(2,p)}$ correspondante évaluée du second oeil.

3. Procédé selon les revendications 1 ou 2 pour l'optimisation et la fabrication du premier verre de lunettes pour une paire de lunettes en vue d'une utilisation avec un second verre de lunettes de la paire de lunettes dans des lunettes pour une situation d'emploi définie, la détermination de la ligne de vision $-\mathbf{e}_{\zeta,k}^{(2,p)}$ primaire correspondante du second oeil comprend une détermination d'un point d'évaluation $i_b^{(2,p)}$ primaire du second verre de lunettes correspondant à un point d'évaluation $i_b^{(1,p)}$ du premier verre de lunettes dans la situation d'utilisation définie en tenant compte d'une influence prismatique du premier verre de lunettes à optimiser et/ou du second verre de lunettes au point d'évaluation primaire du premier, respectivement, du second, verre de lunettes dans la situation d'utilisation définie, le procédé comprenant : une évaluation d'un second axe de cylindre de référence $\alpha_0^{(2)}$ d'une seconde réfraction astigmatique du second oeil dans une ligne de vision de référence $-\mathbf{e}_z^{(2)}$ du second oeil, la première fonction cible primaire pour au moins une surface du premier verre de lunettes dépendant d'une correction d'une seconde réfraction astigmatique primaire transformée par le second verre de lunette dans la situation d'utilisation définie, la seconde réfraction astigmatique primaire transformée présentant un second axe de cylindre de correction $\alpha_K^{(2,p)}$ primaire qui dévie, avec à la fois un second axe de torsion de référence $\mathbf{e}_L^{(2,p)}$ primaire vertical par rapport à la ligne visuelle de référence $-\mathbf{e}_z^{(2)}$ du second oeil ainsi que par rapport à la ligne de vision $-\mathbf{e}_{\zeta,k}^{(2,p)}$ primaire correspondante du second oeil et un second angle de torsion de référence $\Psi_0^{(2,p)}$ primaire entre le second axe de cylindre de référence $\alpha_0^{(2)}$ et le second axe de torsion de référence $\mathbf{e}_L^{(2,p)}$ d'un second angle de correction de torsion $\Psi_\Delta^{(2,p)}(\mathbf{e}_\zeta^{(1,p)})$ qui dépend au moins de la ligne visuelle $-\mathbf{e}_\zeta^{(1,p)}$ du premier oeil.

4. Procédé selon l'une des revendications précédentes pour l'optimisation et la fabrication du premier verre de lunettes pour une paire de lunettes pour la correction d'une anisométropie.

5. Procédé pour l'optimisation et la fabrication d'une paires de lunettes ([D(1) ;C(2)]) pour une situation d'utilisation définie pour la correction d'une première réfraction astigmatique d'un premier oeil d'un porteur de lunettes qui présente un premier axe de cylindre de référence $\alpha_0^{(1)}$ dans une ligne de vision de référence $-\mathbf{e}_z^{(1)}$ du premier oeil, au moyen d'un premier verre de lunettes de la paire de lunettes et la correction d'une seconde réfraction astigmatique du second oeil du porteur de lunettes qui présente un second axe de cylindre de référence $\alpha_0^{(2)}$ dans une ligne de vision de référence $-\mathbf{e}_z^{(2)}$ du second oeil, au moyen d'un second verre de lunettes de la paire de lunettes, comprenant :

- un procédé pour l'optimisation et la fabrication du premier verre de lunettes selon l'une des revendications précédentes ; et
- une seconde étape secondaire de calcul, respectivement d'optimisation, du second verre de lunettes en fonction du premier verre de lunettes évalué dans la première étape de calcul, respectivement d'optimisation, primaires,

la seconde étape secondaire de calcul, respectivement d'optimisation, comprenant :

-- l'évaluation d'une ligne de vision $-\mathbf{e}_\zeta^{(2,s)}$ secondaire du deuxième oeil pour au moins un point d'évaluation $i_b^{(2,s)}$ secondaire du second verre de lunettes ;

-- l'évaluation d'une ligne de vision $-\mathbf{e}_{\zeta,k}^{(1,s)}$ secondaire du premier oeil du porteur de lunettes correspondant à la ligne de vision $-\mathbf{e}_\zeta^{(2,s)}$ secondaire du deuxième oeil dans la situation d'utilisation définie, en fonction du premier verre de lunettes établi dans la première étape primaire de calcul, respectivement, d'optimisation ; et

-- la minimisation d'une deuxième fonction cible secondaire pour au moins une surface du second verre de lunettes, où il est ainsi tenu compte dans la deuxième fonction cible secondaire pour au moins un point d'évaluation $i_b^{(2,s)}$ secondaire du second verre de lunettes d'une correction d'une seconde réfraction astigmatique secondaire transformée par le second verre de lunettes dans la situation d'utilisation définie, de la sorte que la seconde réfraction astigmatique secondaire transformée présente un deuxième axe de cylindre de correction $\alpha_K^{(2,s)}$ secondaire qui englobe à la fois un deuxième axe de torsion de référence $\mathbf{e}_L^{(2,s)}$ secondaire vertical par rapport à la ligne de vision de référence $-\mathbf{e}_z^{(2)}$ du second oeil et également par rapport à la ligne de vision $-\mathbf{e}_\zeta^{(2,s)}$ secondaire du deuxième oeil, et un second angle de torsion de correction $\psi_K^{(2,s)}$ secondaire qui dévie par rapport à un deuxième angle de torsion de référence $\Psi_0^{(2,s)}$ secondaire entre le deuxième axe de cylindre de référence $\alpha_0^{(2)}$ et le deuxième axe de référence de torsion secondaire $\mathbf{e}_L^{(2,s)}$ d'un deuxième angle de correction de torsion $\Psi_\Delta^{(2,s)}(\mathbf{e}_{\zeta,k}^{(1,s)})$, qui dépend au moins de la ligne de vision $-\mathbf{e}_{\zeta,k}^{(1,s)}$ secondaire correspondante évaluée pour le premier oeil.

6. Procédé pour l'optimisation et la fabrication d'une paire de verres de lunettes ([D(1) ;C(2)]) pour une situation d'utilisation définie pour la correction d'une première réfraction astigmatique d'un premier oeil d'un porteur de lunettes qui présente un premier axe de cylindre de référence $\alpha_0^{(1)}$ dans une ligne de vision de référence $-\mathbf{e}_z^{(1)}$ du premier oeil, au moyen d'un premier verre de lunettes d'une paire de lunettes et la correction d'une deuxième réfraction astigmatique d'un second oeil du porteur de lunettes, qui présente un deuxième axe de cylindre de référence $a_0^{(2)}$ dans une ligne de vision de référence $-\mathbf{e}_z^{(2)}$ du second oeil, au moyen d'un deuxième verre de lunettes de la paire de lunettes, comprenant :

- un procédé pour l'optimisation et la fabrication du premier verre de lunettes selon l'une des revendications précédentes ; et
- une deuxième étape secondaire de calcul, respectivement, d'optimisation, du deuxième verre de lunettes en fonction du premier verre de lunettes évalué dans la première étape de calcul, respectivement, d'optimisation,

la deuxième étape secondaire de calcul, respectivement, d'optimisation, du second verre de lunettes comprenant une copie d'au moins une surface du premier verre de lunettes évaluée dans la première étape de calcul, respectivement, d'optimisation.

7. Procédé selon les revendications 5 ou 6, comprenant :

- une deuxième étape primaire de calcul, respectivement, d'optimisation, du second verre de lunettes en fonction de données de départ pour le premier verre de lunettes ; et
- une première étape secondaire de calcul, respectivement, d'optimisation, du premier verre de lunettes en fonction du premier verre de lunettes évalué dans la deuxième étape primaire de calcul, respectivement, d'optimisation,

la seconde étape primaire de calcul, respectivement, d'optimisation, comprenant :

-- l'évaluation d'une ligne de vision primaire $-\mathbf{e}_\zeta^{(2,p)}$ du deuxième oeil pour au moins un point d'évaluation primaire $i_b^{(2,p)}$ du second verre de lunettes ;

-- l'évaluation d'une ligne de vision primaire $-\mathbf{e}_{\zeta,k}^{(1,p)}$ du premier oeil du porteur de lunettes correspondant à la ligne de vision primaire $-\mathbf{e}_\zeta^{(2,p)}$ du second oeil dans une situation d'utilisation définie, en fonction de données de départ pour le premier verre de lunettes ; et

-- la minimisation d'une seconde fonction cible primaire pour au moins une surface du second verre de lunettes, où il est ainsi tenu compte, dans la deuxième fonction cible primaire, pour au moins un point d'évaluation $i_b^{(2,p)}$ primaire du second verre de lunettes, d'une correction d'une seconde réfraction astigmatique primaire transformée par le deuxième verre de lunettes dans la situation d'utilisation définie que la deuxième réfraction astigmatique primaire transformée présente un second axe de cylindre de correction $\alpha_K^{(2,p)}$ qui englobe un second axe de référence de torsion primaire $\mathbf{e}_L^{(2,p)}$ vertical par rapport à la fois à une ligne de vision de référence $-\mathbf{e}_z^{(2)}$ du second oeil et à la ligne de vision primaire $-\mathbf{e}_\zeta^{(2,p)}$ du second oeil, et un second angle de correction de torsion $\psi_K^{(2,p)}$ primaire, qui dévie d'un second angle de torsion de référence $\Psi_0^{(2,p)}$ primaire entre le deuxième axe de cylindre de référence $\alpha_0^{(2)}$ et le deuxième axe de torsion de référence primaire $\mathbf{e}_L^{(2,p)}$ d'un second angle de correction de torsion primaire $\Psi_\Delta^{(2,p)}(e_{\zeta,k}^{(1,p)})$, qui dépend au moins de la ligne de vision primaire $-\mathbf{e}_{\zeta,k}^{(1,p)}$ correspondante évaluée du premier oeil ; et/ou

la première étape secondaire de calcul, respectivement, d'optimisation, comprenant :

-- l'évaluation d'une ligne de vision secondaire $-\mathbf{e}_\zeta^{(1,s)}$ du premier oeil pour au moins un point d'évaluation secondaire $i_b^{(1,s)}$ du premier verre de lunettes ;
-- l'évaluation d'une ligne de vision secondaire $-\mathbf{e}_{\zeta,k}^{(2,s)}$ du deuxième oeil du porteur de lunettes correspondant à la ligne de vision secondaire $-\mathbf{e}_\zeta^{(1,s)}$ du premier oeil dans une situation d'utilisation définie, en fonction du deuxième verre de lunettes évalué dans la deuxième étape de calcul, respectivement d'optimisation ; et
-- la minimisation d'une première fonction cible secondaire pour au moins une surface du premier verre de lunettes, où il est ainsi tenu compte, dans la première fonction cible secondaire pour au moins un point d'évaluation $i_b^{(1,s)}$ secondaire du premier verre de lunettes, il est ainsi tenu compte d'une correction d'une première réfraction astigmatique secondaire transformée par le premier verre de lunettes dans la situation d'utilisation définie que la première réfraction astigmatique secondaire transformée présente un second axe de cylindre de correction $\alpha_K^{(2,p)}$ qui englobe un premier axe de cylindre de correction secondaire $\alpha_K^{(1,s)}$ qui englobe un premier axe de torsion de référence secondaire $\mathbf{e}_L^{(1,s)}$ vertical par rapport à la fois à une ligne de vision de référence $-\mathbf{e}_z^{(1)}$ du premier oeil et à la ligne de vision secondaire $-\mathbf{e}_\zeta^{(1,s)}$ du premier oeil, et un premier angle de correction de torsion $\psi_K^{(1,s)}$ secondaire, qui dévie d'un premier angle de torsion de référence $\psi_0^{(1,s)}$ secondaire entre le premier axe de cylindre de référence $\alpha_0^{(1)}$ et le premier axe de torsion de référence secondaire $\mathbf{e}_L^{(1,s)}$ d'un premier angle de correction de torsion secondaire $\psi_\Delta^{(1,s)}(e_{\zeta,k}^{(2,s)})$, qui dépend au moins de la ligne de vision secondaire $-\mathbf{e}_{\zeta,k}^{(2,s)}$ correspondante évaluée du second oeil.

8. Procédé selon l'une des revendications précédentes comprenant une mise en place d'une première et/ou d'une seconde surface de départ pour au moins une surface du premier, respectivement, du second, verre de lunettes, la première, respectivement, la seconde, surface de départ étant déterminée par une minimisation d'une fonction cible monoculaire qui ne dépend pas de l'autre verre de lunettes.

9. Procédé selon l'une des revendications précédentes comprenant un établissement d'un premier et/ou d'un second domaine de correction de torsion du premier, respectivement, du second, verre de lunettes, qui comprend un domaine proche du premier verre, respectivement, du second, verre de lunettes au moins partiellement, et qui comprend une multitude de premiers, respectivement de seconds, points d'évaluation $i_b$ du premier, respectivement du second, verre de lunettes, l'évaluation de la première, respectivement de la seconde, ligne de vision $-\mathbf{e}_{\zeta,k}$ pour chaque point d'évaluation i du premier, respectivement du second, verre de lunettes et l'évaluation des lignes de vision $-\mathbf{e}_{\zeta,k}$ correspondantes de chaque fois l'autre oeil, s'effectue au moins pour chaque point d'évaluation $i_b$ du premier, respectivement du deuxième, domaine de correction de torsion, et où dans la fonction cible au moins pour chaque point d'évaluation $i_b$, du premier, respectivement du second, domaine de correction de torsion, il est ainsi tenu compte d'une correction d'une réfraction astigmatique transformée respective par le verre de lunettes respectif dans la situation d'utilisation définie, que l'angle de correction de torsion respectif $\psi_\Delta(e_{\zeta,k})$ dépend de la ligne de vision correspondante $-\mathbf{e}_{\zeta,k}$,
où, dans la première et/ou dans la seconde fonction cible primaire, pour chaque point d'évaluation $i^{(1)}$ du premier, respectivement du deuxième verre de lunettes, qui n'est pas compris dans le premier, respectivement, dans le deuxième, domaine de correction de torsion, il est ainsi tenu compte d'une correction d'une première, respectivement, d'une deuxième, réfraction astigmatique transformée par le premier, respectivement, par le second, verre de lunettes dans une situation d'utilisation définie de sorte que le premier, respectivement, le second, angle de correction de torsion $\psi_K^{(p)}$ corresponde avec le premier, respectivement, le second angle de torsion de référence $\psi_0^{(p)}$.

10. Procédé selon l'une des revendications précédentes, l'évaluation de la ligne de vision primaire $-\mathbf{e}_\zeta^{(1,p)}$ du premier oeil comprend une évaluation d'un premier angle d'Helmholtz primaire $\upsilon^{(1,p)}$ du premier oeil et d'un second angle d'Helmholtz primaire $\varphi^{(1,p)}$ du premier oeil pour au moins un premier point d'évaluation primaire $i_b^{(1,p)}$ du premier verre de lunettes, de sorte que la ligne de vision de référence $-\mathbf{e}_z^{(1)}$ du premier oeil passe par une combinaison d'une première rotation du premier oeil autour d'un premier axe de rotation $\mathbf{e}_x^{(1)}$ horizontal, perpendiculaire à une ligne de vision de référence $-\mathbf{e}_z^{(1)}$ du premier oeil d'un premier angle d'Helmholtz primaire $\upsilon^{(1,p)}$ du premier oeil et d'une seconde rotation du premier oeil autour d'un deuxième axe de rotation primaire $\mathbf{e}_{y,H}^{(1,p)}$ du premier oeil d'un second angle primaire d'Helmholtz $\varphi^{(1,p)}$ du premier oeil dans la ligne de vision primaire $-\mathbf{e}_\zeta^{(1,p)}$ du premier oeil, le second axe de rotation primaire $\mathbf{e}_{y,H}^{(1,p)}$ du premier oeil étant un axe tournant par rapport à une ligne de vision de référence $-\mathbf{e}_z^{(1)}$ du premier oeil et par rapport à un premier axe de rotation $\mathbf{e}_x^{(1)}$ du premier oeil d'un premier angle primaire d'Helmholtz $\upsilon^{(1,p)}$ du premier oeil, et

l'évaluation de la ligne de vision primaire $-\mathbf{e}_{\zeta,k}^{(2,p)}$ du second oeil comprenant une évaluation d'un premier angle primaire d'Helmholtz $\upsilon^{(2,p)}$ correspondant du deuxième oeil et comprenant un deuxième angle primaire d'Helmholtz $\varphi^{(2,p)}$ du second oeil, de sorte que la ligne de vision de référence $-\mathbf{e}_z^{(2)}$ du deuxième oeil est composée par une combinaison d'une première rotation du second oeil autour d'un premier axe de rotation $\mathbf{e}_x^{(2)}$ du second oeil horizontal, perpendiculaire à la ligne de vision de référence $-\mathbf{e}_z^{(2)}$ du second oeil d'un premier angle primaire d'Helmholtz $\upsilon^{(2,p)}$ correspondant du deuxième oeil et d'une seconde rotation du deuxième oeil autour d'un second axe de rotation primaire $\mathbf{e}_{y,H,k}^{(2,p)}$ correspondant du deuxième oeil d'un second angle primaire d'Helmholtz $\varphi_k^{(2,p)}$ correspondant du second oeil dans la ligne de vision primaire $-\mathbf{e}_{\zeta,k}^{(2,p)}$ correspondante du deuxième oeil, le second axe de rotation primaire $\mathbf{e}_{y,H,k}^{(2,p)}$ correspondant du deuxième oeil étant un axe $\mathbf{e}_y^{(2)}$ vertical par rapport à une ligne de vision de référence $-\mathbf{e}_z(2)$ du deuxième oeil et par rapport au premier axe de rotation $\mathbf{e}_x^{(2)}$ du second oeil autour du premier axe de rotation $\mathbf{e}_x^{(2)}$ du second oeil d'un premier angle - primaire d'Helmholtz $\upsilon_k^{(2,p)}$ correspondant du deuxième oeil.

11. Procédé selon l'une des revendications précédentes en relation avec la revendication 2, le premier angle de correction de torsion primaire $\psi_\Delta^{(1,p)}(\mathbf{e}_\zeta^{(1,p)}, \mathbf{e}_{\zeta,k}^{(2,p)})$, dépendant à la fois de la ligne de vision primaire $-\mathbf{e}_\zeta^{(1,p)}$ déterminée du premier oeil et de la ligne de vision primaire $-\mathbf{e}_{\zeta,k}^{(1,p)}$ correspondante évaluée du deuxième oeil.

12. Procédé selon la revendication 1 tant qu'elle se rapporte à la revendication 10, où, pour le premier angle de correction de torsion primaire $\psi_\Delta^{(1)}$, on a la relation :

$$\psi_\Delta^{(1)} = \arctan\left(\tan\left(\frac{\vartheta_k^{(2,p)}}{2}\right)\cdot\tan\left(\frac{\varphi_k^{(2,p)}}{2}\right)\right) - \arctan\left(\tan\left(\frac{\vartheta^{(1,p)}}{2}\right)\cdot\tan\left(\frac{\varphi^{(1,p)}}{2}\right)\right).$$

$$\psi_\Delta^{(1)} = 2\cdot\arctan\left(\tan\left(\frac{\vartheta^{(1,p)}+\vartheta_k^{(2,p)}}{4}\right)\cdot\tan\left(\frac{\varphi^{(1,p)}+\varphi_k^{(2,p)}}{4}\right)\right)$$
$$- 2\cdot\arctan\left(\tan\left(\frac{\vartheta^{(1,p)}}{2}\right)\cdot\tan\left(\frac{\varphi^{(1,p)}}{2}\right)\right).$$

13. Procédé selon l'une des revendications précédentes, où, pour au moins un point d'évaluation primaire $i_b^{(1,p)}$ du premier verre de lunettes, la ligne de vision primaire $-\mathbf{e}_{\zeta,k}^{(2,p)}$ correspondante du second oeil dans la situation d'utilisation définie est déterminée par rapport à la ligne de vision primaire $-\mathbf{e}_\zeta^{(1,p)}$ du premier oeil au moyen d'un Ray-Tracing en admettant une orthotropie.

14. Résultat d'un programme d'ordinateur qui contient des parties de programmes qui sont établies, si elles sont chargées et exécutées sur un ordinateur, pour réaliser un procédé pour l'optimisation d'au moins un premier verre de lunettes pour une utilisation définie pour la correction d'au moins une première réfraction astigmatique d'un premier oeil d'un porteur de lunettes, qui présente un premier axe de cylindre de référence $\alpha_0^{(1)}$ dans une ligne de vision de référence $-\mathbf{e}_z^{(1)}$ du premier oeil, le procédé comprenant au moins une première étape primaire de calcul, respectivement d'optimisation, du premier verre de lunettes, procédé comprenant :

- l'évaluation d'une ligne de vision primaire $-\mathbf{e}_\zeta^{(1,p)}$ du premier oeil pour au moins un point d'évaluation primaire $i_b^{(1,p)}$ du premier verre de lunettes ;
- l'évaluation d'une ligne de vision primaire $-\mathbf{e}_{\zeta,k}^{(2,p)}$ d'un second oeil d'un porteur de lunettes correspondant à la ligne de vision primaire $-\mathbf{e}_\zeta^{(1,p)}$ du premier oeil pour une situation d'utilisation définie ; et
- la minimisation d'une première fonction cible primaire pour au moins une surface du premier verre de lunettes, où il est ainsi tenu compte dans la première fonction cible pour au moins un point d'évaluation primaire $i_b^{(1,p)}$ du premier verre d'une correction d'une première réfraction astigmatique primaire transformée par le premier verre de lunettes dans la situation d'utilisation définie que l'orientation de l'axe de cylindre de la première réfraction astigmatique primaire transformée dépend de la ligne de vision primaire $-\mathbf{e}_{\zeta,k}^{(2,p)}$ évaluée correspondante du deuxième oeil.

15. Dispositif pour la fabrication d'au moins un premier verre de lunettes, le dispositif comprenant :

- des moyens de détermination pour déterminer des données cibles au moins pour un premier verre de lunettes ;
- des moyens de calcul et d'optimisation pour calculer et optimiser au moins un premier verre de lunettes pour une situation d'utilisation définie pour la correction d'au moins une première réfraction astigmatique d'un premier oeil d'un porteur de lunettes, qui présente un premier axe de cylindre de référence $\alpha_0^{(1)}$ dans une ligne de vision de référence $-\mathbf{e}_z^{(1)}$ du premier oeil, le calcul et l'optimisation ayant lieu de telle manière qu'ils comprennent une première étape de calcul, respectivement d'optimisation, du premier verre de lunettes, étape qui comprend :
- l'évaluation d'une ligne de vision primaire $-\mathbf{e}_\zeta^{(1,p)}$ du premier oeil pour au moins un point d'évaluation primaire $i_b^{(1,p)}$ du premier verre de lunettes ;
- l'évaluation d'une ligne de vision primaire $-\mathbf{e}_{\zeta,k}^{(2,p)}$ d'un deuxième oeil d'un porteur de lunettes correspondant à la ligne de vision primaire $-\mathbf{e}_\zeta^{(1,p)}$ du premier oeil dans la situation d'utilisation définie ; et
- la minimisation d'une première fonction cible primaire pour au moins une surface du premier verre de lunettes, où, dans la première fonction cible primaire pour au moins un point d'évaluation primaire $i_b^{(1,p)}$ du premier verre de lunettes, il est tenu compte d'une correction d'une première réfraction astigmatique primaire transformée par le premier verre de lunettes dans la situation d'utilisation définie que l'orientation de l'axe de cylindre de la première réfraction astigmatique primaire transformée dépend de la ligne de vision primaire $-\mathbf{e}_{\zeta,k}^{(2,p)}$ correspondante évaluée du second oeil.

Fig. 1

Fig. 2A

Fig. 2B

Fig 3

S1p

A(2)

B(1)

S2s

S1t

C(2)

D(1)

S2q

E(2)

Fig 4

S1p

A(2)

S2s

B(1)

C(2)

S1t

S2q

D(1)

E(2)

F(1)

G(2)

H(1)

J(2)

Fig 5

A(1)　　　　A(2)

S1p　　　　S2p

B(1)　　　　B(2)

S1s　　　　S2s

C(1)　　　　C(2)

S1t　　　　S2t

D(1)　　　　D(2)

S1q　　　　S2q

E(1)　　　　E(2)

Fig.6A  Astigmatismus (Komb. Glas+Auge)

Fig.6B  Astigmatismus (Komb. Glas+Auge)

Fig.6D

GST an HBL VorderFl.

$\dfrac{y}{mm}$

$\dfrac{Abbildungsfehler}{dpt}$

- - - Refraktionsfehler
——— Astigmatismus

Fig.6C

GST an HBL VorderFl.

$\dfrac{y}{mm}$

$\dfrac{Abbildungsfehler}{dpt}$

- - - Refraktionsfehler
——— Astigmatismus

Fig.7B  Astigmatismus (Komb. Glas+Auge)

Fig.7A  Astigmatismus (Komb. Glas+Auge)

Fig.7D

GST an HBL VorderFl.

Fig.7C

GST an HBL VorderFl.

Fig.8 – Teil 1/3

| | -30 | -27.5 | -25 | -22.5 | -20 | -17.5 | -15 | -12.5 |
|---|---|---|---|---|---|---|---|---|
| 25 | | | | | | | 0.33222 | 0.31051 |
| 22.5 | | | | | | 0.31632 | 0.28864 | 0.27013 |
| 20 | | | | | 0.32735 | 0.26894 | 0.24497 | 0.22338 |
| 17.5 | | | | 0.35242 | 0.29045 | 0.22671 | 0.19346 | 0.17886 |
| 15 | | | 0.44097 | 0.2951 | 0.24747 | 0.21466 | 0.19275 | 0.16584 |
| 12.5 | | | 0.41108 | 0.18462 | 0.24759 | 0.25768 | 0.23425 | 0.19477 |
| 10 | | 0.56341 | 0.45553 | 0.4341 | 0.39884 | 0.37866 | 0.34713 | 0.30513 |
| 7.5 | | 1.06786 | 0.88887 | 0.73158 | 0.6267 | 0.59134 | 0.55333 | 0.50101 |
| 5 | | 1.50957 | 1.33613 | 1.19884 | 1.07458 | 0.99288 | 0.92291 | 0.84628 |
| 2.5 | | 1.9585 | 1.76951 | 1.63896 | 1.5248 | 1.44427 | 1.38415 | 1.31831 |
| 0 | 2.72488 | 2.41686 | 2.24167 | 2.11625 | 2.01 | 1.93431 | 1.90013 | 1.8558 |
| -2.5 | | 2.92296 | 2.74289 | 2.61622 | 2.51795 | 2.44675 | 2.42998 | 2.41906 |
| -5 | | 3.30442 | 3.27837 | 3.12924 | 3.01377 | 2.92789 | 2.90477 | 2.90317 |
| -7.5 | | 3.32157 | 3.31882 | 3.30143 | 3.30101 | 3.34283 | 3.32869 | 3.25821 |
| -10 | | 3.34492 | 3.29914 | 3.32459 | 3.31435 | 3.34031 | 3.40506 | 3.40792 |
| -12.5 | | | 3.34552 | 3.34843 | 3.32816 | 3.322 | 3.37882 | 3.31855 |
| -15 | | | 3.35299 | 3.34764 | 3.32264 | 3.31526 | 3.37457 | 3.17767 |
| -17.5 | | | | 3.33896 | 3.32607 | 3.3277 | 3.3728 | 3.12799 |
| -20 | | | | | 3.32688 | 3.33068 | 3.34797 | 3.13552 |
| -22.5 | | | | | | 3.32839 | 3.29512 | 3.19663 |
| -25 | | | | | | | 3.29886 | 3.29366 |

**Tabelle 1:** Pfeilhöhe der Rückfläche des Ausführungsbeispieles

EP 2 356 507 B1

Fig.8 – Teil 2/3

| | -10 | -7.5 | -5 | -2.5 | 0 | 2.5 | 5 | 7.5 | 10 |
|---|---|---|---|---|---|---|---|---|---|
| 30 | | | | | 0.27721 | | | | |
| 27.5 | 0.32698 | 0.30993 | 0.29125 | 0.27279 | 0.25407 | 0.23396 | 0.21186 | 0.20005 | 0.1097 |
| 25 | 0.2971 | 0.29276 | 0.2818 | 0.27075 | 0.25127 | 0.22883 | 0.1984 | 0.16117 | 0.09555 |
| 22.5 | 0.26448 | 0.26262 | 0.25824 | 0.25016 | 0.23309 | 0.2067 | 0.17254 | 0.15445 | 0.08849 |
| 20 | 0.22134 | 0.22372 | 0.22344 | 0.21687 | 0.20053 | 0.17122 | 0.13452 | 0.09752 | 0.15583 |
| 17.5 | 0.17884 | 0.17982 | 0.17886 | 0.17035 | 0.15288 | 0.12192 | 0.0966 | 0.10196 | 0.19796 |
| 15 | 0.1531 | 0.14197 | 0.12946 | 0.12176 | 0.10205 | 0.07974 | 0.08545 | 0.14933 | 0.27051 |
| 12.5 | 0.16233 | 0.13455 | 0.10934 | 0.07202 | 0.06454 | 0.09211 | 0.13137 | 0.2363 | 0.3773 |
| 10 | 0.25105 | 0.20078 | 0.15059 | 0.06865 | 0.02946 | 0.11673 | 0.23274 | 0.36748 | 0.54085 |
| 7.5 | 0.43534 | 0.36204 | 0.26689 | 0.09269 | 0.02989 | 0.18962 | 0.41067 | 0.58667 | 0.79654 |
| 5 | 0.75524 | 0.64224 | 0.52368 | 0.29034 | 0.0847 | 0.31205 | 0.66184 | 0.93556 | 1.19815 |
| 2.5 | 1.23141 | 1.09725 | 0.9112 | 0.58324 | 0.16268 | 0.50181 | 1.00604 | 1.41103 | 1.74639 |
| 0 | 1.77139 | 1.60173 | 1.35831 | 1.05745 | 0.39117 | 0.63085 | 1.30203 | 1.85646 | 2.31417 |
| -2.5 | 2.34141 | 2.14978 | 1.85573 | 1.4569 | 0.74365 | 0.51771 | 1.36029 | 2.07146 | 2.66478 |
| -5 | 2.83495 | 2.6439 | 2.30462 | 1.79916 | 1.06087 | 0.19901 | 1.18595 | 1.96455 | 2.63956 |
| -7.5 | 3.14726 | 2.93428 | 2.55465 | 1.99037 | 1.2328 | 0.2542 | 0.87027 | 1.67038 | 2.33212 |
| -10 | 3.22396 | 2.95805 | 2.52133 | 1.93247 | 1.23445 | 0.3556 | 0.50331 | 1.28637 | 1.9123 |
| -12.5 | 3.07068 | 2.74729 | 2.25888 | 1.66663 | 1.05286 | 0.30384 | 0.20476 | 0.85233 | 1.44223 |
| -15 | 2.84939 | 2.48467 | 1.9677 | 1.40481 | 0.83355 | 0.25409 | 0.09568 | 0.52539 | 1.06803 |
| -17.5 | 2.72238 | 2.30187 | 1.76109 | 1.21107 | 0.67708 | 0.29328 | 0.15411 | 0.36585 | 0.80762 |
| -20 | 2.68095 | 2.21572 | 1.67902 | 1.12615 | 0.61294 | 0.29366 | 0.17288 | 0.31532 | 0.66345 |
| -22.5 | 2.71239 | 2.2095 | 1.68074 | 1.12556 | 0.60541 | 0.3303 | 0.23704 | 0.31153 | 0.57112 |
| -25 | 2.81193 | 2.27913 | 1.74073 | 1.17513 | 0.63404 | 0.34556 | 0.24875 | 0.27893 | 0.48835 |
| -27.5 | 2.97958 | 2.41435 | 1.85707 | 1.27501 | 0.69398 | 0.34463 | 0.18368 | 0.22846 | 0.53166 |
| -30 | | | | | 0.80373 | | | | |

**Tabelle 1: (Forts.)**

EP 2 356 507 B1

Fig.8 – Teil 3/3

| | 12.5 | 15 | 17.5 | 20 | 22.5 | 25 | 27.5 | 30 |
|---|---|---|---|---|---|---|---|---|
| 25 | 0.17608 | 0.27027 | | | | | | |
| 22.5 | 0.22376 | 0.28247 | 0.34257 | | | | | |
| 20 | 0.23367 | 0.32601 | 0.39356 | 0.46308 | | | | |
| 17.5 | 0.29249 | 0.38882 | 0.47443 | 0.53646 | 0.58698 | | | |
| 15 | 0.38677 | 0.48915 | 0.57363 | 0.63195 | 0.67122 | 0.69402 | | |
| 12.5 | 0.51508 | 0.62308 | 0.69911 | 0.75171 | 0.77819 | 0.79015 | | |
| 10 | 0.69838 | 0.80874 | 0.8799 | 0.92048 | 0.93695 | 0.94407 | 1.11766 | |
| 7.5 | 0.97115 | 1.08049 | 1.1477 | 1.18964 | 1.23768 | 1.3403 | 1.49313 | |
| 5 | 1.38421 | 1.49282 | 1.56192 | 1.61501 | 1.66973 | 1.75831 | 1.88786 | |
| 2.5 | 1.94655 | 2.02812 | 2.05853 | 2.07752 | 2.11326 | 2.18525 | 2.29426 | |
| 0 | 2.56692 | 2.62416 | 2.59163 | 2.5513 | 2.55365 | 2.60625 | 2.6838 | 2.87446 |
| -2.5 | 3.05693 | 3.18702 | 3.13201 | 3.03676 | 2.98397 | 3.00112 | 3.08941 | |
| -5 | 3.20501 | 3.54665 | 3.59948 | 3.50087 | 3.43532 | 3.42408 | 3.49288 | |
| -7.5 | 2.99045 | 3.57474 | 3.85191 | 3.87283 | 3.72308 | 3.58091 | 3.47107 | |
| -10 | 2.58906 | 3.30954 | 3.80001 | 3.92848 | 3.73408 | 3.59482 | 3.51238 | |
| -12.5 | 2.10938 | 2.8859 | 3.49138 | 3.79671 | 3.70332 | 3.59928 | | |
| -15 | 1.69011 | 2.4643 | 3.15214 | 3.57983 | 3.69056 | 3.57305 | | |
| -17.5 | 1.40592 | 2.16171 | 2.88996 | 3.37874 | 3.6561 | | | |
| -20 | 1.25138 | 1.98959 | 2.69927 | 3.24736 | | | | |
| -22.5 | 1.16913 | 1.90323 | 2.57753 | | | | | |
| -25 | 1.13261 | 1.84208 | | | | | | |

**Tabelle 1: (Forts.)**

EP 2 356 507 B1

# Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008089998 A1

- US 20030107702 A1

**In der Beschreibung aufgeführte Nicht-Patentliteratur**

- **DIEPES H. ; BLENDOWSKE R.** Optik und Technik der Brille. *Optische Fachveröffentlichung GmbH,* 2002, 481 ff

- **W. BECKEN ; A. SEIDEMANN ; H. ALTHEIMER ; G. ESSER ; D. UTTENWEILER.** Brillengläser im Sport: Optimierung der Abbildungseigenschaften unter physiologischen Aspekten. *Z. Med. Phys.,* 2007, vol. 17, 56-66